# EUROPEAN PATENT APPLICATION

(11) **EP 1 120 426 A1**
(43) Date of publication of application: **01.08.2001**
(21) Application number: 99970399.4
(22) Date of filing: 08.10.1999
(51) Int. Cl.: C07K 14/705, C12N 15/12, C12N 5/10, C12P 21/02, C12P 21/08, C07K 16/28, G01N 33/68, G01N 33/53

(54) **NOVEL G PROTEIN-COUPLED RECEPTORS**

(30) Priority: 09.10.1998 JP 28856598; 07.12.1998 JP 34754698; 21.12.1998 JP 36353798
(71) Applicant: Chugai Research Institute for Molecular Medicine Inc., Niihari-gun, Ibaraki 300-4101 (JP)
(72) Inventor: MAEDA, Masatsugu Chugai Res. Inst. Mole. Med. Inc., Niihari-gun Ibaraki 300-4101 (JP); NAKATA, Yasuhiko Chugai Res. Inst. Mole. Med. Inc., Niihari-gun Ibaraki 300-4101 (JP); NOMURA, Hitoshi Chugai Res. Inst. Mole. Med. Inc., Niihari-gun Ibaraki 300-4101 (JP)
(74) Representative: VOSSIUS & PARTNER
(86) International application number: JP9905578
(87) International publication number: WO0021999

(57) **Abstract**

Partial cDNA sequences of target genes are obtained by performing RT-PCR with the use of an oligonucleotide primer designed based on a human genome sequence containing novel G protein-coupled receptor genes identified within a database. Further, full-length cDNAs of these genes are successfully isolated by the 5'-RACE method and 3'-RACE method, with the use of a human testis-origin cDNA library as a template. The proteins encoded by thus isolated genes have the characteristics of known olfactory receptor (OR) gene family. Moreover, the expression properties of them suggest that these genes may have functions relating to immune response and hemopoiesis.

SEQUENCE LISTING

<110> CHUGAI RESEARCH INSTITUTE FOR MOLECULAR MEDICINE, INC.

<120> Novel G protein-coupled receptors

<130> C2-012DP1PCT

<140>
<141>

<150> JP 1998-288565
<151> 1998-10-09

<150> JP 1998-347546
<151> 1998-12-07

<150> JP 1998-363537
<151> 1998-12-21

<160> 53

<170> PatentIn version 2.0

<210> 1

<211> 1143

<212> DNA

<213> Homo sapiens

<220>

<221> CDS

<222> (9)..(947)

<400> 1

ctcattga atg gac agt cta aac caa aca aga gtg act gaa ttt gtc ttc 50

Met Asp Ser Leu Asn Gln Thr Arg Val Thr Glu Phe Val Phe

1               5                   10

ttg gga ctc act gat aac cgg gtg ctg gaa atg ctg ttt ttc atg gca 98

Leu Gly Leu Thr Asp Asn Arg Val Leu Glu Met Leu Phe Phe Met Ala

15                  20                  25                  30

ttc tca gcc att tat atg cta acg ctt tca ggg aac att ctc atc atc 146

Phe Ser Ala Ile Tyr Met Leu Thr Leu Ser Gly Asn Ile Leu Ile Ile

                35                  40                  45

att gcc aca gtc ttt act cca agt ctc cat acc ccc atg tat ttc ttc 194

Ile Ala Thr Val Phe Thr Pro Ser Leu His Thr Pro Met Tyr Phe Phe

            50                  55                  60

ctg agc aat ctg tcc ttt att gac atc tgc cac tca tct gtc act gtg 242

Leu Ser Asn Leu Ser Phe Ile Asp Ile Cys His Ser Ser Val Thr Val

        65                  70                  75

cct aag atg ttg gag ggt ttg ctt tta gaa aga aag acc att tcc ttt 290

Pro Lys Met Leu Glu Gly Leu Leu Leu Glu Arg Lys Thr Ile Ser Phe

    80                  85                  90

gac aac tgc atc aca cag ctc ttc ttc cta cat ctc ttt gcc tgt gcc 338
Asp Asn Cys Ile Thr Gln Leu Phe Phe Leu His Leu Phe Ala Cys Ala
95                  100                 105                 110
gag atc ttt ctg ctg atc att gtg gcg tat gat cgt tac gtg gct atc 386
Glu Ile Phe Leu Leu Ile Ile Val Ala Tyr Asp Arg Tyr Val Ala Ile
                115                 120                 125
tgc act cca ctc cac tac ccc aat gtg atg aac atg aga gtc tgt ata 434
Cys Thr Pro Leu His Tyr Pro Asn Val Met Asn Met Arg Val Cys Ile
            130                 135                 140
cag ctt gtc ttt gct ctc tgg ttg ggg ggt act gtt cac tca cta ggg 482
Gln Leu Val Phe Ala Leu Trp Leu Gly Gly Thr Val His Ser Leu Gly
        145                 150                 155
cag acc ttc ttg act att cgt cta cct tac tgt ggc ccc aac att att 530
Gln Thr Phe Leu Thr Ile Arg Leu Pro Tyr Cys Gly Pro Asn Ile Ile
    160                 165                 170
gac agc tac ttc tgt gat gtg cct ctt gtt atc aag ctg gcc tgc aca 578
Asp Ser Tyr Phe Cys Asp Val Pro Leu Val Ile Lys Leu Ala Cys Thr
175                 180                 185                 190
gat aca tac ctc aca gga ata ctg att gtg acc aat agt gga acc atc 626
Asp Thr Tyr Leu Thr Gly Ile Leu Ile Val Thr Asn Ser Gly Thr Ile
                195                 200                 205
tcc ctc tcc tgt ttc ttg gcc gtg gtc acc tcc tat atg gtc atc ctg 674
Ser Leu Ser Cys Phe Leu Ala Val Val Thr Ser Tyr Met Val Ile Leu
            210                 215                 220
gtt tct ctt cga aaa cac tca gct gaa ggg cgc cag aaa gcc ctg tct 722
Val Ser Leu Arg Lys His Ser Ala Glu Gly Arg Gln Lys Ala Leu Ser

        225                 230                 235

acc tgc tcg gcc cac ttc atg gtg gtt gcc ctc ttc ttt ggg cca tgt 770

Thr Cys Ser Ala His Phe Met Val Val Ala Leu Phe Phe Gly Pro Cys

    240                 245                 250

atc ttc atc tat act cgg cca gac acc agc ttc tcc att gac aag gtg 818

Ile Phe Ile Tyr Thr Arg Pro Asp Thr Ser Phe Ser Ile Asp Lys Val

255                 260                 265                 270

gtg tct gtc ttc tac aca gtg gtc acc cct ttg ctg aat ccc ttc att 866

Val Ser Val Phe Tyr Thr Val Val Thr Pro Leu Leu Asn Pro Phe Ile

                275                 280                 285

tac acc ttg agg aat gag gag gta aaa agt gcc atg aag cag ctc agg 914

Tyr Thr Leu Arg Asn Glu Glu Val Lys Ser Ala Met Lys Gln Leu Arg

            290                 295                 300

cag aga caa gtt ttt ttc acg aaa tca tat aca taatgggcat tgggattgca 967

Gln Arg Gln Val Phe Phe Thr Lys Ser Tyr Thr

        305                 310

gacataattg cagccacatc cttaatgaaa gagcaaaagt aaagagtcaa aatcaactta 1027

tataacttgg taaattaggt aaaatggcat agagcaggtc agatttctgc tcattaaaga 1087

taagaactta ttctgttcat taaagataag aacttattaa ctattattta aataaa 1143

<210> 2

<211> 1248

<212> DNA

<213> Homo sapiens

<220>

<221> CDS
<222> (13)..(951)
<400> 2
attctctggg at atg gaa aga atc aac agc aca ctg ttg act gcg ttt atc 51
Met Glu Arg Ile Asn Ser Thr Leu Leu Thr Ala Phe Ile
1               5                   10
ctg aca gga att ccg tat cca ctc agg cta agg aca ctc ttt ttt gtg 99
Leu Thr Gly Ile Pro Tyr Pro Leu Arg Leu Arg Thr Leu Phe Phe Val
    15                  20                  25
ttc ttt ttt cta atc tac atc ctg act cag ctg gga aac ctg ctt att 147
Phe Phe Phe Leu Ile Tyr Ile Leu Thr Gln Leu Gly Asn Leu Leu Ile
30                  35                  40                  45
tta atc act gtc tgg gca gac cca agg ctc cat gcc cgc ccc atg tac 195
Leu Ile Thr Val Trp Ala Asp Pro Arg Leu His Ala Arg Pro Met Tyr
                50                  55                  60
atc ttt ctt ggt gtt ctc tca gtc att gat atg agc atc tcc tcc atc 243
Ile Phe Leu Gly Val Leu Ser Val Ile Asp Met Ser Ile Ser Ser Ile
            65                  70                  75
att gtc cct cgc ctc atg atg aac ttc act tta ggt gtc aaa ccc atc 291
Ile Val Pro Arg Leu Met Met Asn Phe Thr Leu Gly Val Lys Pro Ile
        80                  85                  90
cca ttt ggt ggc tgt gtt gct caa ctc tat ttc tat cac ttc ctg ggc 339
Pro Phe Gly Gly Cys Val Ala Gln Leu Tyr Phe Tyr His Phe Leu Gly
    95                  100                 105
agc acc cag tgc ttc ctc tac acc cta atg gcc tat gac agg tac ctg 387
Ser Thr Gln Cys Phe Leu Tyr Thr Leu Met Ala Tyr Asp Arg Tyr Leu

110                 115                 120                 125

gca ata tgt cag ccc ctg cgc tac cct gtg ctc atg act gct aag ctg 435

Ala Ile Cys Gln Pro Leu Arg Tyr Pro Val Leu Met Thr Ala Lys Leu

                130                 135                 140

agc gcc ttg ctt gtg gct gga gcc tgg atg gca gga tcc atc cat ggg 483

Ser Ala Leu Leu Val Ala Gly Ala Trp Met Ala Gly Ser Ile His Gly

            145                 150                 155

gct ctc cag gcc atc cta acc ttc cgc ctg ccc tac tgt ggg ccc aat 531

Ala Leu Gln Ala Ile Leu Thr Phe Arg Leu Pro Tyr Cys Gly Pro Asn

        160                 165                 170

cag gtg gat tac ttc ttc tgt gac atc cct gca gtg ttg aga ctg gcc 579

Gln Val Asp Tyr Phe Phe Cys Asp Ile Pro Ala Val Leu Arg Leu Ala

    175                 180                 185

tgt gct gac aca aca gtc aac gag ctg gtg acg ttt gta gac att ggg 627

Cys Ala Asp Thr Thr Val Asn Glu Leu Val Thr Phe Val Asp Ile Gly

190                 195                 200                 205

gtg gtg gtt gcc agt tgc ttc tcc ctg atc ctc ctc tcc tac ata cag 675

Val Val Val Ala Ser Cys Phe Ser Leu Ile Leu Leu Ser Tyr Ile Gln

                210                 215                 220

atc att cag gcc atc ctg aga atc cac aca gct gat ggg cgg cgc cgg 723

Ile Ile Gln Ala Ile Leu Arg Ile His Thr Ala Asp Gly Arg Arg Arg

            225                 230                 235

gct ttt tca act tgt gga gcc cat gta acc gtg gtc acc gtg tac tat 771

Ala Phe Ser Thr Cys Gly Ala His Val Thr Val Val Thr Val Tyr Tyr

        240                 245                 250

gtg ccc tgt gcc ttc atc tac ctg agg cct gaa acc aac agc ccc ctg 819

Val Pro Cys Ala Phe Ile Tyr Leu Arg Pro Glu Thr Asn Ser Pro Leu
    255                 260                 265
gat ggg gca gct gcc cta gtc ccc acg gcc atc act cct ttc ctc aac 867
Asp Gly Ala Ala Ala Leu Val Pro Thr Ala Ile Thr Pro Phe Leu Asn
270                 275                 280                 285
ccc ctt atc tac act ctg cgg aac caa gag gtg aag ctg gcc ctg aaa 915
Pro Leu Ile Tyr Thr Leu Arg Asn Gln Glu Val Lys Leu Ala Leu Lys
                290                 295                 300
aga atg ctc aga agc cca aga act ccg agt gag gtt tgaaagtgtc 961
Arg Met Leu Arg Ser Pro Arg Thr Pro Ser Glu Val
            305                 310
tttctcccac tagggaagct gccacaatta gaatttatta taatgtttag gcttcggtaa 1021
ctttttttctt ttcttcttgt tttttctctt ttatatagcc atactgtatg atcaaacaca 1081
gtttaaggta aaatactaac tttctaacag ttccttagta tcctctcaag ataactctca 1141
gccactgcaa gagtagagaa tgagaccaaa ttctcacaaa ctaaaccaca ttaaacaatc 1201
cagaagaaag aatgcaatag tgtattttcc aatgtctcag taataaa 1248

<210> 3
<211> 1431
<212> DNA
<213> Homo sapiens
<220>
<221> CDS
<222> (410)..(1339)
<400> 3
ggcaacctaa aagcaagcat ggacagttcc ttggtgaata accaaaaaca agatggagtc 60

tcgctctgtt gcccaggctg gagtgtagtg gcgccatctc ggctcgctgc ggtctccgcc 120

tcccgggttc aggcgattct ccggcctcag cctcccgggt gcgtgggatt gcaggaacta 180

gaactaaagc gaggttaatt tccacagtga gaacatgctc cagacatccg agcaccagtg 240

tggctctgga aactccacag ataccacagg actagaaaat aactggacaa tgggatgttc 300

tatcttgccc gaactgaggg atataaaaag ctccaaagac aaagaaagta ccatccaccc 360

atcccaaaag aaattatcct tccttctgaa aataagactg caaaaagac atg gga aag 418

Met Gly Lys

1

acc aaa aac aca tcg ctg gat gcc gtg gtg aca gat ttc att ctt ctg 466

Thr Lys Asn Thr Ser Leu Asp Ala Val Val Thr Asp Phe Ile Leu Leu

    5                   10                  15

ggt ttg tct cac ccc cca aat cta aga agc ctc ctc ttc ctg gtc ttc 514

Gly Leu Ser His Pro Pro Asn Leu Arg Ser Leu Leu Phe Leu Val Phe

20                  25                  30                  35

ttc atc att tac atc ctc act cag ctg ggg aac ctg ctc att ctg ctc 562

Phe Ile Ile Tyr Ile Leu Thr Gln Leu Gly Asn Leu Leu Ile Leu Leu

                40                  45                  50

acc atg tgg gct gac ccg aag ctc tgt gct cgc ccc atg tac att ctt 610

Thr Met Trp Ala Asp Pro Lys Leu Cys Ala Arg Pro Met Tyr Ile Leu

            55                  60                  65

ctg gga gtg ctc tca ttc ctg gac atg tgg ctc tcc tca gtc acc gtt 658

Leu Gly Val Leu Ser Phe Leu Asp Met Trp Leu Ser Ser Val Thr Val

        70                  75                  80

cct cgg ctt att ttg gat ttt act cct tcc atc aag gct atc ccg ttt 706

Pro Arg Leu Ile Leu Asp Phe Thr Pro Ser Ile Lys Ala Ile Pro Phe

    85                  90                  95

ggt ggc tgt gtg gct caa ctg tat ttc ttt cac ttc ctg ggc agc acc 754
Gly Gly Cys Val Ala Gln Leu Tyr Phe Phe His Phe Leu Gly Ser Thr
100                 105                 110                 115
cag tgc ttc ctc tac acc ttg atg gcc tat gac agg tac cta gca ata 802
Gln Cys Phe Leu Tyr Thr Leu Met Ala Tyr Asp Arg Tyr Leu Ala Ile
                120                 125                 130
tgt cag ccc ctg cac tac cca gtg ctc atg aat ggg agg tta tgc aca 850
Cys Gln Pro Leu His Tyr Pro Val Leu Met Asn Gly Arg Leu Cys Thr
            135                 140                 145
gtc ctt gtg gct gga gct tgg gtc gcc ggc tcc atg cat ggg tct atc 898
Val Leu Val Ala Gly Ala Trp Val Ala Gly Ser Met His Gly Ser Ile
        150                 155                 160
cag gcc acc ttg acc ttc cgc ctg ccc tac tgt ggg ccc aat cag gtg 946
Gln Ala Thr Leu Thr Phe Arg Leu Pro Tyr Cys Gly Pro Asn Gln Val
    165                 170                 175
gat tac ttt atc tgt gac atc cgc gca gta ttg aga ctg gcc tgt gct 994
Asp Tyr Phe Ile Cys Asp Ile Arg Ala Val Leu Arg Leu Ala Cys Ala
180                 185                 190                 195
gac aca act gtc aat gag ctt gtg acc ttt gtg gac gtc agg gta gtg 1042
Asp Thr Thr Val Asn Glu Leu Val Thr Phe Val Asp Val Arg Val Val
                200                 205                 210
gcc gcc agt tgc ttc atg tta att ctg ctc tcc tat gcc aac ata gtc 1090
Ala Ala Ser Cys Phe Met Leu Ile Leu Leu Ser Tyr Ala Asn Ile Val
            215                 220                 225
cat gcc atc ctg aag ata cgc acc gct gat ggg agg cgc cgg gcc ttc 1138
His Ala Ile Leu Lys Ile Arg Thr Ala Asp Gly Arg Arg Arg Ala Phe

        230                 235                 240

tcc acc tgt ggc tcc cac cta atc gtg gtc aca gtc tac tat gtc ccc 1186

Ser Thr Cys Gly Ser His Leu Ile Val Val Thr Val Tyr Tyr Val Pro

    245                 250                 255

tgt att ttc atc tac ctt agg gct ggc tcc aaa gac ccc ctg gat ggg 1234

Cys Ile Phe Ile Tyr Leu Arg Ala Gly Ser Lys Asp Pro Leu Asp Gly

260                 265                 270                 275

gca gcg gct gtg ttt tac act gtt gtc act cca tta ctg aac ccc ctc 1282

Ala Ala Ala Val Phe Tyr Thr Val Val Thr Pro Leu Leu Asn Pro Leu

                280                 285                 290

atc tat aca ctg agg aac cag gaa gtg aag tct gcc ctg aag agg ata 1330

Ile Tyr Thr Leu Arg Asn Gln Glu Val Lys Ser Ala Leu Lys Arg Ile

            295                 300                 305

aca gca ggt tgaaggactg aatgaaaata agtaactaca tctgcatcat 1379

Thr Ala Gly

        310

tatcactgcc actctcttca gctactgctg catgtgacaa atgcccaata aa 1431

<210> 4

<211> 313

<212> PRT

<213> Homo sapiens

<400> 4

Met Asp Ser Leu Asn Gln Thr Arg Val Thr Glu Phe Val Phe Leu Gly

1               5                   10                  15

Leu Thr Asp Asn Arg Val Leu Glu Met Leu Phe Phe Met Ala Phe Ser

            20                  25                  30
Ala Ile Tyr Met Leu Thr Leu Ser Gly Asn Ile Leu Ile Ile Ile Ala
        35                  40                  45
Thr Val Phe Thr Pro Ser Leu His Thr Pro Met Tyr Phe Phe Leu Ser
    50                  55                  60
Asn Leu Ser Phe Ile Asp Ile Cys His Ser Ser Val Thr Val Pro Lys
65                  70                  75                  80
Met Leu Glu Gly Leu Leu Leu Glu Arg Lys Thr Ile Ser Phe Asp Asn
                85                  90                  95
Cys Ile Thr Gln Leu Phe Phe Leu His Leu Phe Ala Cys Ala Glu Ile
            100                 105                 110
Phe Leu Leu Ile Ile Val Ala Tyr Asp Arg Tyr Val Ala Ile Cys Thr
        115                 120                 125
Pro Leu His Tyr Pro Asn Val Met Asn Met Arg Val Cys Ile Gln Leu
    130                 135                 140
Val Phe Ala Leu Trp Leu Gly Gly Thr Val His Ser Leu Gly Gln Thr
145                 150                 155                 160
Phe Leu Thr Ile Arg Leu Pro Tyr Cys Gly Pro Asn Ile Ile Asp Ser
                165                 170                 175
Tyr Phe Cys Asp Val Pro Leu Val Ile Lys Leu Ala Cys Thr Asp Thr
            180                 185                 190
Tyr Leu Thr Gly Ile Leu Ile Val Thr Asn Ser Gly Thr Ile Ser Leu
        195                 200                 205
Ser Cys Phe Leu Ala Val Val Thr Ser Tyr Met Val Ile Leu Val Ser
    210                 215                 220
Leu Arg Lys His Ser Ala Glu Gly Arg Gln Lys Ala Leu Ser Thr Cys

225                 230                 235                 240

Ser Ala His Phe Met Val Val Ala Leu Phe Phe Gly Pro Cys Ile Phe

                245                 250                 255

Ile Tyr Thr Arg Pro Asp Thr Ser Phe Ser Ile Asp Lys Val Val Ser

            260                 265                 270

Val Phe Tyr Thr Val Val Thr Pro Leu Leu Asn Pro Phe Ile Tyr Thr

        275                 280                 285

Leu Arg Asn Glu Glu Val Lys Ser Ala Met Lys Gln Leu Arg Gln Arg

    290                 295                 300

Gln Val Phe Phe Thr Lys Ser Tyr Thr

305                 310

<210> 5

<211> 313

<212> PRT

<213> Homo sapiens

<400> 5

Met Glu Arg Ile Asn Ser Thr Leu Leu Thr Ala Phe Ile Leu Thr Gly

  1               5                  10                  15

Ile Pro Tyr Pro Leu Arg Leu Arg Thr Leu Phe Phe Val Phe Phe Phe

             20                  25                  30

Leu Ile Tyr Ile Leu Thr Gln Leu Gly Asn Leu Leu Ile Leu Ile Thr

         35                  40                  45

Val Trp Ala Asp Pro Arg Leu His Ala Arg Pro Met Tyr Ile Phe Leu

     50                  55                  60

Gly Val Leu Ser Val Ile Asp Met Ser Ile Ser Ser Ile Ile Val Pro

65                  70                  75                  80
Arg Leu Met Met Asn Phe Thr Leu Gly Val Lys Pro Ile Pro Phe Gly
                85                  90                  95
Gly Cys Val Ala Gln Leu Tyr Phe Tyr His Phe Leu Gly Ser Thr Gln
            100                 105                 110
Cys Phe Leu Tyr Thr Leu Met Ala Tyr Asp Arg Tyr Leu Ala Ile Cys
        115                 120                 125
Gln Pro Leu Arg Tyr Pro Val Leu Met Thr Ala Lys Leu Ser Ala Leu
    130                 135                 140
Leu Val Ala Gly Ala Trp Met Ala Gly Ser Ile His Gly Ala Leu Gln
145                 150                 155                 160
Ala Ile Leu Thr Phe Arg Leu Pro Tyr Cys Gly Pro Asn Gln Val Asp
                165                 170                 175
Tyr Phe Phe Cys Asp Ile Pro Ala Val Leu Arg Leu Ala Cys Ala Asp
            180                 185                 190
Thr Thr Val Asn Glu Leu Val Thr Phe Val Asp Ile Gly Val Val Val
        195                 200                 205
Ala Ser Cys Phe Ser Leu Ile Leu Leu Ser Tyr Ile Gln Ile Ile Gln
    210                 215                 220
Ala Ile Leu Arg Ile His Thr Ala Asp Gly Arg Arg Arg Ala Phe Ser
225                 230                 235                 240
Thr Cys Gly Ala His Val Thr Val Val Thr Val Tyr Tyr Val Pro Cys
                245                 250                 255
Ala Phe Ile Tyr Leu Arg Pro Glu Thr Asn Ser Pro Leu Asp Gly Ala
            260                 265                 270
Ala Ala Leu Val Pro Thr Ala Ile Thr Pro Phe Leu Asn Pro Leu Ile

275                 280                 285

Tyr Thr Leu Arg Asn Gln Glu Val Lys Leu Ala Leu Lys Arg Met Leu

    290                 295                 300

Arg Ser Pro Arg Thr Pro Ser Glu Val

305                 310

<210> 6

<211> 310

<212> PRT

<213> Homo sapiens

<400> 6

Met Gly Lys Thr Lys Asn Thr Ser Leu Asp Ala Val Val Thr Asp Phe

 1               5                  10                  15

Ile Leu Leu Gly Leu Ser His Pro Pro Asn Leu Arg Ser Leu Leu Phe

            20                  25                  30

Leu Val Phe Phe Ile Ile Tyr Ile Leu Thr Gln Leu Gly Asn Leu Leu

        35                  40                  45

Ile Leu Leu Thr Met Trp Ala Asp Pro Lys Leu Cys Ala Arg Pro Met

    50                  55                  60

Tyr Ile Leu Leu Gly Val Leu Ser Phe Leu Asp Met Trp Leu Ser Ser

65                  70                  75                  80

Val Thr Val Pro Arg Leu Ile Leu Asp Phe Thr Pro Ser Ile Lys Ala

                85                  90                  95

Ile Pro Phe Gly Gly Cys Val Ala Gln Leu Tyr Phe Phe His Phe Leu

            100                 105                 110

Gly Ser Thr Gln Cys Phe Leu Tyr Thr Leu Met Ala Tyr Asp Arg Tyr

        115                 120                 125
Leu Ala Ile Cys Gln Pro Leu His Tyr Pro Val Leu Met Asn Gly Arg
    130                 135                 140
Leu Cys Thr Val Leu Val Ala Gly Ala Trp Val Ala Gly Ser Met His
145                 150                 155                 160
Gly Ser Ile Gln Ala Thr Leu Thr Phe Arg Leu Pro Tyr Cys Gly Pro
                165                 170                 175
Asn Gln Val Asp Tyr Phe Ile Cys Asp Ile Arg Ala Val Leu Arg Leu
            180                 185                 190
Ala Cys Ala Asp Thr Thr Val Asn Glu Leu Val Thr Phe Val Asp Val
        195                 200                 205
Arg Val Val Ala Ala Ser Cys Phe Met Leu Ile Leu Leu Ser Tyr Ala
    210                 215                 220
Asn Ile Val His Ala Ile Leu Lys Ile Arg Thr Ala Asp Gly Arg Arg
225                 230                 235                 240
Arg Ala Phe Ser Thr Cys Gly Ser His Leu Ile Val Val Thr Val Tyr
                245                 250                 255
Tyr Val Pro Cys Ile Phe Ile Tyr Leu Arg Ala Gly Ser Lys Asp Pro
            260                 265                 270
Leu Asp Gly Ala Ala Ala Val Phe Tyr Thr Val Val Thr Pro Leu Leu
        275                 280                 285
Asn Pro Leu Ile Tyr Thr Leu Arg Asn Gln Glu Val Lys Ser Ala Leu
    290                 295                 300
Lys Arg Ile Thr Ala Gly
305                 310

<210> 7

<211> 27

<212> DNA

<213> Artificial Sequence

<220>

<223> Artificially Synthesized Primer Sequence

<400>7

ATGGACAGTC TAAACCAAAC AAGAGTG 27

<210> 8

<211> 27

<212> DNA

<213> Artificial Sequence

<220>

<223> Artificially Synthesized Primer Sequence

<400> 8

ATGGCATTCT CAGCCATTTA TATGCTA 27

<210> 9

<211> 27

<212> DNA

<213> Artificial Sequence

<220>

<223> Artificially Synthesized Primer Sequence

<400> 9

GGGAACATTC TCATCATCAT TGCCACA 27

<210> 10

<211> 27

<212> DNA

<213> Artificial Sequence

<220>

<223> Artificially Synthesized Primer Sequence

<400> 10

TTATGTATAT GATTTCGTGA AAAAAAC 27

<210> 11

<211> 27

<212> DNA

<213> Artificial Sequence

<220>

<223> Artificially Synthesized Primer Sequence

<400> 11

TACCTCCTCA TTCCTCAAGG TGTAAAT 27

<210> 12

<211> 27

<212> DNA

<213> Artificial Sequence

<220>

<223> Artificially Synthesized Primer Sequence

<400> 12

GGTGACCACT GTGTAGAAGA CAGACAC 27

<210> 13

<211> 27

<212> DNA

<213> Artificial Sequence

<220>

<223> Artificially Synthesized Primer Sequence

<400> 13

ATGGAAAGAA TCAACAGCAC ACTGTTG 27

<210> 14

<211> 27

<212> DNA

<213> Artificial Sequence

<220>

<223> Artificially Synthesized Primer Sequence

<400> 14

TCTAATCTAC ATCCTGACTC AGCTGGG 27

<210> 15

<211> 27

<212> DNA

<213> Artificial Sequence

<220>

<223> Artificially Synthesized Primer Sequence

<400> 15

TTCAAACCTC ACTCGGAGTT CTTGGGC 27

<210> 16

<211> 27

<212> DNA

<213> Artificial Sequence

<220>

<223> Artificially Synthesized Primer Sequence

<400> 16

AGCTTCACCT CTTGGTTCCG CAGAGTG 27

<210> 17

<211> 27

<212> DNA

<213> Artificial Sequence

<220>

<223> Artificially Synthesized Primer Sequence

<400> 17

ATGGGAAAGA CCAAAAACAC ATCGCTG 27

<210> 18

<211> 27

<212> DNA

<213> Artificial Sequence

<220>

<223> Artificially Synthesized Primer Sequence

<400> 18

CGTGGTGACA GATTTCATTC TTCTGGG 27

<210> 19

<211> 27

<212> DNA

<213> Artificial Sequence

<220>

<223> Artificially Synthesized Primer Sequence

<400> 19

TCAACCTGCT GTTATCCTCT TCAGGGC 27

<210> 20

<211> 27

<212> DNA

<213> Artificial Sequence

<220>

<223> Artificially Synthesized Primer Sequence

<400> 20

CCTGGTTCCT CAGTGTATAG ATGAGGG 27

<210> 21

<211> 942

<212> DNA

<213> Homo sapiens

<400> 21

atggacagtc taaaccaaac aagagtgact gaatttgtct tcttgggact cactgataac 60

cgggtgctgg aaatgctgtt tttcatggca ttctcagcca tttatatgct aacgctttca 120

gggaacattc tcatcatcat tgccacagtc tttactccaa gtctccatac ccccatgtat 180

ttcttcctga gcaatctgtc ctttattgac atctgccact catctgtcac tgtgcctaag 240

atgttggagg gtttgctttt agaaagaaag accatttcct ttgacaactg catcacacag 300

ctcttcttcc tacatctctt tgcctgtgcc gagatctttc tgctgatcat tgtggcgtat 360

gatcgttacg tggctatctg cactccactc cactacccca atgtgatgaa catgagagtc 420

tgtatacagc ttgtctttgc tctctggttg gggggtactg ttcactcact agggcagacc 480

ttcttgacta ttcgtctacc ttactgtggc cccaacatta ttgacagcta cttctgtgat 540

gtgcctcttg ttatcaagct ggcctgcaca gatacatacc tcacaggaat actgattgtg 600

accaatagtg gaaccatctc cctctcctgt ttcttggccg tggtcacctc ctatatggtc 660

atcctggttt ctcttcgaaa acactcagct gaagggcgcc agaaagccct gtctacctgc 720

tcggcccact tcatggtggt tgccctcttc tttgggccat gtatcttcat ctatactcgg 780

ccagacacca gcttctccat tgacaaggtg gtgtctgtct tctacacagt ggtcacccct 840

ttgctgaatc ccttcattta caccttgagg aatgaggagg taaaaagtgc catgaagcag 900

ctcaggcaga gacaagtttt tttcacgaaa tcatatacat aa 942

<210> 22

<211> 942

<212> DNA

<213> Homo sapiens

<400> 22

atggaaagaa tcaacagcac actgttgact gcgtttatcc tgacaggaat tccgtatcca 60

ctcaggctaa ggacactctt ttttgtgttc ttttttctaa tctacatcct gactcagctg 120
ggaaacctgc ttattttaat cactgtctgg gcagacccaa ggctccatgc ccgccccatg 180
tacatctttc ttggtgttct ctcagtcatt gatatgagca tctcctccat cattgtccct 240
cgcctcatga tgaacttcac tttaggtgtc aaacccatcc catttggtgg ctgtgttgct 300
caactctatt tctatcactt cctgggcagc acccagtgct tcctctacac cctaatggcc 360
tatgacaggt acctggcaat atgtcagccc ctgcgctacc ctgtgctcat gactgctaag 420
ctgagcgcct tgcttgtggc tggagcctgg atggcaggat ccatccatgg ggctctccag 480
gccatcctaa ccttccgcct gccctactgt gggcccaatc aggtggatta cttcttctgt 540
gacatccctg cagtgttgag actggcctgt gctgacacaa cagtcaacga gctggtgacg 600
tttgtagaca ttggggtggt ggttgccagt tgcttctccc tgatcctcct ctcctacata 660
cagatcattc aggccatcct gagaatccac acagctgatg ggcggcgccg ggctttttca 720
acttgtggag cccatgtaac cgtggtcacc gtgtactatg tgccctgtgc cttcatctac 780
ctgaggcctg aaaccaacag ccccctggat ggggcagctg ccctagtccc cacggccatc 840
actcctttcc tcaaccccct tatctacact ctgcggaacc aagaggtgaa gctggccctg 900
aaaagaatgc tcagaagccc aagaactccg agtgaggttt ga 942

<210> 23
<211> 933
<212> DNA
<213> Homo sapiens
<400> 23
   atgggaaaga ccaaaaacac atcgctggat gccgtggtga cagatttcat tcttctgggt 60
   ttgtctcacc ccccaaatct aagaagcctc ctcttcctgg tcttcttcat catttacatc 120
   ctcactcagc tggggaacct gctcattctg ctcaccatgt gggctgaccc gaagctctgt 180
   gctcgcccca tgtacattct tctgggagtg ctctcattcc tggacatgtg gctctcctca 240
   gtcaccgttc ctcggcttat tttggatttt actccttcca tcaaggctat cccgtttggt 300
   ggctgtgtgg ctcaactgta tttctttcac ttcctgggca gcacccagtg cttcctctac 360
   accttgatgg cctatgacag gtacctagca atatgtcagc ccctgcacta cccagtgctc 420
   atgaatggga ggttatgcac agtccttgtg gctggagctt gggtcgccgg ctccatgcat 480
   gggtctatcc aggccacctt gaccttccgc ctgccctact gtgggcccaa tcaggtggat 540
   tactttatct gtgacatccg cgcagtattg agactggcct gtgctgacac aactgtcaat 600
   gagcttgtga cctttgtgga cgtcagggta gtggccgcca gttgcttcat gttaattctg 660
   ctctcctatg ccaacatagt ccatgccatc ctgaagatac gcaccgctga tgggaggcgc 720
   cgggccttct ccacctgtgg ctcccaccta atcgtggtca cagtctacta tgtcccctgt 780
   attttcatct accttagggc tggctccaaa gaccccctgg atggggcagc ggctgtgttt 840
   tacactgttg tcactccatt actgaacccc ctcatctata cactgaggaa ccaggaagtg 900
   aagtctgccc tgaagaggat aacagcaggt tga 933

<210> 24

<211> 1060

<212> DNA

<213> Homo sapiens

<220>

<221> CDS

<222> (17)..(892)

<400> 24

aaatgcctaa agaaga atg acc atg gaa aat tat tct atg gca gct cag ttt 52

Met Thr Met Glu Asn Tyr Ser Met Ala Ala Gln Phe

1               5                   10

gtc tta gat ggt tta aca cag caa gca gag ctc cag ctg ccc ctc ttc 100
Val Leu Asp Gly Leu Thr Gln Gln Ala Glu Leu Gln Leu Pro Leu Phe
        15                  20                  25

ctc ctg ttc ctg gga atc tat gtg gtc aca gta gtg ggc aac ctg ggc 148
Leu Leu Phe Leu Gly Ile Tyr Val Val Thr Val Val Gly Asn Leu Gly
    30                  35                  40

atg att ctc ctg att gca gtc agc cct cta ctt cac acc ccc atg tac 196
Met Ile Leu Leu Ile Ala Val Ser Pro Leu Leu His Thr Pro Met Tyr
45                  50                  55                  60

tat ttc ctc agc agc ttg tcc ttc gtc gat ttc tgc tat tcc tct gtc 244
Tyr Phe Leu Ser Ser Leu Ser Phe Val Asp Phe Cys Tyr Ser Ser Val
                65                  70                  75

att act ccc aaa atg ctg gtg aac ttc cta gga aag aag aat aca atc 292
Ile Thr Pro Lys Met Leu Val Asn Phe Leu Gly Lys Lys Asn Thr Ile
            80                  85                  90

ctt tac tct gag tgc atg gtc cag ctc ttt ttc ttt gtg gtc ttt gtg 340
Leu Tyr Ser Glu Cys Met Val Gln Leu Phe Phe Phe Val Val Phe Val
        95                  100                 105

gtg gct gag ggt tac ctc ctg act gcc atg gca tat gat cgc tat gtt 388
Val Ala Glu Gly Tyr Leu Leu Thr Ala Met Ala Tyr Asp Arg Tyr Val

    110                 115                 120

gcc atc tgt agc cca ctg ctt tat aat gcg atc atg tcc tca tgg gtc 436
Ala Ile Cys Ser Pro Leu Leu Tyr Asn Ala Ile Met Ser Ser Trp Val
125                 130                 135                 140

tgc tca ctg cta gtg ctg gct gcc ttc ttc ttg ggc ttt ctc tct gcc 484
Cys Ser Leu Leu Val Leu Ala Ala Phe Phe Leu Gly Phe Leu Ser Ala
                145                 150                 155

ttg act cat aca agt gcc atg atg aaa ctg tcc ttt tgc aaa tcc cac 532
Leu Thr His Thr Ser Ala Met Met Lys Leu Ser Phe Cys Lys Ser His
            160                 165                 170

att atc aac cat tac ttc tgt gat gtt ctt ccc ctc ctc aat ctc tcc 580
Ile Ile Asn His Tyr Phe Cys Asp Val Leu Pro Leu Leu Asn Leu Ser
        175                 180                 185

tgc tcc aac aca cac ctc aat gag ctt cta ctt ttt atc att gcg ggg 628
Cys Ser Asn Thr His Leu Asn Glu Leu Leu Leu Phe Ile Ile Ala Gly
    190                 195                 200

ttt aac acc ttg gtg ccc acc cta gct gtt gct gtc tcc tat gcc ttc 676
Phe Asn Thr Leu Val Pro Thr Leu Ala Val Ala Val Ser Tyr Ala Phe
205                 210                 215                 220

atc ctc tac agc atc ctt cac atc cgc tcc tca gag ggc cgg tcc aaa 724
Ile Leu Tyr Ser Ile Leu His Ile Arg Ser Ser Glu Gly Arg Ser Lys
                225                 230                 235

gct ttt gga aca tgc agc tct cat ctc atg gct gtg gtg atc ttc ttt 772
Ala Phe Gly Thr Cys Ser Ser His Leu Met Ala Val Val Ile Phe Phe
            240                 245                 250

ggg tcc att acc ttc atg tat ttc aag ccc cct tca agt aac tcc ctg 820
Gly Ser Ile Thr Phe Met Tyr Phe Lys Pro Pro Ser Ser Asn Ser Leu
        255                 260                 265

gac cag gag aag gtg tcc tct gtg ttc tac acc acg gtg atc ccc atg 868
Asp Gln Glu Lys Val Ser Ser Val Phe Tyr Thr Thr Val Ile Pro Met
    270                 275                 280

ctg aac cct tta ata tac agt ctg taatcacagc actttggaag gctgaggcag 922
Leu Asn Pro Leu Ile Tyr Ser Leu
285                 290

ggttgcttga gtccagtttg agaccatcct ggggaacata gtgcgatctt gtttctttcc 982

actgcctaaa aacttcaatg ctcaatttta cttgcaattt cctcttcctg acatggagaa 1042

tgttggcttg gaatgttc 1060

<210> 25

<211> 1069

<212> DNA

<213> Homo sapiens

<220>

<221> CDS

<222> (18)..(956)

<400> 25

attttttgaag acaaaaa atg ctg gct aga aac aac tcc tta gtg act gaa 50



1               5                   10

ttt att ctt gct gga tta aca gat cgt cca gag ttc tgg caa ccc ttc 98



            15                  20                  25

ttt ttc ctg ttc cta gtg atc tac att gtc acc atg gta ggc aac ctt 146



        30                  35                  40

ggc ttg atc act ctt ttc ggt cta aat tct cac ctc cac aca cca atg 194



    45                  50                  55

tac tat ttc ctc ttc aat ctc tcc ttc att gat ctc tgt tac tcc tct 242

60                  65                  70                  75

gtt ttc act ccc aaa atg cta atg aac ttt gtg tca aaa aag aat att 290

                80                  85                  90

atc tcc aat gtt ggg tgc atg act cgg ctg ttt ttc ttt ctc ttt ttc 338

            95                  100                 105

gtc atc tct gaa tgt tac atg ttg acc tca atg gca tat gat cgc tat 386

        110                 115                 120

gtg gcc atc tgt aat cca ttg ctg tat aag gtc acc atg tcc cat cag 434

    125                 130                 135

gtc tgt tct atg ctc act ttt gct gct tac ata atg gga ttg gct gga 482

140                 145                 150                 155

gcc acg gcc cac acc ggg tgc atg ttt aga ctc acc ttc tgc agt gct 530


                160                 165                 170

aat atc att aac cat tac ttg tgt gac ata ctc ccc ctc ctc cag ctt 578

            175                 180                 185

tcc tgc acc agc acc tat gtc aac gag gtg gtt gtt ctc att gtt gtg 626

        190                 195                 200

ggt act aat atc acg gta ccc agt tgt acc atc ctc att tct tat gtt 674

    205                 210                 215

ttc att gtc act agc att ctt cat atc aaa tcc act caa gga aga tca 722

220                 225                 230                 235

aaa gcc ttc agt act tgt agc tct cat gtc att gct ctg tct ctg ttt 770

                240                 245                 250

ttt ggg tca gcg gca ttc atg tat att aaa tat tct tct gga tct atg 818

            255                 260                 265

gag cag gga aaa gtt ttt tct gtt ttc tac act aat gtg gtg ccc atg 866

        270                 275                 280

ctc aat ccc ctc atc tac agt ttg agg aac aag gat gtc aaa gtt gca 914

    285                 290                 295

ctg agg aaa gct ctg att aaa att cag agg aga aat ata ttc 956

300                 305                 310

taattagaag cagtaatgat gtaaaacaat tgaaggactt caaattttta ttagtgtttt 1016

tcatgaagag atttgttgt ttctacagat ggtgttatgt gtgatttaat aaa 1069

<210> 26
<211> 1069
<212> DNA
<213> Homo sapiens

<220>
<221> CDS
<222> (18)..(956)

<400> 26

attttgaag acaaaaa atg ctg gct aga aac aac tcc tta gtg act gaa    50



1               5                   10

ttt att ctt gct gga tta aca gat cgt cca gag ttc cgg caa ccc ctc    98



        15                  20                  25

ttt ttc ctg ttt cta gtg atc tac att gtc acc atg gta ggc aac ctt    146



    30                  35                  40

ggc ttg atc att ctt ttc ggt cta aat tct cac ctc cac aca cca atg    194



45                  50                  55

tac tat ttc ctc ttc aat ctc tcc ttc att gat ctc tgt tac tcc tct    242



60                  65                  70                  75

gtt ttc act ccc aaa atg cta atg aac ttt gta tca aaa aag aat att    290



                80                  85                  90

atc tcc tat gtt ggg tgc atg act cag ctg ttt ttc ttt ctc ttt ttt 338

            95                  100                 105

gtc atc tct gaa tgc tac ata ttg acc tca atg gca tat gat cgc tat 386

        110                 115                 120

gtg gcc atc tgt aat cca ttg ctg tat aag gtc acc atg tcc cat cag 434

    125                 130                 135

gtc tgt tct atg ctc act ttt gct gct tac ata atg gga ttg gct gga 482

140                 145                 150                 155

gcc acg gcc cac acc ggg tgc atg ctt aga ctc acc ttc tgc agt gct 530

                160                 165                 170

aat atc atc aac cat tac ttg tgt gac ata ctc ccc ctc ctc cag ctt 578

            175                 180                 185

tcc tgc acc agc acc tat gtc aac gag gtg gtt gtt ctc att gtt gtg 626


        190                 195                 200

ggt att aat atc atg gta ccc agt tgt acc atc ctc att tct tat gtt 674

    205                 210                 215

ttc att gtc act agc att ctt cat atc aaa tcc act caa gga aga tca 722

220                 225                 230                 235

aaa gcc ttc agt act tgt agc tct cat gtc att gct ctg tct ctg ttt 770

                240                 245                 250

ttt ggg tca gcg gca ttc atg tat att aaa tat tct tct gga tct atg 818

            255                 260                 265

gag cag gga aaa gtt tct tct gtt ttc tac act aat gtg gtg ccc atg 866

        270                 275                 280

ctc aat cct ctc atc tac agt ttg agg aac aag gat gtc aaa gtt gca 914

    285                 290                 295

ctg agg aaa gct ctg att aaa att cag aga aga aat ata ttc 956

300                 305                 310

taattagaag cagtaataat gtaaaacgat tgaagaactt taaattttta ttagtgtgtt 1016

ccatgaagag atttgttgt ttctacagat ggtgttatgt gtgatttaat aaa 1069

<210> 27
<211> 976
<212> DNA
<213> Homo sapiens

<220>
<221> CDS
<222> (19)..(945)

<400> 27
acagctcgcc aagagaga atg act ctg aga aac agc tcc tca gtg act gag 51
Met Thr Leu Arg Asn Ser Ser Ser Val Thr Glu
1               5                   10

ttt atc ctt gtg gga tta tca gaa cag cca gag ctc cag ctc cct ctt 99
Phe Ile Leu Val Gly Leu Ser Glu Gln Pro Glu Leu Gln Leu Pro Leu
            15                  20                  25

ttc ctt cta ttc tta ggg atc tat gtg ttc act gtg gtg ggc aac ttg 147
Phe Leu Leu Phe Leu Gly Ile Tyr Val Phe Thr Val Val Gly Asn Leu
        30                  35                  40

ggc ttg atc acc tta att ggg ata aat cct agc ctt cac acc ccc atg 195
Gly Leu Ile Thr Leu Ile Gly Ile Asn Pro Ser Leu His Thr Pro Met
    45                  50                  55

tac ttt ttc ctc ttc aac ttg tcc ttt ata gat ctc tgt tat tcc tgt 243
Tyr Phe Phe Leu Phe Asn Leu Ser Phe Ile Asp Leu Cys Tyr Ser Cys
60                  65                  70                  75

gtg ttt acc ccc aaa atg ctg aat gac ttt gtt tca gaa agt atc atc 291
Val Phe Thr Pro Lys Met Leu Asn Asp Phe Val Ser Glu Ser Ile Ile
                80                  85                  90

tct tat gtg gga tgt atg act cag cta ttt ttc ttc tgt ttc ttt gtc 339
Ser Tyr Val Gly Cys Met Thr Gln Leu Phe Phe Phe Cys Phe Phe Val
            95                  100                 105

aat tct gag tgc tat gtg ttg gta tca atg gcc tat gat cgc tat gtg 387
Asn Ser Glu Cys Tyr Val Leu Val Ser Met Ala Tyr Asp Arg Tyr Val
        110                 115                 120

gcc atc tgc aac ccc ctg ctc tac atg gtc acc atg tcc cca agg gtc 435

Ala Ile Cys Asn Pro Leu Leu Tyr Met Val Thr Met Ser Pro Arg Val
    125                 130                 135

tgc ttt ctg ctg atg ttt ggt tcc tat gtg gta ggg ttt gct ggg gcc 483
Cys Phe Leu Leu Met Phe Gly Ser Tyr Val Val Gly Phe Ala Gly Ala
140                 145                 150                 155

atg gcc cac act gga agc atg ctg cga ctg acc ttc tgt gat tcc aac 531
Met Ala His Thr Gly Ser Met Leu Arg Leu Thr Phe Cys Asp Ser Asn
                160                 165                 170

gtc att gac cat tat ctg tgt gac gtt ctc ccc ctc ttg cag ctc tcc 579
Val Ile Asp His Tyr Leu Cys Asp Val Leu Pro Leu Leu Gln Leu Ser
            175                 180                 185

tgc acc agc acc cat gtc agt gag ctg gta ttt ttc att gtt gtt gga 627
Cys Thr Ser Thr His Val Ser Glu Leu Val Phe Phe Ile Val Val Gly
        190                 195                 200

gta atc acc atg cta tcc agc ata agc atc gtc atc tct tac gct ttg 675
Val Ile Thr Met Leu Ser Ser Ile Ser Ile Val Ile Ser Tyr Ala Leu
    205                 210                 215

ata ctc tcc aac atc ctc tgt att cct tct gca gag ggc aga tcc aaa 723
Ile Leu Ser Asn Ile Leu Cys Ile Pro Ser Ala Glu Gly Arg Ser Lys
220                 225                 230                 235

gcc ttt agc aca tgg ggc tcc cac ata att gct gtt gct ctg ttt ttt 771
Ala Phe Ser Thr Trp Gly Ser His Ile Ile Ala Val Ala Leu Phe Phe
                240                 245                 250

ggg tca ggg aca ttc acc tac tta aca aca tct ttt cct ggc tct atg 819
Gly Ser Gly Thr Phe Thr Tyr Leu Thr Thr Ser Phe Pro Gly Ser Met
            255                 260                 265

aac cat ggc aga ttt gcc tca gtc ttt tac acc aat gtg gtt ccc atg 867
Asn His Gly Arg Phe Ala Ser Val Phe Tyr Thr Asn Val Val Pro Met
        270                 275                 280

ctt aac cct tcg atc tac agt ttg agg aat aag gat gat aaa ctt gcc 915
Leu Asn Pro Ser Ile Tyr Ser Leu Arg Asn Lys Asp Asp Lys Leu Ala
    285                 290                 295

ctg ggc aaa acc ctg aag aga gtg ctc ttc taatgggtct cttcatatca 965
Leu Gly Lys Thr Leu Lys Arg Val Leu Phe
300                 305

ctggcaaccg a 976

<210> 28
<211> 292

<212> PRT
<213> Homo sapiens

<400> 28
Met Thr Met Glu Asn Tyr Ser Met Ala Ala Gln Phe Val Leu Asp Gly
1               5                   10                  15
Leu Thr Gln Gln Ala Glu Leu Gln Leu Pro Leu Phe Leu Leu Phe Leu
            20                  25                  30
Gly Ile Tyr Val Val Thr Val Val Gly Asn Leu Gly Met Ile Leu Leu
        35                  40                  45
Ile Ala Val Ser Pro Leu Leu His Thr Pro Met Tyr Tyr Phe Leu Ser
    50                  55                  60
Ser Leu Ser Phe Val Asp Phe Cys Tyr Ser Ser Val Ile Thr Pro Lys
65                  70                  75                  80
Met Leu Val Asn Phe Leu Gly Lys Lys Asn Thr Ile Leu Tyr Ser Glu
                85                  90                  95
Cys Met Val Gln Leu Phe Phe Phe Val Val Phe Val Val Ala Glu Gly
            100                 105                 110
Tyr Leu Leu Thr Ala Met Ala Tyr Asp Arg Tyr Val Ala Ile Cys Ser

        115                 120                 125

Pro Leu Leu Tyr Asn Ala Ile Met Ser Ser Trp Val Cys Ser Leu Leu

    130                 135                 140

Val Leu Ala Ala Phe Phe Leu Gly Phe Leu Ser Ala Leu Thr His Thr

145                 150                 155                 160

Ser Ala Met Met Lys Leu Ser Phe Cys Lys Ser His Ile Ile Asn His

                165                 170                 175

Tyr Phe Cys Asp Val Leu Pro Leu Leu Asn Leu Ser Cys Ser Asn Thr

            180                 185                 190

His Leu Asn Glu Leu Leu Leu Phe Ile Ile Ala Gly Phe Asn Thr Leu

        195                 200                 205

Val Pro Thr Leu Ala Val Ala Val Ser Tyr Ala Phe Ile Leu Tyr Ser

    210                 215                 220

Ile Leu His Ile Arg Ser Ser Glu Gly Arg Ser Lys Ala Phe Gly Thr

225                 230                 235                 240

Cys Ser Ser His Leu Met Ala Val Val Ile Phe Phe Gly Ser Ile Thr

                245                 250                 255

Phe Met Tyr Phe Lys Pro Pro Ser Ser Asn Ser Leu Asp Gln Glu Lys
            260                 265                 270
Val Ser Ser Val Phe Tyr Thr Thr Val Ile Pro Met Leu Asn Pro Leu
        275                 280                 285
Ile Tyr Ser Leu
    290

<210> 29
<211> 313
<212> PRT
<213> Homo sapiens

<400> 29

<210> 30

<211> 313

<212> PRT

<213> Homo sapiens

<400> 30

<210> 31
<211> 309
<212> PRT
<213> Homo sapiens

<400> 31
Met Thr Leu Arg Asn Ser Ser Ser Val Thr Glu Phe Ile Leu Val Gly
1               5                   10                  15

Leu Ser Glu Gln Pro Glu Leu Gln Leu Pro Leu Phe Leu Leu Phe Leu
            20                  25                  30

Gly Ile Tyr Val Phe Thr Val Val Gly Asn Leu Gly Leu Ile Thr Leu
        35                  40                  45
Ile Gly Ile Asn Pro Ser Leu His Thr Pro Met Tyr Phe Phe Leu Phe
    50                  55                  60
Asn Leu Ser Phe Ile Asp Leu Cys Tyr Ser Cys Val Phe Thr Pro Lys
65                  70                  75                  80
Met Leu Asn Asp Phe Val Ser Glu Ser Ile Ile Ser Tyr Val Gly Cys
                85                  90                  95
Met Thr Gln Leu Phe Phe Phe Cys Phe Phe Val Asn Ser Glu Cys Tyr
            100                 105                 110
Val Leu Val Ser Met Ala Tyr Asp Arg Tyr Val Ala Ile Cys Asn Pro
        115                 120                 125
Leu Leu Tyr Met Val Thr Met Ser Pro Arg Val Cys Phe Leu Leu Met
    130                 135                 140
Phe Gly Ser Tyr Val Val Gly Phe Ala Gly Ala Met Ala His Thr Gly
145                 150                 155                 160
Ser Met Leu Arg Leu Thr Phe Cys Asp Ser Asn Val Ile Asp His Tyr
                165                 170                 175

Leu Cys Asp Val Leu Pro Leu Leu Gln Leu Ser Cys Thr Ser Thr His
            180                 185                 190
Val Ser Glu Leu Val Phe Phe Ile Val Val Gly Val Ile Thr Met Leu
        195                 200                 205
Ser Ser Ile Ser Ile Val Ile Ser Tyr Ala Leu Ile Leu Ser Asn Ile
    210                 215                 220
Leu Cys Ile Pro Ser Ala Glu Gly Arg Ser Lys Ala Phe Ser Thr Trp
225                 230                 235                 240
Gly Ser His Ile Ile Ala Val Ala Leu Phe Phe Gly Ser Gly Thr Phe
                245                 250                 255
Thr Tyr Leu Thr Thr Ser Phe Pro Gly Ser Met Asn His Gly Arg Phe
            260                 265                 270
Ala Ser Val Phe Tyr Thr Asn Val Val Pro Met Leu Asn Pro Ser Ile
        275                 280                 285
Tyr Ser Leu Arg Asn Lys Asp Asp Lys Leu Ala Leu Gly Lys Thr Leu
    290                 295                 300
Lys Arg Val Leu Phe

305

<210> 32

<211> 762

<212> DNA

<213> Homo sapiens

<220>

<221> CDS

<222> (17)...(760)

<400> 32

aaatgcctaa agaaga atg acc atg gaa aat tat tct atg gca gct cag ttt 52

Met Thr Met Glu Asn Tyr Ser Met Ala Ala Gln Phe

1               5                   10

gtc tta gat ggt tta aca cag caa gca gag ctc cag ctg ccc ctc ttc 100

Val Leu Asp Gly Leu Thr Gln Gln Ala Glu Leu Gln Leu Pro Leu Phe

        15                  20                  25

ctc ctg ttc ctg gga atc tat gtg gtc aca gta gtg ggc aac ctg ggc 148

Leu Leu Phe Leu Gly Ile Tyr Val Val Thr Val Val Gly Asn Leu Gly

    30                  35                  40

atg att ctc ctg att gca gtc agc cct cta ctt cac acc ccc atg tac 196

Met Ile Leu Leu Ile Ala Val Ser Pro Leu Leu His Thr Pro Met Tyr
45                  50                  55                  60

tat ttc ctc agc agc ttg tcc ttc gtc gat ttc tgc tat tcc tct gtc    244
Tyr Phe Leu Ser Ser Leu Ser Phe Val Asp Phe Cys Tyr Ser Ser Val
                65                  70                  75

att act ccc aaa atg ctg gtg aac ttc cta gga aag aag aat aca atc    292
Ile Thr Pro Lys Met Leu Val Asn Phe Leu Gly Lys Lys Asn Thr Ile
            80                  85                  90

ctt tac tct gag tgc atg gtc cag ctc ttt ttc ttt gtg gtc ttt gtg    340
Leu Tyr Ser Glu Cys Met Val Gln Leu Phe Phe Phe Val Val Phe Val
        95                  100                 105

gtg gct gag ggt tac ctc ctg act gcc atg gca tat gat cgc tat gtt    388
Val Ala Glu Gly Tyr Leu Leu Thr Ala Met Ala Tyr Asp Arg Tyr Val
    110                 115                 120

gcc atc tgt agc cca ctg ctt tat aat gcg atc atg tcc tca tgg gtc    436
Ala Ile Cys Ser Pro Leu Leu Tyr Asn Ala Ile Met Ser Ser Trp Val
125                 130                 135                 140

tgc tca ctg cta gtg ctg gct gcc ttc ttc ttg ggc ttt ctc tct gcc    484
Cys Ser Leu Leu Val Leu Ala Ala Phe Phe Leu Gly Phe Leu Ser Ala
                145                 150                 155

ttg act cat aca agt gcc atg atg aaa ctg tcc ttt tgc aaa tcc cac 532
Leu Thr His Thr Ser Ala Met Met Lys Leu Ser Phe Cys Lys Ser His
            160                 165                 170

att atc aac cat tac ttc tgt gat gtt ctt ccc ctc ctc aat ctc tcc 580
Ile Ile Asn His Tyr Phe Cys Asp Val Leu Pro Leu Leu Asn Leu Ser
        175                 180                 185

tgc tcc aac aca cac ctc aat gag ctt cta ctt ttt atc att gcg ggg 628
Cys Ser Asn Thr His Leu Asn Glu Leu Leu Leu Phe Ile Ile Ala Gly
    190                 195                 200

ttt aac acc ttg gtg ccc acc cta gct gtt gct gtc tcc tat gcc ttc 676
Phe Asn Thr Leu Val Pro Thr Leu Ala Val Ala Val Ser Tyr Ala Phe
205                 210                 215                 220

atc ctc tac agc atc ctt cac atc cgc tcc tca gag ggc cgg tcc aaa 724
Ile Leu Tyr Ser Ile Leu His Ile Arg Ser Ser Glu Gly Arg Ser Lys
                225                 230                 235

gct ttt gga aca tgc agc tct cat ctc atg gct gtg gt 762
Ala Phe Gly Thr Cys Ser Ser His Leu Met Ala Val
            240                 245

<210> 33

<211> 248

<212> PRT

<213> Homo sapiens

<400> 33

Met Thr Met Glu Asn Tyr Ser Met Ala Ala Gln Phe Val Leu Asp Gly
1               5                   10                  15

Leu Thr Gln Gln Ala Glu Leu Gln Leu Pro Leu Phe Leu Leu Phe Leu
            20                  25                  30

Gly Ile Tyr Val Val Thr Val Val Gly Asn Leu Gly Met Ile Leu Leu
        35                  40                  45

Ile Ala Val Ser Pro Leu Leu His Thr Pro Met Tyr Tyr Phe Leu Ser
    50                  55                  60

Ser Leu Ser Phe Val Asp Phe Cys Tyr Ser Ser Val Ile Thr Pro Lys
65                  70                  75                  80

Met Leu Val Asn Phe Leu Gly Lys Lys Asn Thr Ile Leu Tyr Ser Glu
                85                  90                  95

Cys Met Val Gln Leu Phe Phe Phe Val Val Phe Val Val Ala Glu Gly
            100                 105                 110

Tyr Leu Leu Thr Ala Met Ala Tyr Asp Arg Tyr Val Ala Ile Cys Ser
        115                 120                 125

Pro Leu Leu Tyr Asn Ala Ile Met Ser Ser Trp Val Cys Ser Leu Leu
    130                 135                 140

Val Leu Ala Ala Phe Phe Leu Gly Phe Leu Ser Ala Leu Thr His Thr
145                 150                 155                 160

Ser Ala Met Met Lys Leu Ser Phe Cys Lys Ser His Ile Ile Asn His
                165                 170                 175

Tyr Phe Cys Asp Val Leu Pro Leu Leu Asn Leu Ser Cys Ser Asn Thr
            180                 185                 190

His Leu Asn Glu Leu Leu Leu Phe Ile Ile Ala Gly Phe Asn Thr Leu
        195                 200                 205

Val Pro Thr Leu Ala Val Ala Val Ser Tyr Ala Phe Ile Leu Tyr Ser
    210                 215                 220

Ile Leu His Ile Arg Ser Ser Glu Gly Arg Ser Lys Ala Phe Gly Thr
225                 230                 235                 240

Cys Ser Ser His Leu Met Ala Val

                245

<210> 34

<211> 27

<212> DNA

<213> Artificial Sequence

<220>

<223> Description of Artificial Sequence: Artificially

synthsized primer sequence

<400> 34

gaagagcagt gagggtccat gttaagg 27

<210> 35

<211> 28

<212> DNA

<213> Artificial Sequence

<220>

<223> Description of Artificial Sequence: Artificially

synthesized primer sequence

<400> 35

cagcagcttg tccttcgtcg atttctgc 28

<210> 36
<211> 29
<212> DNA
<213> Artificial Sequence

<220>
<223> Description of Artificial Sequence: Artificially
      synthesized primer sequence

<400> 36
gctagggtgg gcaccaaggt gttaaaccc 29

<210> 37
<211> 27
<212> DNA
<213> Artificial Sequence

<220>
<223> Description of Artificial Sequence: Artificially
      synthesized primer sequence

<400> 37

tgcaaaagga cagtttcatc atggcac 27

<210> 38

<211> 28

<212> DNA

<213> Artificial Sequence

<220>

<223> Description of Artificial Sequence: Artificially

synthesized primer sequence

<400> 38

caaagaactc acccaaattc ctacagct 28

<210> 39

<211> 27

<212> DNA

<213> Artificial Sequence

<220>

<223> Description of Artificial Sequence: Artificially

synthesized primer sequence

<400> 39

catggtaggc aaccttggct tgatcac 27

<210> 40
<211> 29
<212> DNA
<213> Artificial Sequence

<220>
<223> Description of Artificial Sequence: Artificially
      synthesized primer sequence

<400> 40
gtttattaaa tcacacataa caccatctg 29

<210> 41
<211> 29
<212> DNA
<213> Artificial Sequence

<220>
<223> Description of Artificial Sequence: Artificially
      synthesized primer sequence

<400> 41

cagagacaga gcaatgacat gagagctac 29

<210> 42
<211> 28
<212> DNA
<213> Artificial Sequence

<220>
<223> Description of Artificial Sequence: Artificially
      synthesized primer sequence

<400> 42
caaagaactc acccaaattc ctacagcc 28

<210> 43
<211> 27
<212> DNA
<213> Artificial Sequence

<220>
<223> Description of Artificial Sequence: Artificially
      synthesized primer sequence

<400> 43

catggtaggc aaccttggct tgatcat 27

<210> 44

<211> 29

<212> DNA

<213> Artificial Sequence

<220>

<223> Description of Artificial Sequence: Artificially

synthesized primer sequence

<400> 44

gtttattaaa tcacacataa caccatctg 29

<210> 45

<211> 29

<212> DNA

<213> Artificial Sequence

<220>

<223> Description of Artificial Sequence: Artificially

synthesized primer sequence

<400> 45

cagagacaga gcaatgacat gagagctac                                    29

<210> 46
<211> 27
<212> DNA
<213> Artificial Sequence

<220>
<223> Description of Artificial Sequence: Artificially
      synthesized primer sequence

<400> 46
ccagacagct cgccaagaga gaatgac                                      27

<210> 47
<211> 29
<212> DNA
<213> Artificial Sequence

<220>
<223> Description of Artificial Sequence: Artificially
      synthesized primer sequence

<400> 47

cctttataga tctctgttat tcctgtgtg 29

<210> 48

<211> 27

<212> DNA

<213> Artificial Sequence

<220>

<223> Description of Artificial Sequence: Artificially

synthesized primer sequence

<400> 48

tcggttgcca gtgatatgaa gagaccc 27

<210> 49

<211> 27

<212> DNA

<213> Artificial Sequence

<220>

<223> Description of Artificial Sequence: Artificially

synthesized primer sequence

<400> 49

ggctttggat ctgccctctg cagaagg 27

<210> 50

<211> 450

<212> DNA

<213> Homo sapiens

<400> 50

cagcagcttg tccttcgtcg atttctgcta ttcctctgtc attactccca aaatgctggt 60

gaacttccta ggaaagaaga atacaatcct ttactctgag tgcatggtcc agctcttttt 120

ctttgtggtc tttgtggtgg ctgagggtta cctcctgact gccatggcat atgatcgcta 180

tgttgccatc tgtagcccac tgctttataa tgcgatcatg tcctcatggg tctgctcact 240

gctagtgctg gctgccttct tcttgggctt tctctctgcc ttgactcata caagtgccat 300

gatgaaactg tccttttgca aatcccacat tatcaaccat tacttctgtg atgttcttcc 360

cctcctcaat ctctcctgct ccaacacaca cctcaatgag cttctacttt ttatcattgc 420

ggggtttaac accttggtgc ccaccctagc 450

<210> 51

<211> 637

<212> DNA

<213> Homo sapiens

<400> 51

catggtaggc aaccttggct tgatcactct tttcggtcta aattctcacc tccacacacc 60

aatgtactat ttcctcttca atctctcctt cattgatctc tgttactcct ctgttttcac 120

tcccaaaatg ctaatgaact ttgtgtcaaa aaagaatatt atctccaatg ttgggtgcat 180

gactcggctg tttttctttc tctttttcgt catctctgaa tgttacatgt tgacctcaat 240

ggcatatgat cgctatgtgg ccatctgtaa tccattgctg tataaggtca ccatgtccca 300

tcaggtctgt tctatgctca cttttgctgc ttacataatg ggattggctg gagccacggc 360

ccacaccggg tgcatgttta gactcacctt ctgcagtgct aatatcatta accattactt 420

gtgtgacata ctccccctcc tccagctttc ctgcaccagc acctatgtca acgaggtggt 480

tgttctcatt gttgtgggta ctaatatcac ggtacccagt tgtaccatcc tcatttctta 540

tgttttcatt gtcactagca ttcttcatat caaatccact caaggaagat caaaagcctt 600

cagtacttgt agctctcatg tcattgctct gtctctg 637

<210> 52

<211> 637

<212> DNA

<213> Homo sapiens

<400> 52

catggtaggc aaccttggct tgatcattct tttcggtcta aattctcacc tccacacacc 60

aatgtactat ttcctcttca atctctcctt cattgatctc tgttactcct ctgttttcac 120

tcccaaaatg ctaatgaact ttgtatcaaa aaagaatatt atctcctatg ttgggtgcat 180

gactcagctg tttttctttc tctttttgt catctctgaa tgctacatat tgacctcaat 240

ggcatatgat cgctatgtgg ccatctgtaa tccattgctg tataaggtca ccatgtccca 300

tcaggtctgt tctatgctca cttttgctgc ttacataatg ggattggctg gagccacggc 360

ccacaccggg tgcatgctta gactcacctt ctgcagtgct aatatcatca accattactt 420

gtgtgacata ctccccctcc tccagctttc ctgcaccagc acctatgtca acgaggtggt 480

tgttctcatt gttgtgggta ttaatatcat ggtacccagt tgtaccatcc tcatttctta 540

tgttttcatt gtcactagca ttcttcatat caaatccact caaggaagat caaaagcctt 600

cagtacttgt agctctcatg tcattgctct gtctctg 637

<210> 53
<211> 509
<212> DNA
<213> Homo sapiens

<400> 53
   cctttataga tctctgttat tcctgtgtgt ttacccccaa aatgctgaat gactttgttt 60
   cagaaagtat catctcttat gtgggatgta tgactcagct atttttcttc tgtttctttg 120
   tcaattctga gtgctatgtg ttggtatcaa tggcctatga tcgctatgtg gccatctgca 180
   accccctgct ctacatggtc accatgtccc caagggtctg ctttctgctg atgtttggtt 240
   cctatgtggt agggtttgct ggggccatgg cccacactgg aagcatgctg cgactgacct 300
   tctgtgattc caacgtcatt gaccattatc tgtgtgacgt tctccccctc ttgcagctct 360
   cctgcaccag cacccatgtc agtgagctgg tatttttcat tgttgttgga gtaatcacca 420
   tgctatccag cataagcatc gtcatctctt acgctttgat actctccaac atcctctgta 480
   ttccttctgc agagggcaga tccaaagcc 509

## Description

### Technical Field

The present invention relates to novel G protein-coupled receptor proteins and their genes, as well as methods for producing and using same.

### Background Art

The olfactory receptor (OR) gene family has been known as a multiple gene family encoding odorant receptors that govern the system for olfactory response in the living body. A member of the gene family was first reported to be expressed in rat olfactory neurons in 1991 (Buck L. et al. Cell 65:175-187 (1991)), and subsequently, a number of homologous genes were found across species, in canine (Permentier M. et al. Nature 355:453-455 (1992)), mouse (Ressler K.J. et al. Cell 73:597-609 (1993)), human (Selbie L.A. et al. Mol. Brain Res. 13:159-163 (1992)), catfish (Ngai J. et al. Cell 72:657-666 (1993)), and frog (Freitag J. et al. Neuron 15:1383-1392 (1995)). The receptor proteins encoded by the genes of the family have a unique seven transmembrane structure that is similar to other G protein-coupled receptor subfamilies, and their first extracellular domain contains a common [N-x-S/T] motif (x: any amino acid) which is modified by sugar. Their second and third extracellular domains, loop-1 and loop-2, respectively, are cross-linked by a disulfide bond between the conserved cysteine residues of each. The [M-A-Y-D-R-Y-L/V-A-I/V-C] sequence within the second intracellular domain is another common motif predicted to be functionally important. Especially, the [D-R-Y] motif is suggested to be essential for the binding to intracellular G proteins (Rosenthal W. et al. J. Biol. Chem. 268:13030-13033 (1993); Marchese A. et al. Genomics 23:609-618 (1994)).

Although it is predicted that there exist ligands for the odorant receptor family, such as odour ligands and neuropeptide-like molecules, the exact nature has remained obscure (Buck L. et al. Cell 65:175-187 (1991); Ngai J. et al. Cell 72:657-666 (1993)). Little is known about their signal transduction except for coupling with G proteins, and thus functional analysis of the gene family is desired. Recently, it has been proposed to classify the entire gene family into eight subfamilies according to structural similarity. However, it is unlikely that the classification itself can reflect functional similarity among the genes. Inhuman, it is assumed that approximately one thousand copies of this gene family exists. However, about 70% of the copies are assumed to be pseudo genes (Sylvie R. et al. Nature Gen. 18:243-250 (1998)).

### Disclosure of the Invention

The present invention provides novel G protein-coupled receptor proteins and their genes, as well as materials and methods for producing them, molecules used for the method and such, and their use.

The present inventors performed BLAST search of the GenBank public database and found multiple human genome sequences that encode novel seven transmembrane G protein-coupled receptor genes. The inventors carried out RT-PCR using oligonucleotide primers that were designed based on the above genome sequences and mRNA prepared from human tissues as a template, and obtained partial cDNA sequences of the target genes. This RT-PCR was also used to evaluate the expression pattern and profile of the genes in those tissues. Then, the inventors carried out 5'- and 3'- RACE using a cDNA library prepared from human testis as a template, in which the genes were highly expressed based on the result of RT-PCR, and succeeded in isolating the full length cDNA. The first three genes isolated, originating from human chromosome 14, are designated as GTAR14-1, GTAR14-3, and GTAR14-5, and the next four genes isolated, originating from human chromosome 11, are designated as GTAR11-1, GTAR11-2, GTAR11-3, and GTAR11-4.

These genes encode novel seven transmembrane G protein-coupled receptors, and fulfill the characteristics of the known olfactory receptor family genes. The result of the analysis of gene expression in human tissues showed that GTAR14-1 was detected strongly in lymphatic/hematopoietic tissues, including thymus and spleen, testis, and pancreas, and was also expressed in fetal thymus. GTAR14-3 was detected strongly in thymus and testis, but was also expressed widely in multiple tissues, including digestive tissues such as small intestine and colon, and hormone secreting tissues such as placenta and prostate. GTAR14-5 was detected strongly in lymphatic/hematopoietic tissues, such as thymus, spleen, and peripheral leukocytes, and testis. Its expression was intensified in fetal thymus, fetal spleen, and fetal lung, and was detected weakly in fetal brain and fetal kidney. GTAR11-1 was detected strongly in lymphatic/hematopoietic tissues including thymus, spleen, and peripheral lymphocytes, digestive tissues such as small intestine and colon, and testis and placenta. GTAR11-2 was detected strongly in lymphatic/hematopoietic tissues including thymus and peripheral lymphocytes, and testis and placenta. GTAR11-3 was detected strongly in lymphatic/hematopoietic tissues including thymus and spleen, and genital organs, and was also detected broadly in multiple tissues including digestive tissues such as small intestine and colon, muscle, and neuronal tissues. GTAR11-4 was detected strongly in lymphatic/hematopoietic tissues including thymus and spleen, and genital organs, and was also extensively expressed in multiple tissues including digestive tissues such as small intestine and colon, and endocrine tissues.

These expression profiles suggest that proteins encoded by those genes have an important function in lymphatic/hematopoietic and endocrine tissues. Consequently, it was considered that the proteins can be used for screening a novel hematopoietic peptide factor or a novel hormone-like peptide factor.

The present invention relates to novel G protein-coupled receptor proteins and their genes, and their production and use as well. More specifically, it provides:
(1) a G protein-coupled receptor protein selected from the group consisting of:
   (a) a protein having an amino acid sequence selected from the group consisting of SEQ ID NO: 4, 5, 6, 28, 29, 30, and 31;
   (b) a protein of (a), wherein one or more amino acids are modified by deletion, addition, insertion, and/or substitution by another amino acid residue; and
   (c) a protein encoded by DNA that hybridizes with DNA having a nucleotide sequence selected from the group consisting of SEQ ID NO: 1, 2, 3, 24, 25, 26, and 27;
(2) a fusion protein comprising the protein of (1) and another peptide or polypeptide;
(3) a peptide comprising a part of the protein of (1);
(4) a DNA encoding the protein or peptide of any of (1) to (3);
(5) a vector comprising the DNA of (4) inserted therein;
(6) a transformant carrying the DNA of (4) in an expressible manner;
(7) a method of producing the protein or peptide of any of (1) to (3), comprising the steps of: cultivating the transformant of (6), and recovering the protein or peptide expressed therein;
(8) a method of screening for a compound that is capable of binding to the protein of (1), comprising the steps of:
   (a) exposing a test sample to the protein or peptide of any of (1) to (3); and
   (b) selecting the compound that binds to the protein or peptide of any of (1) to (3);
(9) a method of screening for a ligand and/or agonist that is capable of binding to the protein of (1), comprising the steps of:
   (a) exposing a test sample to a cell expressing the protein or peptide of any of (1) to (3) on its surface;
   (b) measuring a biochemical change in said cell; and
   (c) selecting the compound that induces said biochemical change in said cell;
(10) an antibody that binds to the protein of (1);
(11) a method of detecting or measuring the protein or peptide of any of (1) to (3), comprising the steps of: exposing the antibody of (10) to a sample which is assumed to comprise said protein or peptide, and detecting or measuring the generation of an immune complex between said antibody and said protein or peptide; and
(12) A DNA of a length of 15 nucleotides or longer that hybridizes with a DNA having a nucleotide sequence selected from the group consisting of SEQ ID NO: 1, 2, 3, 24, 25, 26, 27, and their complementary strands.

The nucleotide sequences of GTAR14-1, GTAR14-3, and GTAR14-5 cDNAs that were isolated by the present inventors, and the amino acid sequences of the proteins encoded by the cDNAs are shown in SEQ ID NO: 1, 2, 3, 4, 5, and 6, respectively. The nucleotide sequences of GTAR11-1, GTAR11-2, GTAR11-3, and GTAR11-4 cDNAs that were also isolated by the present inventors, and the amino acid sequences of the proteins encoded by the cDNAs are shown in SEQ ID NOs: 24, 25, 26, 27, 28, 29, 30, and 31, respectively.

The receptor proteins of the OR gene family have a seven transmembrane type structure like other G protein-coupled receptors, and their first extracellular domains have a [N-x-S/T] motif (x: any amino acid) in common that is modified by sugar. There are conserved cysteine residues in the second (loop-1) and third (loop-2) extracellular domains that form a disulfide bond for cross-linking. Furthermore, there is a [M-A-Y-D-R-Y-L/V-A-I/V-C] sequence (especially [D-R-Y] motif) in the second intracellular domain, which is suggested to be essential for binding to the G protein (Rosenthal W. et al. J. Biol. Chem. 268:13030-13033 (1993); Marchese A. et al. Genomics 23:609-618 (1994)).

The seven proteins encoded by the seven genes isolated by the present inventors (designated as GTAR proteins below) are cell membrane proteins and are seven transmembrane receptors that contain seven hydrophobic transmembrane domains.

GTAR proteins, like known odorant receptors, have the above described motifs characteristic to G protein-coupled receptors. The [N-x-S/T] motif (x: any amino acid) modified by sugar is found within the first extracellular domain as [N-Q-T], [N-S-T], and [N-T-S] in GTAR14-1, GTAR14-3, and GTAR14-5 proteins, respectively, as [N-Y-S] in GTAR11-1 protein, and as [N-S-T] in GTAR11-2, GTAR11-3, and GTAR11-4 proteins.

GTAR proteins have conserved cysteine residues in the second and third extracellular domains (loop-1 and loop-2, respectively). Furthermore, they have the [M-A-Y-D-R-Y-L/V-A-I/V-C] sequence in the second intracellular domain, which is suggested to be essential for binding to G protein, as well: [V-A-Y-D-R-Y-V-A-I-C] in GTAR14-1 protein, and [M-A-Y-D-R-Y-L-A-I-C] in all the others.

These features suggest that GTAR proteins of the present invention, like other G protein-coupled receptor proteins, function through binding to intracellular G proteins in response to the stimuli of extracellular ligands.

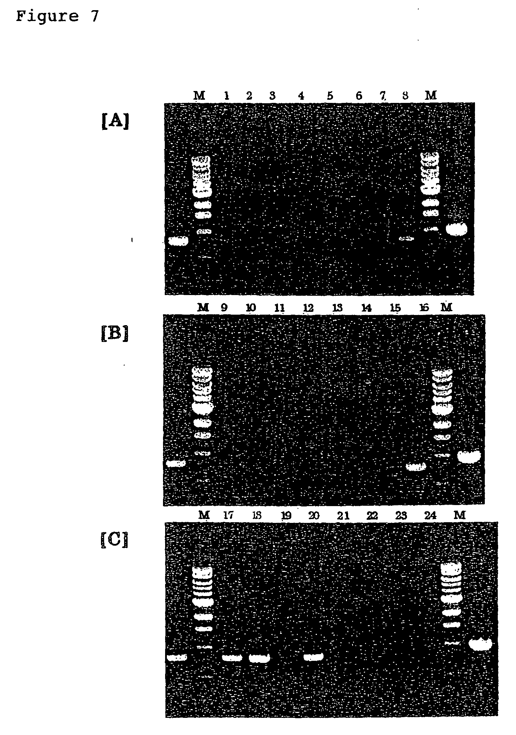

Furthermore, the result of RT-PCR analysis revealed a strong expression of GTAR genes in thymus and testis (See, for example, Example 1(3) and 2(3), Figures 7 to 9, and Figures 20 to 23). This suggests that the GTAR proteins of the present invention may regulate the differentiation, or proliferation and activation of a particular group of lymphatic cells. Thus, GTAR proteins could function as a central molecule that controls the activities characteristic to lymphocytes through interaction with G proteins in response to ligand stimuli. As these proteins are expressed in testis, where cells are changing continuously in the cell cycle at a high turn over rate, it is possible that they regulate cell cycle and cell division.

On the other hand, the expression of GTAR14-1 gene was detected in spleen, where hematopoietic cells are expected to exist. The GTAR14-5 gene was also expressed in spleen and peripheral leukocytes, where hematopoietic cells are thought to be contained. The GTAR11 genes were expressed in peripheral leukocytes, in which hematopoietic cells are considered to be included, and furthermore the genes for GTAR11-1, GTAR11-3, and GTAR11-4 were expressed in spleen, which is expected to have hematopoietic cells. These suggest that GTAR proteins, particularly GTAR14-1, GTAR14-5, and GTAR11 proteins, which have structural similarities to known odorant receptors, could function as a receptor for a novel hematopoietic regulator.

The GTAR14-3 gene was expressed in thymus and testis as described above, and also in digestive tissues, including small intestine and colon, and endocrine organs, such as placenta and prostate. Moreover, the result of RT-PCR using mRNA prepared from human fetal tissues showed a broad distribution pattern of GTAR14-3 gene expression, with strong expression in thymus, brain, heart, smooth muscle, and kidney, and weak expression in fetal liver and fetal lung. This not only suggests that GTAR14-3 protein can function as a receptor of a novel hematopoietic factor, but also strongly suggests that the protein could regulate diverse physiological functions as a receptor of a novel hormone-like peptide molecule.

The GTAR proteins of the present invention, which can play a central role in the function of immune responsive cells, may find utility in clinical treatment of the following field of diseases.

First, it is possible to enhance the cellular immune system of the living body by promoting the activity of GTAR proteins through *in vivo* administration of (1) a ligand or agonist derived from the living body that is capable of functionally binding to GTAR proteins, or (2) a specific antibody that can activate the function of GTAR proteins. Such substances that are capable of functionally binding to GTAR proteins can be clinically applied as a growth promoting factor, a differentiation inducing factor, or an activator of immune cell functions in immune responsive cells, especially in the group of T-cells. This means that they can promote *in vivo* immune response, including anti tumor immunity and anti infection immunity. More specifically, it is possible to enhance cytotoxic immunity toward a particular type of tumor tissue, and also increase resistance against a variety of viruses, including but not limited to HCV and HIV, by enhancing anti infection immunity in the living body.

Next, it is possible to suppress the cellular immunity in the living body by promoting the inhibition of the activity of GTAR proteins through *in vivo* administration of (1) an antagonist or inhibitor that is capable of functionally binding to GTAR proteins, or (2) a specific antibody that can inhibit the function of GTAR proteins. Such substances that are capable of functionally binding to GTAR proteins can be clinically applied as a growth inhibiting factor, a differentiation inhibiting factor, or a suppressor of immune cell functions of immune responsive cells, especially of the group of T-cells. Thus, they can suppress autoimmune diseases resulting from self damaging, and rejection reactions accompanying tissue transplantation, a most critical issue in transplantation immunity. More specifically, they are expected to be very effective for diseases including, but not limited to, diabetes, liver damage, collagen arthritis, GVHD, and EAE, all of which are induced by abnormal activation of immune response. Immunosuppression by the inhibitors could also be effective for a variety of antigen specific allergies, such as metal allergy and pollen allergy.

The present invention further provides proteins that are functionally equivalent to the GTAR proteins of the present invention. Herein, "functionally equivalent" means that a protein has an equivalent biological activity to that of GTAR proteins. An illustrative biological activity is the ability of GTAR to act as a G protein-coupled receptor protein, which is an activity to interact with intracellular G proteins in response to ligand stimuli and transduce signals from outside to inside of cells.

A functionally equivalent protein may be obtained by introducing a mutation into the amino acid sequence of the protein. For instance, site-directed mutagenesis using synthetic oligonucleotide primers may be used to introduce a desired mutation (Kramer W. and Fritz H.J. Methods Enzymol. 154:350-367 (1987)). PCR mediated site-directed mutagenesis system (GIBCO-BRL) may also be used for introducing mutations into amino acid sequences of proteins. By using these methods, it is possible to obtain a functionally equivalent protein to GTAR proteins having an amino acid sequence of a GTAR protein (i.e., SEQ ID NO: 4 to 6, and SEQ ID NO: 28 to 31) in which one or more amino acids are deleted, added, and/or substituted by other amino acid residues without affecting their biological activities.

More specifically, a functionally equivalent protein to GTAR proteins may have a sequence in which one or more, preferably two or more but not more than 30, more preferably two or more but not more than 10 amino acid residues are deleted from the amino acid sequence of SEQ ID NOs: 4 to 6 or SEQ ID NOs: 28 to 31. A functionally equivalent protein to a GTAR protein of the present invention may have a sequence in which one or more, preferably two or more but not more than 30, more preferably two or more but not more than 10 amino acid residues are added to the amino acid sequence of SEQ ID NOs: 4 to 6 or SEQ ID NOs: 28 to 31. A functionally equivalent protein to a GTAR protein of the present invention may have a sequence in which one or more, preferably two or more but not more than 30, more preferably two or more but not more than 10 amino acid residues are substituted by other amino acids in the amino acid sequence of SEQ ID NOs: 4 to 6 or SEQ ID NOs: 28 to 31.

It is well known that a protein having deletion, addition, and/or substitution of one or more amino acid residues in the sequence of a protein can retain the original biological activity (Mark D.F. et al. Proc. Natl. Acad. Sci. U.S.A. 81:5662-5666 (1984); Zoller M.J. and Smith M. Nucleic Acids Res. 10:6487-6500 (1982); Wang A. et al. Science 224:1431-1433; Dalbadie-McFarland G. et al. Proc. Natl. Acad. Sci. U.S.A. 79:6409-6413 (1982)).

For instance, a protein in which one or more amino acid residues are added to GTAR proteins may be a fusion protein comprising GTAR proteins. The present invention provides for a fusion protein, wherein one or more GTAR proteins and one or more of another protein or peptide are fused. Conventional methods may be used to generate a fusion protein of the present invention. For example, a DNA encoding the GTAR protein and a DNA encoding the other protein or peptide are ligated so as to match their ORFs in the same frame, and introduced into an expression vector. The resulting fusion protein is then expressed in a host cell. The protein or peptide to be fused to the GTAR protein of the present invention is not limited to any specific protein or peptide.

For instance, known peptides including FLAG peptide (Hopp T.P. et al. BioTechnology 6:1204-1210 (1988)), 6xHis that is made up of six histidine residues, 10xHis, influenza hemagglutinin (HA), human c-myc fragment, VSV-GP fragment, p18HIV fragment, T7-tag, HSV-tag, E-tag, SV40 T antigen fragment, lck tag, α-tubulin fragment, B-tag, and Protein C fragment may be used for fusion. Also, glutathione-S-transferase (GST), influenza hemagglutinin (HA), the constant region of immunoglobulin, β-galactosidase, maltose binding protein (MBP), and the like may be used as a protein to be fused. DNA encoding these proteins are known in the art and/or commercially available.

The present invention also provides proteins encoded by DNA that hybridizes with DNA having a nucleotide sequence of SEQ ID NOs: 1 to 3 or 24 to 27 under stringent conditions, and is functionally equivalent to GTAR proteins. Stringent conditions may be appropriately chosen by a skilled artisan, and include low stringent conditions. Low stringent conditions may be, for example, at 42°C in 2x SSC and 0.1% SDS, preferably at 50°C in 2x SSC and 0.1% SDS. More preferably, high stringent conditions such as at 65°C in 2x SSC and 0.1% SDS may be chosen. DNA with higher homology can be obtained at higher temperature under these conditions.

Furthermore, the present invention provides proteins that are functionally equivalent to GTAR proteins and homologous to the amino acid sequence of the proteins (SEQ ID NOs: 4 to 6 and 28 to 31). In the context of the present invention, a protein is "homologous" provided it has at least 70% homology, preferably 80% or higher, more preferably 90% or higher, and even more preferably 95% or higher homology to an amino acid sequence of SEQ ID NOs: 4 to 6 or 28 to 31. The degree of homology between two proteins can be determined by using. an algorithm described in the literature (Wilbur W.J. and Lipman D.J. Proc. Natl. Acad. Sci. U.S.A. 80:726-730 (1983)).

A protein of the present invention may have variations in amino acid sequence, molecular weight, isoelectric point, the presence or absence of sugar chains, or form, depending on the cell or host used to produce it or the purification method utilized . Nevertheless, as long as it has an activity as a G protein-coupled receptor, it is within the scope of the present invention.

A protein of this invention may be produced by inserting the obtained DNA into an expression vector and expressing under the regulation of an expression regulating region, such as an enhancer or promoter. The expression vector is used to transform a host cell, where the protein is expressed.

More specifically, for expression in mammalian host cells, one may construct a DNA or a vector in which a standard useful promoter/enhancer, a DNA encoding a protein of the invention, and a polyA signal at the 3' downstream are functionally integrated. For instance, human cytomegalovirus immediate early promoter/enhancer may be used as a promoter/enhancer.

Also, virus promoter/enhancers such as those of retrovirus, polyomavirus, adenovirus, and simian virus 40 (SV40), or mammalian promoter/enhancers such as that of human elongation factor 1α (HEF1α) may be used as a promoter/enhancer for protein expression.

For instance, the SV40 promoter/enhancer may be readily used according to the method of Mulligan et al. (Nature 277:108 (1979)), and HEF1α promoter/enhancer may be readily used following the method of Mizushima et al. (Nucleic Acids Res. 18:5322 (1990)).

For expression in *E*. *coli,* a DNA may be constructed in which a standard useful promoter, a signal sequence for polypeptide secretion, and the gene to be expressed are functionally integrated. The promoter may be, for example, the LacZ promoter, the araB promoter, and the like. The LacZ promoter may be used according to the method of Ward et al. (Nature 341:544-546 (1998); FASEB J. 6:2422-2427 (1992)). The araB promoter may be used following the method of Better et al. (Science 240:1041-1043 (1988)).

For producing the protein in the periplasm of *E*. *coli*, the pelB signal sequence (Lei S.P. et al. J. Bacteriol. 169:4379 (1987)) may be used as a signal sequence.

The origin used for replication may be those of SV40, polyomavirus, adenovirus, bovine papilloma virus (BPV), and the like. In addition, the expression vector may include a selection marker gene for amplification of the gene copies in host cells. Examples of such markers include, but are not limited to, the aminoglycoside transferase (APH) gene, the thymidine kinase (TK) gene, the *E*. *coli* xanthine-guanine phosphoribosyl transferase (Ecogpt) gene, and the dihydrofolate reductase (dhfr) gene..

The expression vector for production of the protein of the invention is not limited to any specific one as long as it is suitably used for the purpose. For example, the expression vector may be a mammal-derived expression vector (e.g., pEF and pCDM8), an insect cell-derived expression vector (e.g., pBacPAK8)., a plant-derived expression vector (e.g., pMH1 and pMH2), an animal virus-derived expression vector (e.g., pHSV, pMV, and pAdexLcw), a retrovirus-derived expression vector (e.g., pZIpneo), an yeast-derived expression vector (e.g., pNV11 and SP-Q01), a *Bacillus* subtilis-derived expression vector (e.g., pPL608 and pKTH50), or an *E*. *coli*-derived expression vector (e.g., pQE, pGEAPP, pGEMEAPP, and pMALp2).

The vector of the present invention not only may be used for producing the protein of the invention in vivo or *in vitro,* but also may be used for gene therapy in mammals including humans.

Known methods, such as calcium phosphate method (Virology 52:456-467 (1973)) and electroporation (EMBO J. 1:841-845 (1982)), may be used to introduce the expression vector constructed above into a host cells.

Any production system can be used herein for producing proteins. The present invention provides methods of producing a protein of the invention both *in vitro* or *in vivo.*

For *in vitro* production, eukaryotic cells or prokaryotic cells can be used. Useful eukaryotic cells may be animal, plant, or fungi cells. As animal cells, mammalian cells such as CHO (J. Exp. Med. 108:945 (1995)), COS, myeloma, baby hamster kidney (BHK), HeLa, or Vero cells, amphibian cells such as Xenopus oocytes (Valle et al. Nature 291:340-358 (1981)), or insect cells such as sf9, sf21, or Tn5 cells can be used. CHO cells lacking DHFR gene (dhfr-CHO) (Proc. Natl. Acad. Sci. U.S.A. 77:4216-4220 (1980)) or CHO K-1 (Proc. Natl. Acad. Sci. U.S.A. 60:1275 (1968)) may also be used.

As plant cells, plant cells originating from *Nicotiana tabacum* are known and may be used as callus cultures. As fungi cells, yeast cells such as *Saccharomyces,* including *Saccharomyces cerevisiae,* or filamentous fungi such as *Aspergillus,* including *Aspergillus niger,* are known and may be used herein.

Useful prokaryotic cells include bacterial cells, such as *E. coli* or *Bacillus subtilis.*

These cells are transformed by a desired DNA, and the resulting transformants are cultured *in vitro* to obtain the protein. Transformants can be cultured using known methods. Culture medium such as DMEM, MEM, RPMI1640, or IMDM may be used with or without serum supplement such as fetal calf serum (FCS). The pH of the culture medium is preferably between about 6 and 8. Such cells are typically cultured at about 30 to 40 °C for about 15 to 200 hr, and the culture medium may be replaced, aerated, or stirred if necessary.

Animal and plant hosts may be used for *in vivo* production. For example, a desired DNA can be introduced into an animal or plant host. Encoded proteins are produced in *vivo,* and then recovered. These animal and plant hosts are included in host cells of the present invention.

Animals to be used for the production system described above include, but are not limited to, mammals and insects. Mammals such as goat, porcine, sheep, mouse, and bovine may be used (Vicki Glaser, SPECTRUM Biotechnology Applications (1993)). Alternatively, the mammals may be transgenic animals.

For instance, a desired DNA may be prepared as a fusion gene by inserting it into the middle of a gene, such as goat β casein gene which encodes a protein specifically produced into milk. DNA fragments comprising the fusion gene having the desired DNA are injected into goat embryos, which are then introduced back to female goats. Proteins are recovered from milk produced by the transgenic goats (i.e., those born from the goats that had received the modified embryos) or from their offspring. To increase the amount of milk containing the proteins produced by transgenic goats, appropriate hormones may be administered to them (Ebert K.M. et al. Bio/Technology 12:699-702 (1994)).

Alternatively, insects, such as the silkworm, may be used. A desired DNA inserted into baculovirus can be used to infect silkworms, and the desired protein is recovered from their body fluid (Susumu M. et al. Nature 315:592-594 (1985)).

As plants, for example, tobacco can be used. In use of tobacco, a desired DNA may be inserted into a plant expression vector, such as pMON530, which is introduced into a bacteria, such as *Agrobacterium tumefaciens.* Then the bacteria is used to infect tobacco, such as *Nicotiana tabacum,* and a desired polypeptide is recovered from their leaves (Julian K.-C. Ma et al. Eur. J. Immunol. 24:131-138 (1994)).

A protein of the present invention obtained as above may be isolated from inside or outside of cells or hosts, and purified as a substantially pure homogeneous protein. The method for protein isolation and purification is not limited to any specific method; in fact, any standard method may be used. For instance, column chromatography, filter, ultrafiltration, salt precipitation, solvent precipitation, solvent extraction, distillation, immunoprecipitation, SDS-polyacrylamide gel electrophoresis, isoelectric point electrophoresis, dialysis, and recrystallization may be appropriately selected and combined to isolate and purify the protein.

For chromatography, for example, affinity chromatography, ion-exchange chromatography, hydrophobic chromatography, gel filtration, reverse phase chromatography, adsorption chromatography, and such may be used (Strategies for Protein Purification and Characterization: A Laboratory Course Manual. Ed. Daniel R. Marshak et al., Cold Spring Harbor Laboratory Press (1996)). These chromatographies may be performed by liquid chromatography such as HPLC and FPLC. Thus, the present invention provides for highly purified proteins, produced by the above methods .

A protein of the present invention may be optionally modified or partially deleted by treating it with an appropriate protein modification enzyme before or after purification. Useful protein modification enzymes include, but are not limited to, trypsin, chymotrypsin, lysylendopeptidase, protein kinase, and glucosidase.

The present invention also provides partial peptides of the GTAR proteins of the present invention (i.e., SEQ ID NOs: 4 to 6 and 28 to 31). A partial peptide may be, for example, a peptide which is one of the partial peptide sequences encoded by GTAR genes, and corresponds to the binding domain to an *in vivo* ligand. Such a partial peptide, when administered *in vivo*, may bind to the ligand competitively, and inhibit the binding between the GTAR protein and the ligand. Similarly, a partial peptide corresponding to the G-protein binding domain of a GTAR protein may antagonistically inhibit the binding between GTAR protein and intracellular G protein. Such partial peptides may be useful as inhibitors of signal transduction pathways mediated by GTAR proteins.

A partial peptide of the protein of the invention can be produced by genetic engineering, known methods of peptide synthesis, or by digesting the protein of the invention with an appropriate peptidase. For peptide synthesis, for example, solid phase synthesis or liquid phase synthesis may be used.

Furthermore, the present invention relates to a DNA encoding the protein of the present invention as described. A cDNA encoding the protein may be obtained by, for example, screening a human cDNA library using one or more probes described herein.

A cDNA may also be obtained from different cells, tissues, organs, or species by further screening a cDNA library using the obtained cDNA or cDNA fragment as a probe. A cDNA library may be prepared, for example, by the method described in literature (Sambrook J. et al. Molecular Cloning, Cold Spring Harbor Laboratory Press (1989)), or may be commercially available.

The nucleotide sequence of the obtained cDNA is determined to find an open reading frame, and thereby the amino acid sequence of the protein of the invention can be obtained. The cDNA obtained may also be used as a probe for screening a genomic library to isolate a genomic DNA.

More specifically, mRNAs may first be prepared from a cell, tissue, or organ in which the protein of the invention is expressed. Known methods can be used to isolate mRNAs; for instance, total RNA is prepared by guanidine ultracentrifugation (Chirgwin J.M. et al. Biochemistry 18:5294-5299 (1979)) or AGPC method (Chomczynski P. and Sacchi N. Anal. Biochem. 162:156-159 (1987)), and mRNA is purified from total RNA using mRNA Purification Kit (Pharmacia) and such. Alternatively, mRNA may be directly purified by QuickPrep mRNA Purification Kit (Pharmacia).

The obtained mRNA is used to synthesize cDNA using reverse transcriptase. cDNA may be synthesized by using a kit such as the AMV Reverse Transcriptase First-strand cDNA Synthesis Kit (Seikagaku Kogyo). Alternatively, cDNA may be synthesized and amplified following the 5'-RACE method(Frohman M.A. et al. Proc. Natl. Acad. Sci. U.S.A. 85:8998-9002 (1988); Belyavsky A. et al. Nucleic Acids Res. 17:2919-2932 (1989)) which uses a probe described herein, the 5'-Ampli FINDER RACE Kit (Clontech), and polymerase chain reaction (PCR).

A desired DNA fragment is prepared from the PCR products and ligated with a vector DNA. The recombinant vectors are used to transform *E*. *coli* and such, and a desired recombinant vector is prepared from a selected colony. The nucleotide sequence of the desired DNA is verified by conventional methods, such as dideoxynucleotide chain termination.

A DNA of the invention may be designed to have a sequence that is expressed more efficiently by taking into account the frequency of codon usage in the host to be used for expression (Grantham R. et al. Nucleic Acids Res. 9:43-74 (1981)). The DNA of the present invention may be altered by a commercially available kit or a conventional method. For instance, the DNA may be altered by digestion with restriction enzymes, insertion of a synthetic oligonucleotide or an appropriate DNA fragment, addition of a linker, or insertion of the initiation codon (ATG) and/or the stop codon (TAA, TGA, or TAG).

The present invention particularly provides DNA having the following nucleotide sequences: from A at 9 to C at 947 of SEQ ID NO: 1, from A at 13 to T at 951 of SEQ ID NO: 2, from A at 410 to T at 1339 of SEQ ID NO: 3, from A at 17 to G at 892 of SEQ ID NO: 24, from A at 18 to C at 956 of SEQ ID NO: 25, from A at 18 to C at 956 of SEQ ID NO: 26, or from A at 19 to C at 945 of SEQ ID NO: 27.

Furthermore, the present invention provides DNA that is capable of hybridizing with DNA having a nucleotide sequence of SEQ ID NOs: 1 to 3 or 24 to 27 under stringent conditions, and encoding a protein functionally equivalent to the protein of the invention described above. Stringent conditions may be appropriately chosen by one skilled in the art. For instance, low stringent conditions may be 42°C in 2xSSC and 0.1% SDS, and preferably 50°C in 2xSSC and 0.1% SDS. More preferably, high stringent conditions such as 65°C in 2xSSC and 0.1% SDS may be chosen. Under the conditions, DNAs having higher homologies can be obtained with increasing temperature. The hybridizing DNA above is preferably a cDNA or a chromosomal DNA.

The protein of the invention may be used for screening a compound that is capable of binding to the protein, such compound beinguseful as a drug, or the like. Specifically, the protein may be used in a method of screening the compound, such method comprising the steps of exposing the protein of the present invention to a test sample in which a compound binding to the protein is expected to be contained, and selecting the compound having the activity of binding to the protein.

The proteins of the invention used for screening may be recombinant or wild type proteins, or partial peptides. Alternatively, they may be purified proteins or partial peptides, or in the form expressed on the surface of cells or in the form of a membrane fraction.

A compound having the activity of binding to the protein of the invention may be a natural or artificially synthesized compound. A natural compound may be, for example, a ligand binding to the protein of the invention. A ligand herein is defined as a natural compound that binds to a protein of the invention and induces a specific physiological reaction in cells expressing the protein. The physiological reaction includes, but is not limited to, biochemical changes such as production of cAMP, change in the concentration of intracellular calcium, or activation of protein kinase C, as described below.

Screening for a protein which is capable of binding to the protein of the invention may be carried out by, for example, using the West-Western blotting method (SkolnikE.Y. et al. Cell 65:83-90 (1991)). Specifically, cDNAs are isolated from cells, tissues, or organs in which the proteins binding to the protein of the present invention is expected to be expressed, and introduced into a phage vector such as λgt11 or ZAPII to construct a cDNA library. The phage vectors of the library are spread on plates containing medium, and proteins are expressed. The proteins expressed are transferred to a filter membrane, which is reacted with a labeled protein of the invention and then purified. The plaques expressing those proteins that bound to the protein of the invention can be identified by detecting the label. The protein of the invention may be labeled by a method utilizing the binding between biotin and avidin, or a method utilizing an antibody that specifically binds to the protein of the present invention, or a peptide or polypeptide that is fused to the protein of the present invention. Methods using radioisotope or fluorescence and such may also be used.

Alternatively, in another embodiment of the method of screening of the present invention, a two-hybrid system utilizing cells may be used (Fields S. and Sternglanz R. Trends Genet. 10:286-292 (1994)).

The two-hybrid system can be used as follows. A firstexpression vector, comprising a DNA encoding a fusion protein of the present invention and a subunit of a transcription factor that forms a heterodimer, and a second expression vector, comprising a desired cDNA to be tested ligated to a DNA encoding the other subunit of the transcription factor, are introduced into cells and induced. A reporter gene that was integrated into the cells is expressed only if the protein of the invention and the protein encoded by a desired cDNA bind to each other and when the transcription factors form a heterodimer. Thus, a protein that is capable of binding to a protein of the invention can be selected by detecting or measuring the expression level of the reporter gene.

More specifically, a DNA encoding a protein of the invention and a gene encoding the DNA binding domain of LexA are ligated in frame to construct an expression vector. Next, the desired cDNA and a gene encoding the GAL4 transcription activation domain are ligated to obtain another expression vector.

Cells carrying the HIS3 gene under the expressional regulation of a promoter containing a LexA binding motif are transformed with the above two expression plasmids of the two-hybrid system, and incubated in a synthetic medium lacking histidine. Cells can grow only if there is a protein-protein interaction. Thus, the expression level of the reporter gene is examined by the growth rate of transformants.

Alternatively, instead of HIS3 gene, the reporter gene may be a luciferase gene, a plasminogen activator inhibitor typel (PAI-1) gene, or else.

The two-hybrid system may be constructed by a standard method, or a commercial kit such as MATCHMAKER Two-Hybrid System, Mammalian MATCHMAKER Two-Hybrid Assay Kit (both from Clontech), or HybriZAP Two-Hybrid Vector System (Stratagene) may be used.

In another embodiment, the method for screening of the present invention utilizes affinity chromatography. The protein of the invention is immobilized on a carrier of an affinity column, and a test sample, in which a protein capable of binding to the protein of the invention is supposed to be expressed, is applied to the column. A test sample herein may be cell culture supernatant, cell extracts, cell lysates, etc. After loading the test sample, the column is washed, and proteins bound to the protein of the invention can be obtained.

Alternatively, a compound that binds to the protein of the invention may be screened by stably expressing the protein of the present invention on the surface of an appropriate host cell, exposing the cell to a sample which is assumed to contain a ligand, and detecting a biochemical change induced in the cell. The host cell may be one of a variety of mammalian cells, but preferably it is a cell which naturally express the protein of the invention, for example, the immune cells. Different biochemical changes may be used as an index, depending on the type of G proteins that bind to the protein of the invention. Examples include, but are not limited to, cAMP production, change in intracellular calcium concentration, and activation of protein kinase C. Alternatively, as a more generally used index, micro extracellular pH change can be measured by a microphysiometer (for instance, "Cytosensor^{R}", Molecular Device Inc.).

Samples to be tested in the method for screening of the present invention include, but are not limited to, peptides, purified or crude preparation of proteins, non-peptide compounds, synthetic compounds, products of microorganism fermentation, cell extracts, animal tissue extracts, marine organism extracts, and plant extracts.

Compounds isolated by the above method for screening may be a candidate for a drug that can promote or inhibit the activity of the protein of the invention and thus beuseful in the treatment of a disease caused by the abnormal function of a receptor protein of the invention. That part of the compound having the activity to bind to the protein of the present invention obtained by the method for screening of the present invention may be structurally changed by addition, deletion, and/or substitution. Such compound parts or fragments are also within the scope of the present invention.

It is possible to screen an agonist or antagonist of a receptor protein of the invention by using as an index the biochemical changes described above or the activity inhibiting the binding between the receptor protein and its ligand. Herein, an agonist is defined herein as a compound that is not derived from nature but is capable of binding to the protein(s) of the invention and inducing a physiological reaction in cells in a manner analogous to that of a ligand. Likewise, an antagonist is defined herein as a compound that inhibits the function of a ligand or agonist toward the protein(s) of the invention and includes both natural and unnatural compounds.

In a method in which an intracellular biochemical change is used as an index, for example, the above-described cells stably expressing a protein of the invention may be exposed to a test sample in the presence of a ligand, and the above-described biochemical index, such as cAMP production, change of intracellular calcium concentration, or activation of protein kinase C, may be measured. If inhibition is seen in the biochemical index, the compound contained in the test sample is judged to be an antagonist of the protein of the present invention.

Alternatively, for example, the above cells stably expressing a protein of the invention may be directly exposed to a test sample, and a biochemical change as above described may be measured to isolate an agonist.

In a method in which the activity to inhibit the binding between a receptor protein of the invention and a ligand is used as an index, for example, phage display method may be used to isolate an agonist or antagonist. Phage display may be performed using, for example, the pSKAN Phagemid Display System (Catalogue #OB-15 18-00) according to the literature (Nicholas C. et al. Science 273:458 (1996)).

A compound obtained by the method of screening of the present invention may be applied as a medicine to humans or others mammals, such as mouse, rat, guinea pig, rabbit, chicken, cat, dog, sheep, porcine, bovine, monkey, sacred baboon, or chimpanzee, according to the standard procedures.

For instance, the compound may be administered orally, in the form of tablet, if necessary coated with sugar, capsule, elixir, or microcapsule, or may be parenterally administered, in the form of sterile solution in water or other pharmaceutically acceptable solution, or injection of suspension. A medicine may be prepared by, for example, mixing a substance having activity to bind to the protein of the present invention with a physiologically acceptable carrier, flavor, excipient, vehicle, preservative, stabilizer, or binder to a form a unit or dosage that is required by standard pharmaceutical procedures. The amount of active ingredient contained within the medicine may provide an appropriate dose within an indicated range.

Additives, for instance, binders such as gelatin, corn starch, tragacanth gum, or Arabia gum, excipients such as crystalline cellulose, swelling agents such as corn starch, gelatin, or alginic acid, lubricants such as magnesium stearate, sweeteners such as sucrose, lactose, or saccharine, or flavors such as peppermint, Gaultheria adenothrix oil, or cherry may be mixed within the tablet or capsule. If the preparative unit form is a capsule, liquid carriers such as fatty oils may be included as well. Sterile compositions for injection may be prepared by using vehicles such as distilled water for injection according to a standard pharmaceutical procedures.

Aqueous solutions such as saline, or isotonic solutions containing glucose or other adjuvants such as D-sorbitol, D-mannose, D-mannitol, or sodium chloride may be used for injection. To increase solubility, alcohols such as ethanol, polyalcohols such as propylene glycol, or polyethylene glycol, or non-ionic detergents such as polysorbate 80™, or HCO-50 may be appropriately added as an adjuvant.

Sesame oil or soybean oil may be used as oleaginous solution. Solubilizing agents such as benzyl-benzoate or benzyl alcohol may be added to increase solubility. Buffers such as phosphate buffer or sodium acetate buffer, pain-killers such as procaine chloride, stabilizers such as benzyl alcohol or phenol, or antioxidants may be mixed as well. Prepared solutions for injection may be packed in an appropriate ampoule.

The dose of the compound having the activity to bind to the protein of the invention may vary depending on symptom, but may be, in general, approximately 0.1 to 100 mg, preferably approximately 1.0 to 50 mg, and more preferably approximately 1.0 to 20 mg administered orally per day for adult (60 kg body weight).

In case of parenteral administration, a dose of, for example, approximately 0.01 to 30 mg, preferably approximately 0.1 to 20 mg, and more preferably approximately 0.1 to 10 mg may be injected intravenously per day for adult (60 kg body weight), although the dose may vary depending on the recipient, target organ, symptom, or method. In case of other animals, a dose normalized by the dose per 60 kg body weight may be applied.

An antibody of the present invention may be obtained as a monoclonal or polyclonal antibody by conventional methods.

To produce an antibody that specifically binds to the protein of the invention, the protein may be used as an antigen to immunize animals according to a standardmethod. Immune cells are thus obtained, and fused with known parental cells by standard cell fusion methods. Fusion cells are then screened for cells producing antibodies using standard methods.

More specifically, a monoclonal or polyclonal antibody that specifically binds to the protein of the invention may be generated as follows:

For instance, a protein of the invention used as an antigen to obtain an antibody may be derived from any animal species, but preferably it is from a mammal such as a human, mouse, or rat, or more preferably from a human. A human-derived protein may be obtained from the nucleotide or amino acid sequences disclosed herein.

Any protein having a biological activity of the proteins described herein, or its partial peptide may be used as an antigen in the present invention. A partial peptide may be, for example, an amino (N)-terminal or carboxy (C)-terminal fragment of the protein. Herein, an antibody is defined as an antibody that specifically reacts with either the full length or a fragment of the protein.

A gene encoding a protein of the invention or its fragment may be inserted into a known expression vector, which is used to transform a host cell as described herein. The desired protein or its fragment may be recovered from the outside or inside of host cells, or from host cells by any standard method, and may be used as an antigen. Alternatively, cells expressing the protein or their lysates, or a chemically synthesized protein may be used as an antigen.

Any mammalian animal may be immunized with the antigen, but preferably the compatibility with parental cells used for cell fusion is taken into account. In general, animals of Rodentia, Lagomorpha, or Primates are used.

Animals of Rodentia include, for example, mouse, rat, and hamster. Animals of Lagomorpha include, for example, rabbit. Animals of Primates include, for example, amonkey of Catarrhini (old world monkey) such as *Macaca fascicularis,* rhesus monkey, sacred baboon, or chimpanzee.

Methods for immunizing animals with antigens are known in the art. For instance, intraperitoneal injection or subcutaneous injection of antigens is used as a standard method for immunization of mammals. More specifically, antigens may be diluted and suspended in an appropriate amount with phosphate buffered saline (PBS), physiological saline, etc. If desired, the antigen suspension may be mixed with an appropriate amount of a standard adjuvant, such as Freund's complete adjuvant, made into emulsion, and then administered to mammalian animals. Preferably, it is followed by several administrations of antigen mixed with an appropriately amount of Freund's incomplete adjuvant every 4 to 21 days. An appropriate carrier may also be used for immunization. After immunization as above, serum is examined for increase of the amount of desired antibodies by a standard method.

Polyclonal antibodies to the proteins of the present invention may be prepared by collecting blood from the immunized mammal examined for the increase of desired antibodies in the serum, and by separating serum from the blood by any conventional method. Polyclonal antibodies may be used as serum containing the polyclonal antibodies, or if necessary, a fraction containing the polyclonal antibodies may be isolated from the serum.

To prepare monoclonal antibodies, immune cells are collected from the mammal immunized with the antigen and checked for the increased level of desired antibodies in the serum as described above, and are subjected to cell fusion. The immune cells used for cell fusion are preferably obtained from spleen. The other parent cell which is fused with the above immune cell is preferably a mammalian myeloma cell, and more preferably a myeloma cell that has acquired a special feature that can be used for selection of fusion cells by drug.

Cell fusion of the above immune cell and myeloma cell may be performed by any standard method, such as those described in the literature (Galfre G. and Milstein C. Methods Enzymol. 73:3-46 (1981)).

Resulting hybridomas obtained by the cell fusion may be selected by cultivating them in a standard selection medium, such as HAT medium (hypoxanthine, aminopterin, and thymidine containing medium). The cell culture is typically continued in the HAT medium for several days to several weeks, the time being sufficient to allow all the other cells, except desired hybridoma (non-fused cells), to die. Then, the standard limiting dilution is performed to screen and clone a hybridoma cell producing the desired antibody.

Besides the above method, in which anonhuman animal is immunized with an antigen for preparing hybridoma, human lymphocytes such as that infected by EB virus may be immunized with a protein, protein expressing cells, or their lysates in vitro. Then, the immunized lymphocytes are fused with human-derived myeloma cells that is capable of indefinitely dividing, such as U266, to yield a hybridoma producing a desired human antibody, able to bind to the protein can be obtained (Unexamined Published Japanese Patent Application (JP-A) No. Sho 63-17688).

Furthermore, transgenic animals having a repertory of human antibody genes may be immunized with a protein, protein expressing cells, or their lysates as an antigen. Antibody producing cells are collected from the animals, and fused with myeloma cells to obtain hybridoma, from which human antibodies against the protein can be prepared (see WO92-03918, WO93-2227, WO94-02602, WO94-25585, WO96-33735, and WO96-34096)

Alternatively, an immune cell, such as an immunized lymphocyte, producing antibodies may be immortalized by an oncogene and used for preparing monoclonal antibodies.

Monoclonal antibodies thus obtained can be also be recombinantly prepared using conventional genetic engineering techniques (see, for example, Borrebaeck C.A.K. and Larrick J.W. Therapeutic Monoclonal Antibodies, published in the United Kingdom by MacMillan Publishers LTD (1990)). A DNA encoding an antibody may be cloned from an immune cell such as a hybridoma or an immunized lymphocyte producing the antibody, inserted into an appropriate vector, and introduced into host cells to prepare a recombinant antibody. The present invention also provides recombinant antibodies prepared as described above.

An antibody of the present invention may be a fragment of an antibody or modified antibody, solong as it binds to one or more of the proteins of the invention. For instance, the antibody fragment may be Fab, F(ab')₂, Fv, or single chain Fv (scFv), in which Fv fragments from H and L chains are ligated by an appropriate linker (Huston J.S. et al. Proc. Natl. Acad. Sci. U.S.A. 85:5879-5883 (1988)). More specifically, an antibody fragment may be generated by treating an antibody with an enzyme such as papain or pepsin. Alternatively, a gene encoding the antibody fragment may be constructed, inserted into an expression vector, and expressed in an appropriate host cell (see, for example, Co M.S. et al. J. Immunol. 152:2968-2976 (1994); Better M. and Horwitz A.H. Methods Enzymol. 178:476-496 (1989); Pluckthun A. and Skerra A. Methods Enzymol. 178:497-515 (1989); Lamoyi E. Methods Enzymol. 121:652-663 (1986); Rousseaux J. et al. Methods Enzymol. 121:663-669 (1986); Bird R.E. and Walker B.W. Trends Biotechnol. 9:132-137 (1991)).

An antibody may be modified by conjugation with a variety of molecules, such as polyethylene glycol (PEG). The present invention provides such modified antibodies. The modified antibody can be obtained by chemically modifying an antibody. These modification methods are conventional in the field.

Alternatively, an antibody of the present invention may be obtained as a chimeric antibody, between a variable region derived from nonhuman antibody and the constant region derived from human antibody, or as a humanized antibody, comprising the complementarity determining region (CDR) derived from nonhuman antibody, the frame work region (FR) derived from human antibody, and the constant region. Such antibodies can be prepared by using known technology.

Obtained antibodies may be purified to homogeneity. The invention is not limited to one particular method for antibody separation and purification. Any standard method of protein separation and purification may be used. The concentration of the obtained antibody may be determined by measuring absorbance, by enzyme-linked immunosorbent assay (ELISA), etc.

ELISA, enzyme immunoassay (EIA), radioimmunoassay (RIA), and/or immunofluorescence may be used to measure the antigen binding activity of the antibody of the invention. In ELISA, the antibody of the present invention is immobilized on a plate, protein of the invention is applied to the plate, and then a sample containing a desired antibody, such as culture supernatant of antibody producing cells or purified antibodies, is applied. Then, a secondary antibody, that recognizes the primary antibody and is labeled with an enzyme such as alkaline phosphatase, is applied, and the plate is incubated. After washing, an enzyme substrate, such as p-nitrophenyl phosphate, is added to the plate, and the absorbance is measured to evaluate the antigen binding activity of the sample. A fragment of the protein, such as a C-terminal or N-terminal fragment, may be used as a protein. BIAcore (Pharmacia) may be used to evaluate the activity of the antibody according to the present invention.

The above methods allow for the detection or measurement of the protein of the invention, by exposing the antibody of the invention to a sample assumed to contain the protein of the invention, and detecting or measuring the immune complex formed by the antibody and the protein.

Because the method of detection or measurement of the protein according to the invention can specifically detect or measure a protein, the method may be useful in a variety of experiments in which the protein is used.

The present invention also provides DNA of a length of 15 nucleotides or longer that hybridize with DNA having the nucleotide sequence of SEQ ID NOs: 1 to 3 or 24 to 27, or with DNA complementary to the DNA (complementary strand). Thus, the present invention provides probes that can selectively hybridize with a DNA encoding a protein of the invention, a DNA complementary to such a DNA, or a nucleotide or its derivative, such as antisense oligonucleotide, a ribozyme, etc.

The present invention comprises, for example, an antisense oligonucleotide that hybridizes with any portion of the nucleotide sequence of SEQ ID. NOs: 1 to 3 and 24 to 27. The antisense oligonucleotide is preferably an antisense of a continuous sequence of a length of 15 nucleotides or longer within the nucleotide sequence of any of SEQ ID NOs: 1 to 3 and 24 to 27. More preferably, the above continuous sequence of a length of 15 nucleotides or longer contains the translation initiation codon.

A derivative or modified form of antisense oligonucleotide may also be used. The latter form may modified with lower alkylphosphonate such as methylphosphonate or ethylphosphonate, or with phosphorothioate, or phosphoroamidate.

Herein, an antisense oligonucleotide is not restricted to one in which all nucleotides are complementary to the corresponding nucleotides within a given region of a DNA or mRNA; as long as it can selectively form a stable hybrid with any of the nucleotide sequences of SEQ ID NOs: 1 to 3 or 24 to 27, it may have one or more nucleotide mismatches.

The phrase "selectively form a stable hybrid" is defined herein as not forming a significant cross hybrid with a DNA encoding other proteins under normal conditions of hybridization, or preferably under stringent conditions. Such DNA include those which have a homology of at least 70%, preferably 80% or higher, more preferably 90% or higher, and even more preferably 95% or higher within a continuous sequence of a length of 15 nucleotide or longer. To determine the degree of homology, the algorithm described herein may be used. As described in the following Examples, the above DNA is useful as a probe for detection or isolation of a DNA encoding the protein of the invention, or as a primer for its amplification.

A derivative of an antisense oligonucleotide of a present invention may act on cells producing the protein of the invention and bind to a DNA or mRNA encoding the protein, and then, it may inhibit the expression of the protein of the invention by inhibiting its transcription or translation, or by promoting the degradation of mRNA, and thereby inhibiting the function of the protein of the invention.

A derivative of an antisense oligonucleotide of the present invention may be mixed with an appropriate base which is inactive against the derivative, and used as a medicine for externally application such as salve or poultice.

If necessary, it may be mixed with an excipient, isotonizing agent, solubilizing agent, stabilizer, preservative, pain-killer, or the like, and prepared as a tablet, powder, granule, capsule, liposome capsule, injectable solution, liquid formulation, nose drops, freeze-dried agent, etc. The above may be achieved according to standard methods.

For treating patients, a derivative of an antisense oligonucleotide of the present invention may be, for example, directly applied to the affected area of a patient, or administered into blood vessels so as to finally reach the affected area. Moreover, the derivative may be encapsulated in antisense-encapsulating materials such as liposome, poly-L-lysine, lipid, cholesterol, lipofectin, or their derivative in order to increase durability and/or membrane permeability.

Dose of the derivative of the antisense oligonucleotide of the present invention may be appropriately adjusted depending on the patient's conditions, and a favorable amount such as 0.1 to 100 mg/kg, or more preferably 0.1 to 50 mg/kg may be administered.

As the antisense oligonucleotides of the present invention inhibit expression of the protein of the invention, it finds utility as an inhibitor of biological activity of the protein of the invention. An inhibitor of expression comprising the antisense oligonucleotide of the present invention is useful because it can inhibit the biological activity of the protein of the invention.

### Brief Description of the Drawings

Figure 1 shows a comparison of the amino acid sequences among known human olfactory receptors and GTAR14-1 and GTAR14-2. Shadowed residues are shared by at least four proteins out of five. Dotted residues are matched in all five proteins. Abbreviations are as follows:

OLF1 for human olfactory receptor 1 (GenBank Accession# U56420); OLF2 for human olfactory receptor 2 (GenBank Accession# L35475); OLF3 for human olfactory receptor 3 (GenBank Accession# L56421); 14-1 for GTAR14-1; and 14-2 for GTAR14-2 (pseudogene).

Figure 2 shows a comparison of the amino acid sequences among known human olfactory receptors and GTAR14-3. Shadowed residues are shared by at least three proteins out of four. Dotted residues are matched in all four proteins. Abbreviations are as follows:

OLF1 for human olfactory receptor 1 (GenBank Accession# U56420); OLF2 for human olfactory receptor 2 (GenBank Accession# L35475); OLF3 for human olfactory receptor 3 (GenBank Accession# L56421); and 14-3 for GTAR14-3.

Figure 3 shows a comparison of the amino acid sequences among known human olfactory receptors and GTAR14-4 and GTAR14-5. Shadowed residues are shared by at least three proteins out of five. Dotted residues are matched in all five proteins. Abbreviations are as follows:

OLF1 for human olfactory receptor 1 (GenBank Accession# U56420); OLF2 for human olfactory receptor 2 (GenBank Accession# L35475); OLF3 for human olfactory receptor 3 (GenBank Accession# L56421); 14-4 for GTAR14-4 (pseudogene); and 14-5 for GTAR14-5.

Figure 4 shows the nucleotide sequence of GTAR14-1 which was obtained by genomic PCR. The positions of the primers used are underlined. The right-directed and left-directed arrows indicate the positions of 14-1-S1 primer, and 14-1-A1 primer, respectively.

Figure 5 shows the nucleotide sequence of GTAR14-3 which was obtained by genomic PCR. The positions of the primers used are underlined. The right-directed and left-directed arrows indicate the positions of 14-3-S1 primer, and 14-3-A1 primer, respectively.

Figure 6 shows the nucleotide sequence of GTAR14-5 which was obtained by genomic PCR. The positions of the primers used are underlined. The right-directed and left-directed arrows indicate the positions of 14-5-S1 primer, and 14-5-A1 primer, respectively.

Figure 7 is a photograph showing the result of RT-PCR analysis of the gene expression pattern of GTAR14-1 in human tissues.

A: Human Fetal MTC Panel (Clontech #K1425-1), B: Human MTC Panel I (Clontech #K1420-1), C: Human MTC Panel II (Clontech #K1421-1).

Lanes 1: fetal brain, 2: fetal heart, 3: fetal kidney, 4: fetal liver, 5: fetal lung, 6: fetal muscle, 7: fetal spleen, 8: fetal thymus, 9: heart, 10:brain, 11: placenta, 12: lung, 13: liver, 14: skeletal muscle, 15: kidney, 16: pancreas, 17: spleen, 18: thymus, 19: prostate, 20: testis, 21: ovary, 22: small intestine, 23: colon, and24: peripheral leukocytes.

The left lane of each panel shows the result of the positive control in which human genomic DNA was amplified as a template using the same primer set. The right lane of each panel shows the product of the control reaction in which G3PDH was amplified from a standard cDNA as a template using a primer set for G3PDH.

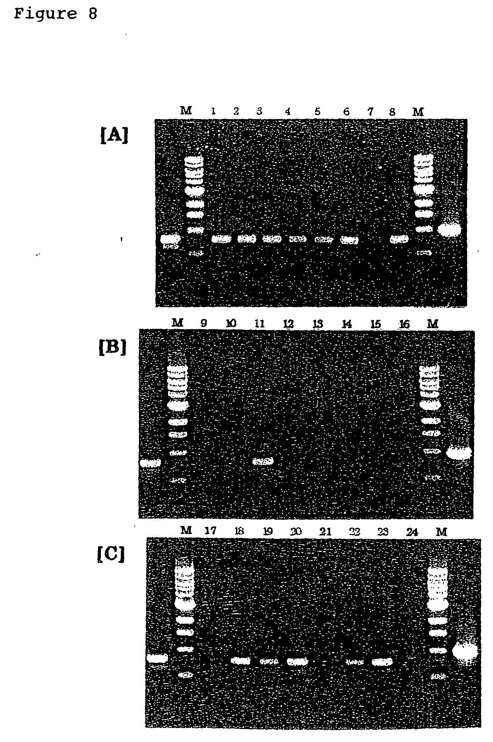

Figure 8 is a photograph showing the result of RT-PCR analysis of the gene expression pattern of GTAR14-3 in human tissues.

A: Human Fetal MTC Panel (Clontech #K1425-1), B: Human MTC Panel I (Clontech #K1420-1), C: Human MTC Panel II (Clontech #K1421-1).

Lanes 1: fetal brain, 2: fetal heart, 3: fetal kidney, 4: fetal liver, 5: fetal lung, 6: fetal muscle, 7: fetal spleen, 8: fetal thymus, 9: heart, 10:brain, 11: placenta, 12: lung, 13: liver, 14: skeletal muscle, 15: kidney, 16: pancreas, 17: spleen, 18: thymus, 19: prostate, 20: testis, 21: ovary, 22: small intestine, 23: colon, and 24: peripheral leukocytes.

The left lane of each panel shows the result of the positive control in which human genomic DNA was amplified as a template using the same primer set. The right lane of each panel shows the product of the control reaction in which G3PDH was amplified from a standard cDNA as a template using a primer set for G3PDH.

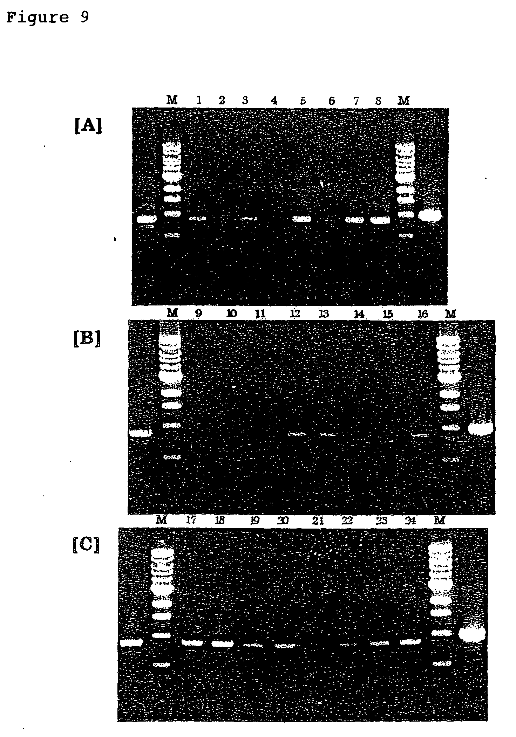

Figure 9 is a photograph showing the result of RT-PCR analysis of the gene expression pattern of GTAR14-5 in human tissues.

A: Human Fetal MTC Panel (Clontech #K1425-1), B: Human MTC Panel I (Clontech #K1420-1), C: Human MTC Panel II (Clontech #K1421-1).

Lanes 1: fetal brain, 2: fetal heart, 3: fetal kidney, 4: fetal liver, 5: fetal lung, 6: fetal muscle, 7: fetal spleen, 8: fetal thymus, 9: heart, 10:brain, 11: placenta, 12: lung, 13: liver, 14: skeletal muscle, 15: kidney, 16: pancreas, 17: spleen, 18: thymus, 19: prostate, 20: testis, 21: ovary, 22: small intestine, 23: colon, and 24: peripheral leukocytes.

The left lane of each panel shows the result of the positive control in which human genomic DNA was amplified as a template using the same primer set. The right lane of each panel shows the product of the control reaction in which G3PDH was amplified from a standard cDNA as a template using a primer set for G3PDH.

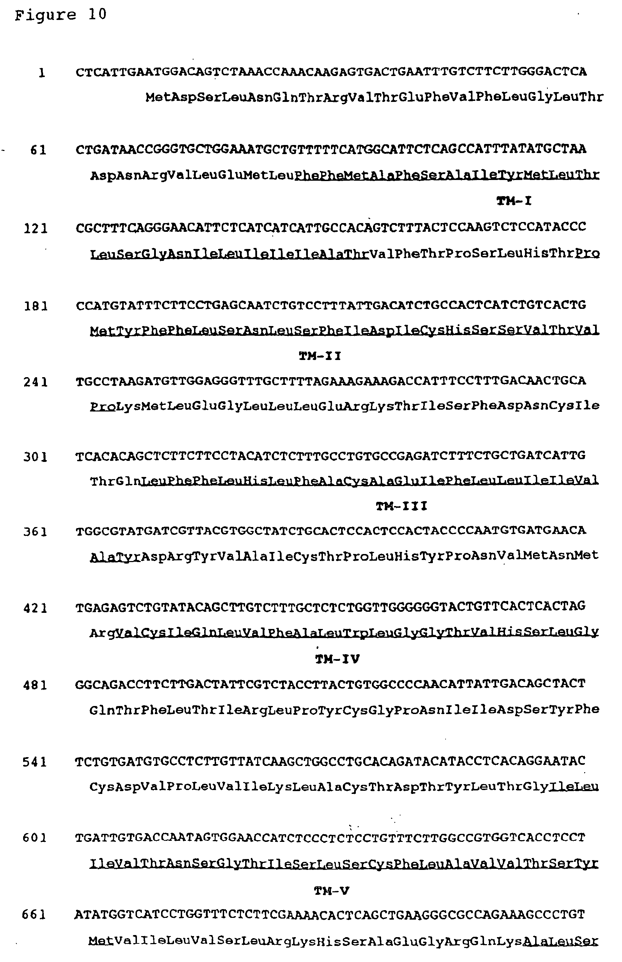

Figure 10 shows the nucleotide sequence of the full length GTAR14-1 cDNA that was obtained by integrating the products of 5'-RACE and 3'-RACE. The amino acid sequence encoded by the GTAR14-1 cDNA is also indicated. The amino acid sequences predicted to be a transmembrane domain are underlined (TM-I, -II, -III, -IV, and -V).

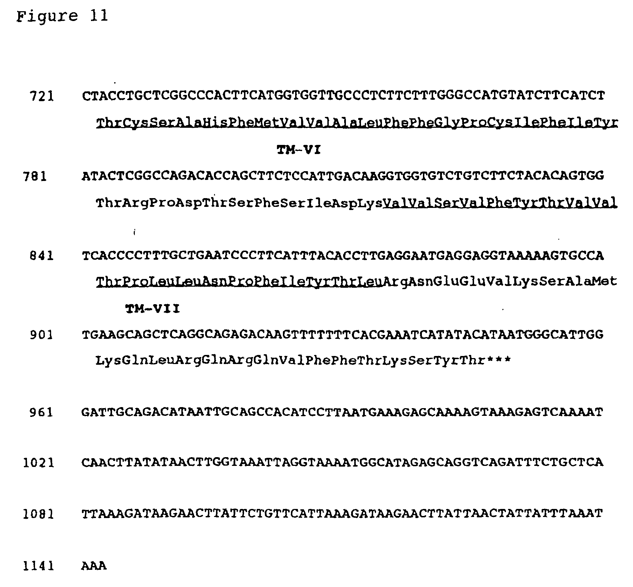

Figure 11 shows the nucleotide sequence of the full length GTAR14-1 cDNA that was obtained by integrating the products of 5'-RACE and 3'-RACE, continued from Figure 10. The amino acid sequence encoded by the GTAR14-1 cDNA is also indicated. The amino acid sequences predicted to be a transmembrane domain are underlined (TM-VI and -VII).

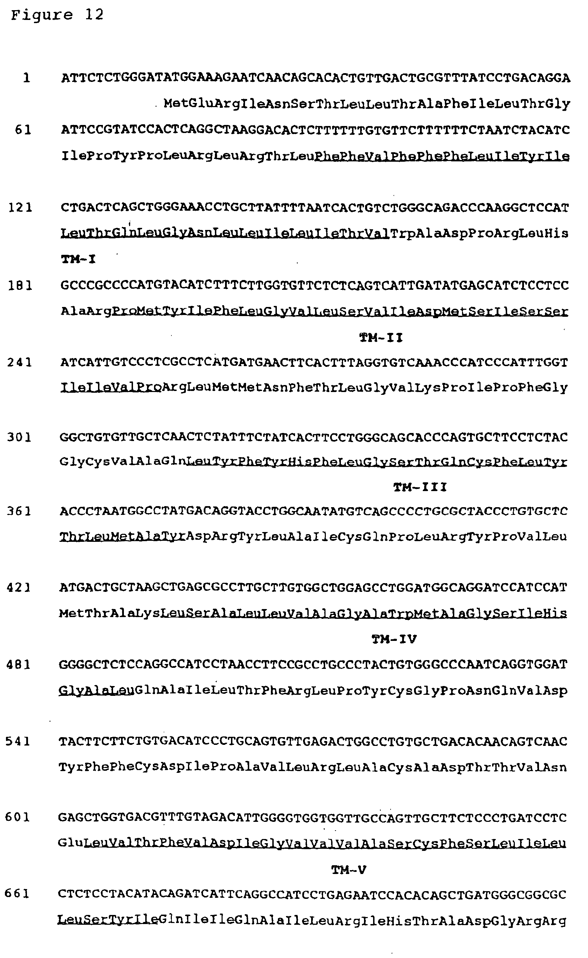

Figure 12 shows the nucleotide sequence of the full length GTAR14-3 cDNA that was obtained by integrating the products of 5'-RACE and 3'-RACE. The amino acid sequence encoded by the GTAR14-3 cDNA is also indicated. Amino acid sequences predicted to be a a transmembrane domain are underlined (TM-I, -II, -III, -IV, and -V) .

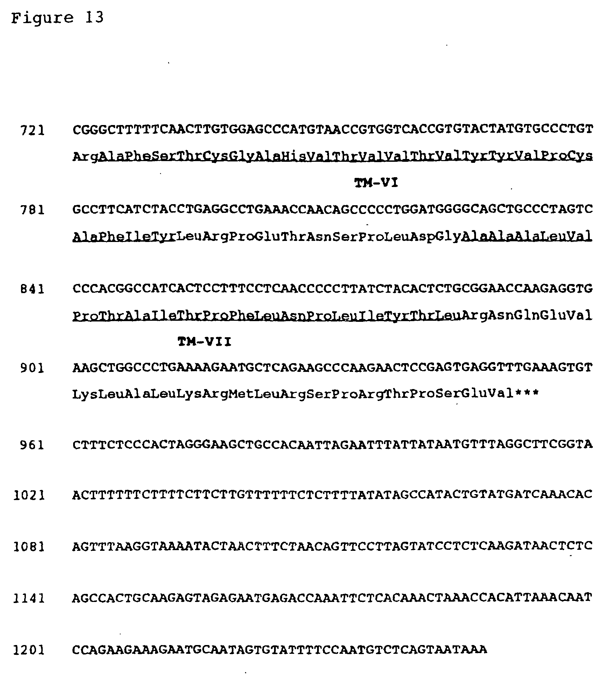

Figure 13 shows the nucleotide sequence of the full length GTAR14-3 cDNA that was obtained by integrating the products of 5'-RACE and 3' -RACE, continued from Figure 12. The amino acid sequence encoded by the GTAR14-3 cDNA is also indicated. Amino acid sequences predicted to be a transmembrane domain are underlined (TM-VI and -VII).

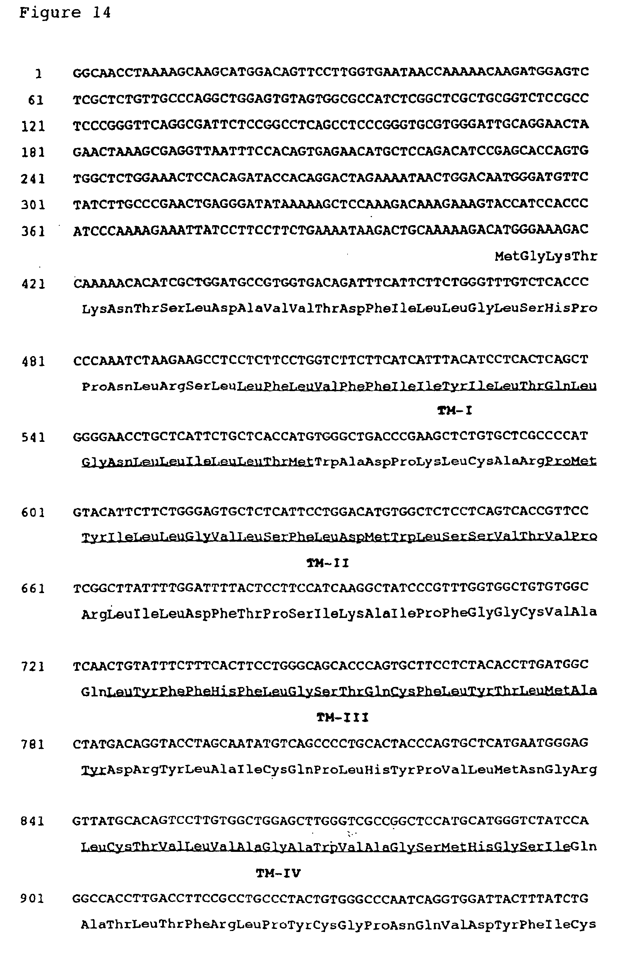

Figure 14 shows the nucleotide sequence of the full length GTAR14-5 cDNA that was obtained by integrating the products of 5'-RACE and 3'-RACE. The amino acid sequence encoded by the GTAR14-5 cDNA is also indicated. Amino acid sequences predicted to be a transmembrane domain are underlined (TM-I, -II, -III, and -IV).

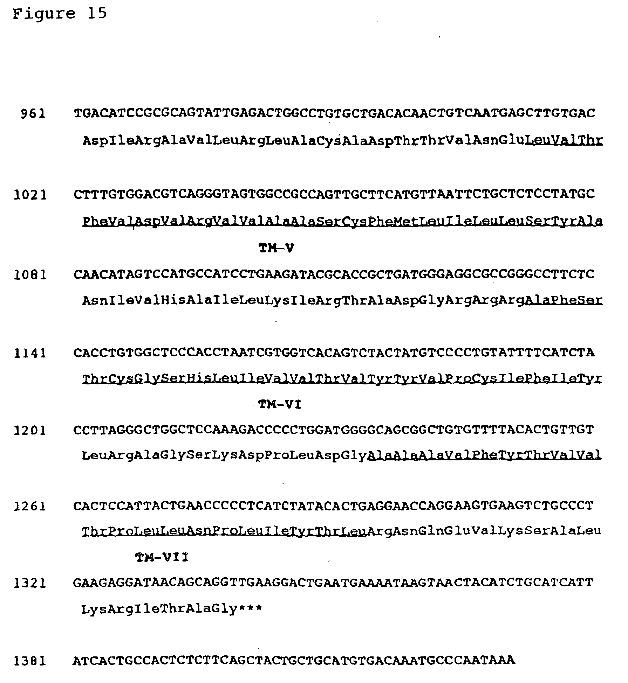

Figure 15 shows the nucleotide sequence of the full length GTAR14-5 cDNA that was obtained by integrating the products of 5'-RACE and 3'-RACE, continued fromFigure 14. The amino acid sequence encoded by the GTAR14-5 cDNA is also indicated. minAo acid sequences predicted to be a transmembrane domain are underlined (TM-V, -VI, and -VII).

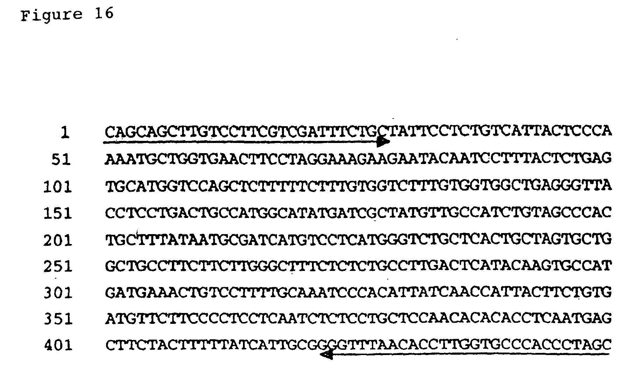

Figure 16 shows the nucleotide sequence of GTAR11-1 which was obtained by genomic PCR. The positions of the primers used are underlined. The right-directed and left-directed arrows indicate the positions of 11-1-S2 primer, and 11-1-A3 primer, respectively.

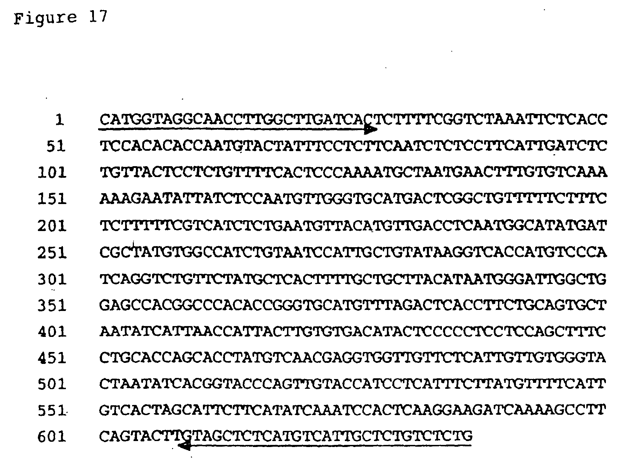

Figure 17 shows the nucleotide sequence of GTAR11-2 which was obtained by genomic PCR. The positions of the primers used are underlined. The right-directed and left-directed arrows indicate the positions of 11-2-S2 primer, and 11-2-A2 primer, respectively.

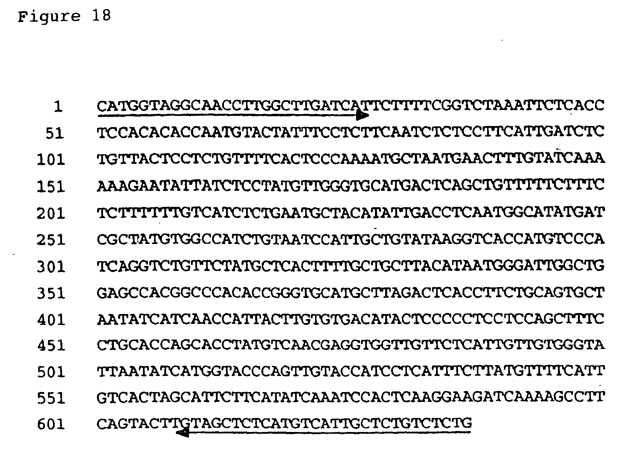

Figure 18 shows the nucleotide sequence of GTAR11-3 which was obtained by genomic PCR. The positions of primers used are underlined. The right-directed and left-directed arrows indicate the positions of 11-3-S2 primer, and 11-3-A2 primer, respectively.

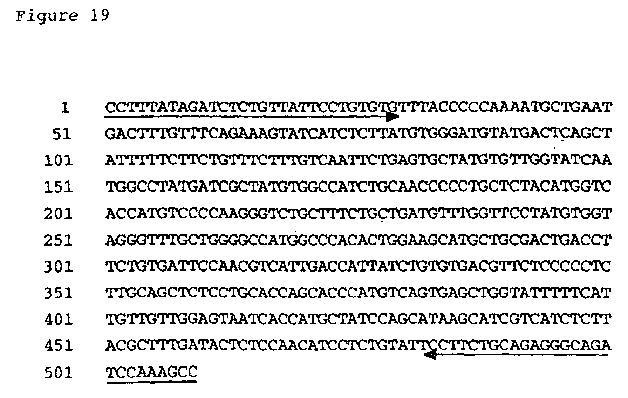

Figure 19 shows the nucleotide sequence of GTAR11-4 which was obtained by genomic PCR. The positions of the primers used are underlined. The right-directed and left-directed arrows indicate the positions of 11-4-S2 primer, and 11-4-A2 primer, respectively.

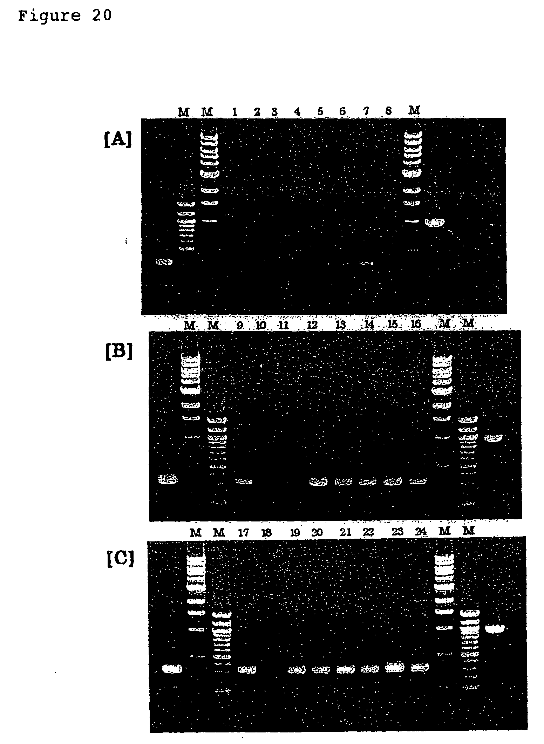

Figure 20 is a photograph showing the result of RT-PCR analysis of the gene expression pattern of GTAR11-1 in human tissues.

A: Human Fetal MTC Panel (Clontech #K1425-1), B: Human MTC Panel I (Clontech #K1420-1), C: Human MTC Panel II (Clontech #K1421-1).

Lanes 1: fetal brain, 2: fetal heart, 3: fetal kidney, 4: fetal liver, 5: fetal lung, 6: fetalmuscle, 7: fetal spleen, 8: fetal thymus, 9: brain, 10: heart, 11: kidney, 12: liver, 13: lung, 14: pancreas, 15: placenta, 16: skeletal muscle, 17: colon, 18: ovary, 19: peripheral leukocytes, 20: prostate, 21: small intestine, 22: spleen, 23: testis, and 24: thymus.

The left lane of each panel shows the result of the positive control in which human genomic DNA was amplified as a template using the same primer set. The right lane of each panel shows the product of the control reaction in which G3PDH was amplified from a standard cDNA as a template using a primer set for G3PDH.

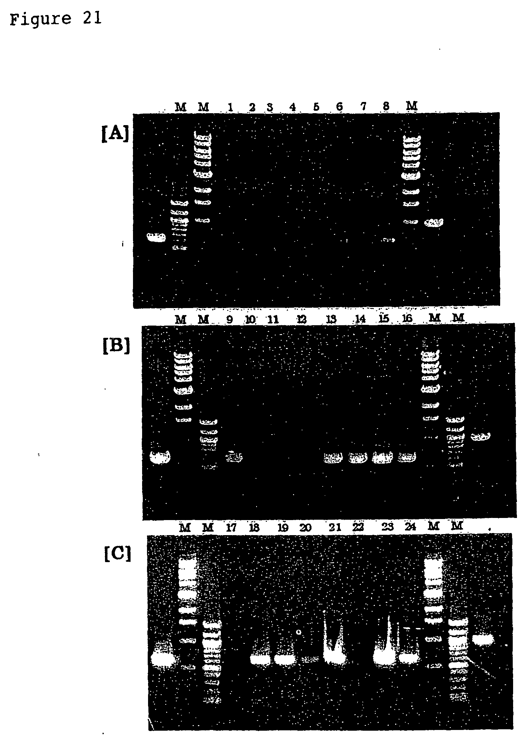

Figure 21 is a photograph showing the result of RT-PCR analysis of the gene expression pattern of GTAR11-2 in human tissues.

A: Human Fetal MTC Panel (Clontech #K1425-1), B: Human MTC Panel I (Clontech #K1420-1), C: Human MTC Panel II (Clontech #K1421-1).

Lanes 1: fetal brain, 2: fetal heart, 3: fetal kidney, 4: fetal liver, 5: fetal lung, 6: fetal muscle, 7: fetal spleen, 8: fetal thymus, 9: brain, 10: heart, 11: kidney, 12: liver, 13: lung, 14: pancreas, 15: placenta, 16: skeletal muscle, 17: colon, 18: ovary, 19: peripheral leukocytes, 20: prostate, 21: small intestine, 22: spleen, 23: testis, and 24: thymus.

The left lane of each panel shows the result of the positive control in which human genomic DNA was amplified as a template using the same primer set. The right lane of each panel shows the product of the control reaction in which G3PDH was amplified from a standard cDNA as a template using a primer set for G3PDH.

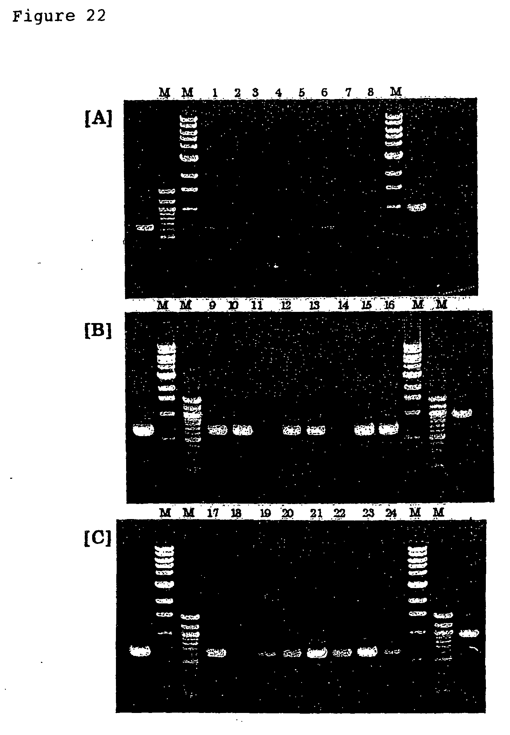

Figure 22 is a photograph showing the result of RT-PCR analysis of the gene expression pattern of GTAR11-3 in human tissues.

A: Human Fetal MTC Panel (Clontech #K1425-1), B: Human MTC Panel I (Clontech #K1420-1), C: Human MTC Panel II (Clontech #K1421-1).

Lanes 1: fetal brain, 2: fetal heart, 3: fetal kidney, 4: fetal liver, 5: fetal lung, 6: fetal muscle, 7: fetal spleen, 8: fetal thymus, 9: brain, 10: heart, 11: kidney, 12: liver, 13: lung, 14: pancreas, 15: placenta, 16: skeletal muscle, 17: colon, 18: ovary, 19: peripheral leukocytes, 20: prostate, 21: small intestine, 22: spleen, 23: testis, and 24: thymus.

The left lane of each panel shows the result of the positive control in which human genomic DNA was amplified as a template using the same primer set. The right lane of each panel shows the product of the control reaction in which G3PDH was amplified from a standard cDNA as a template using a primer set for G3PDH.

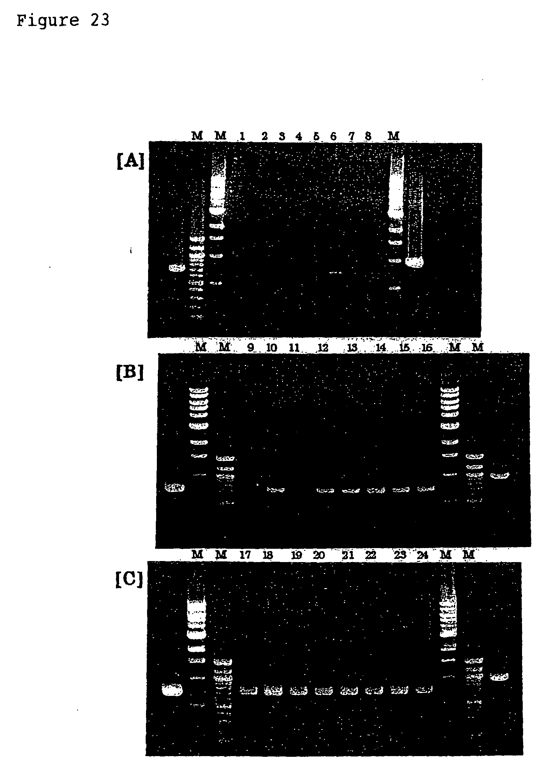

Figure 23 is a photograph showing the result of RT-PCR analysis of the gene expression pattern of GTAR11-4 in human tissues.

A: Human Fetal MTC Panel (Clontech #K1425-1), B: Human MTC Panel I (Clontech #K1420-1), C: Human MTC Panel II (Clontech #K1421-1).

Lanes 1: fetal brain, 2: fetal heart, 3: fetal kidney, 4: fetal liver, 5: fetal lung, 6: fetal muscle, 7: fetal spleen, 8: fetal thymus, 9: brain, 10: heart, 11: kidney, 12: liver, 13: lung, 14: pancreas, 15: placenta, 16: skeletal muscle, 17: colon, 18: ovary, 19: peripheral leukocytes, 20: prostate, 21: small intestine, 22: spleen, 23: testis, and 24: thymus.

The left lane of each panel shows the result of the positive control in which human genomic DNA was amplified as a template using the same primer set. The right lane of each panel shows the product of the control reaction in which G3PDH was amplified from a standard cDNA as a template using a primer set for G3PDH.

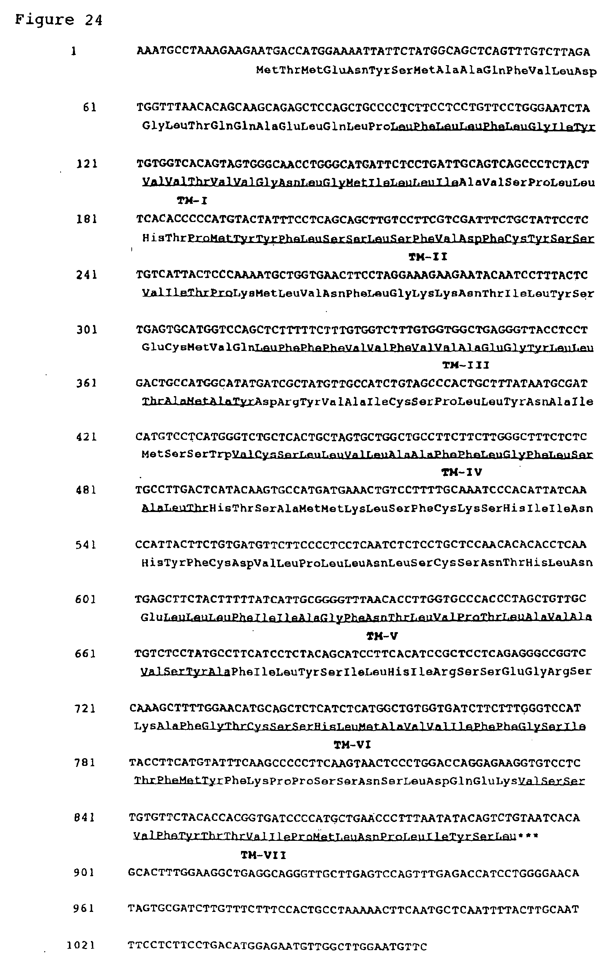

Figure 24 shows the nucleotide sequence of the GTAR11-1 cDNA that was obtained by integrating the products of 5'-RACE and 3'-RACE. The amino acid sequence encoded by the GTAR11-1 cDNA is also indicated. Amino acid sequences predicted to be a transmembrane domain are underlined (TM-I, -II, -III, -IV, -V, -VI, and -VII).

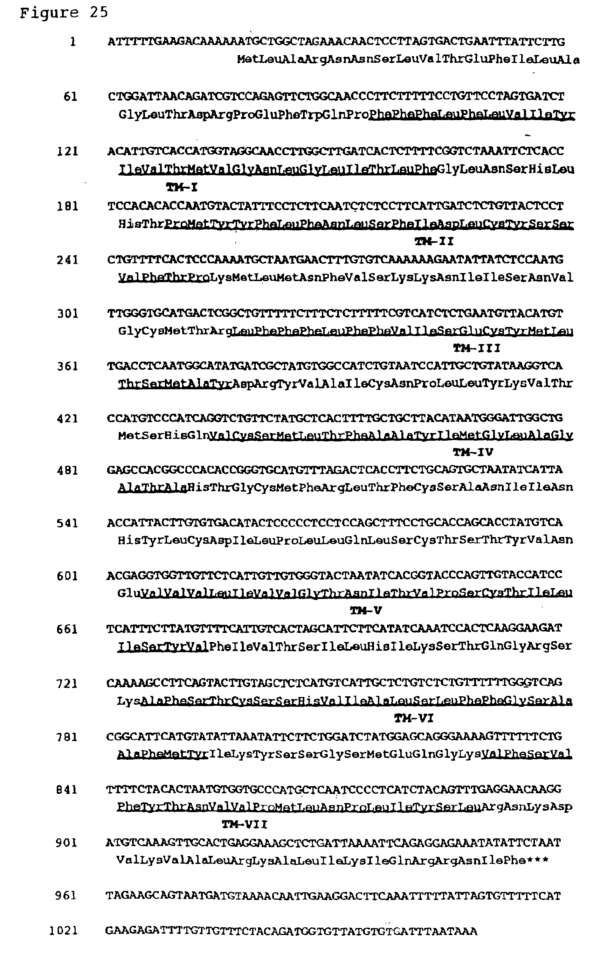

Figure 25 shows the nucleotide sequence of the GTAR11-2 cDNA that was obtained by integrating the products of 5'-RACE and 3'-RACE. The amino acid sequence encoded by the GTAR11-2 cDNA is also indicated. Amino acid equences predicted to be a transmembrane domain are underlined (TM-I, -II, -III, -IV, -V, -VI, and -VII).

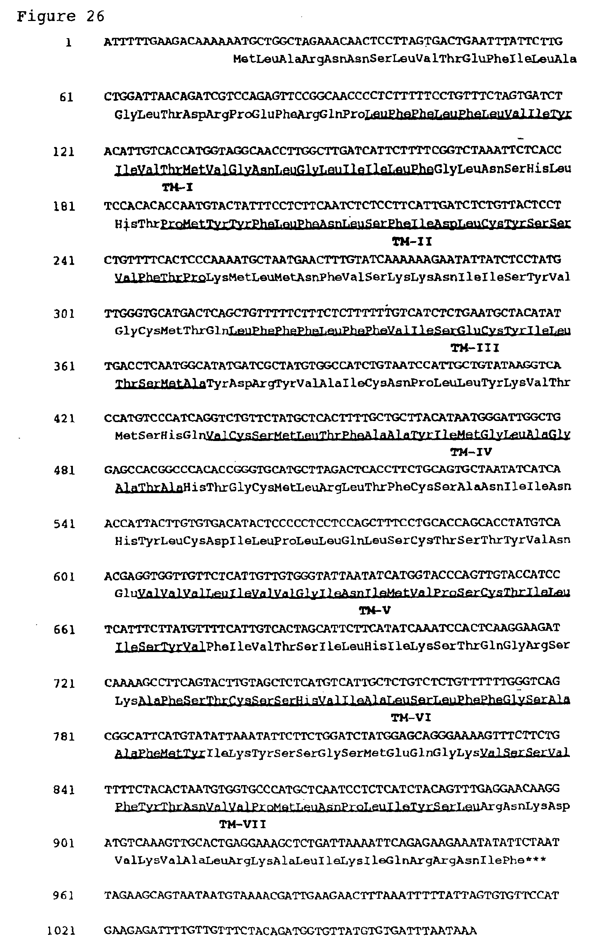

Figure 26 shows the nucleotide sequence of the GTAR11-3 cDNA that was obtained by integrating the products of 5'-RACE and 3'-RACE. The amino acid sequence encoded by the GTAR11-3 cDNA is also indicated. Amino acid sequences predicted to be a transmembrane domain are underlined (TM-I, -II, -III, -IV, -V, -VI, and -VII).

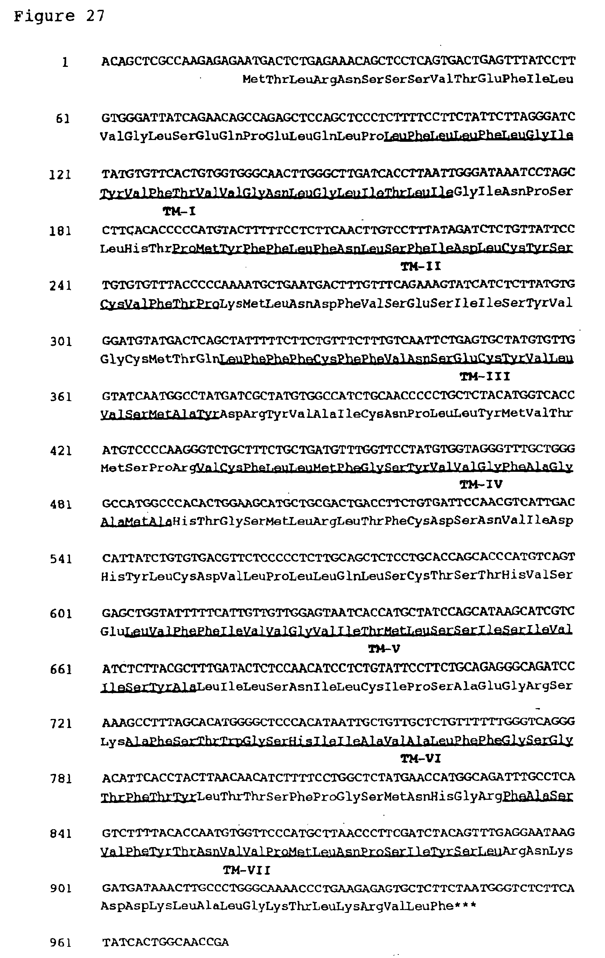

Figure 27 shows the nucleotide sequence of the GTAR11-4 cDNA that was obtained by integrating the products of 5'-RACE and 3'-RACE. The amino acid sequence encoded by the GTAR11-4 cDNA is also indicated. Amino acid sequences predicted to be a transmembrane domain are underlined (TM-I, -II, -III, ,-IV, -V, -VI, and -VII).

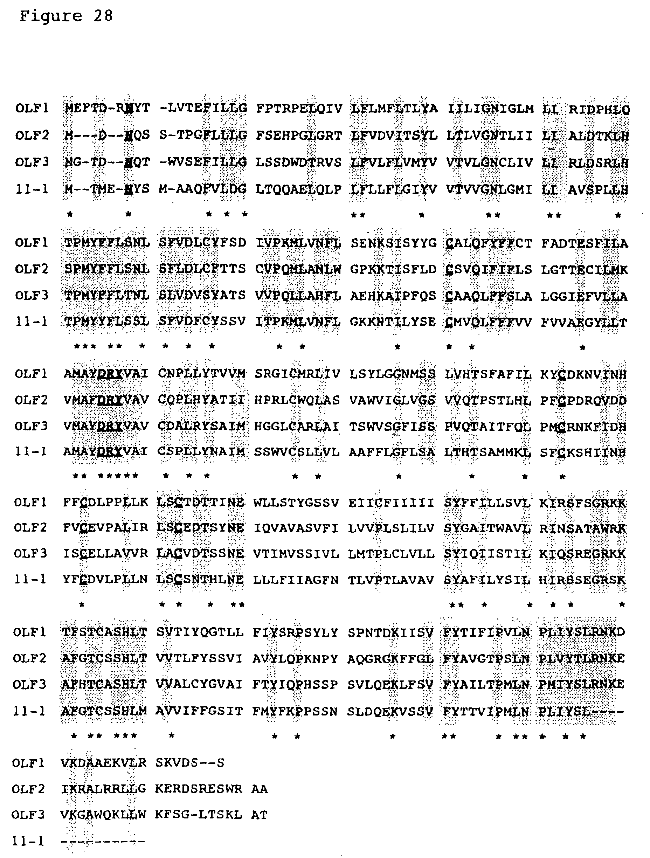

Figure 28 shows comparison of the amino acid sequences among known human olfactory receptors and GTAR11-1. Shadowed residues are shared by at least three proteins out of four. Dotted residues are matched in all four proteins. The N residue of the [N-x-S/T] motif (x, any amino acid residue) that is modified by sugar, conserved cysteine residues that form a disulfide bond required for cross-linking, and the D-R-Y sequence in the [M-A-Y-D-R-Y-L/V-A-I/V-C] motif in the second intracellular domain, which is suggested to be essential for the binding to intracellular G proteins, are underlined. Abbreviations are as follows:

OLF1 for human olfactory receptor 1 (GenBankAccession# U56420); OLF2 for human olfactory receptor 2 (GenBank Accession# L35475); OLF3 for human olfactory receptor 3 (GenBank Accession# L56421); and 11-1 for GTAR11-1.

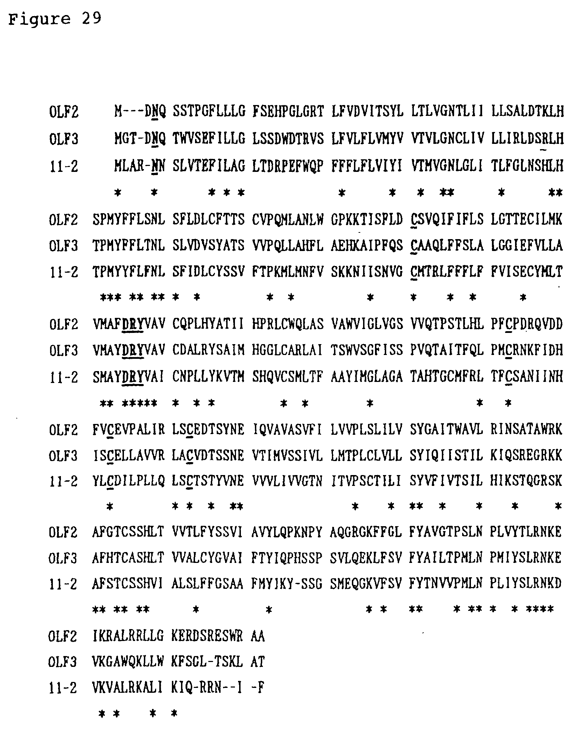

Figure 29 shows comparison of the amino acid sequences among known human olfactory receptors and GTAR11-2. Dotted residues are matched in all three proteins. The N residue of [N-x-S/T] motif (x, any amino acid residue) that is modified by sugar, conserved cysteine residues that form a disulfide bond required for cross-linking, and the D-R-Y sequence in [M-A-Y-D-R-Y-L/V-A-I/V-C] motif in the second intracellular domain, which is suggested to be essential for the binding to intracellular G proteins, are underlined. Abbreviations are as follows:

OLF2 for human olfactory receptor 2 (GenBank Accession# L35475); OLF3 for human olfactory receptor 3 (GenBank Accession# L56421); and 11-2 for GTAR11-2.

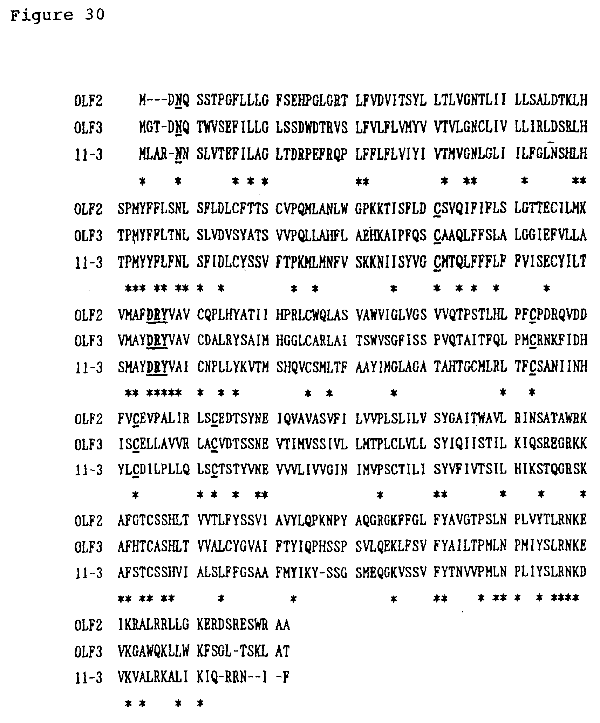

Figure 30 shows comparison of the amino acid sequences among known human olfactory receptors and GTAR11-3. Dotted residues are matched in all three proteins. The N residue of [N-x-S/T] motif (x, any amino acid residue) that is modified by sugar, conserved cysteine residues that form a disulfide bond required for cross-linking, and the D-R-Y sequence in [M-A-Y-D-R-Y-L/V-A-I/V-C] motif in the second intracellular domain, which is suggested to be essential for the binding to intracellular G proteins, are underlined. Abbreviations are as follows:

OLF2 for human olfactory receptor 2 (GenBankAccession# L35475); OLF3 for human olfactory receptor 3 (GenBank Accession# L56421); and 11-3 for GTAR11-3.

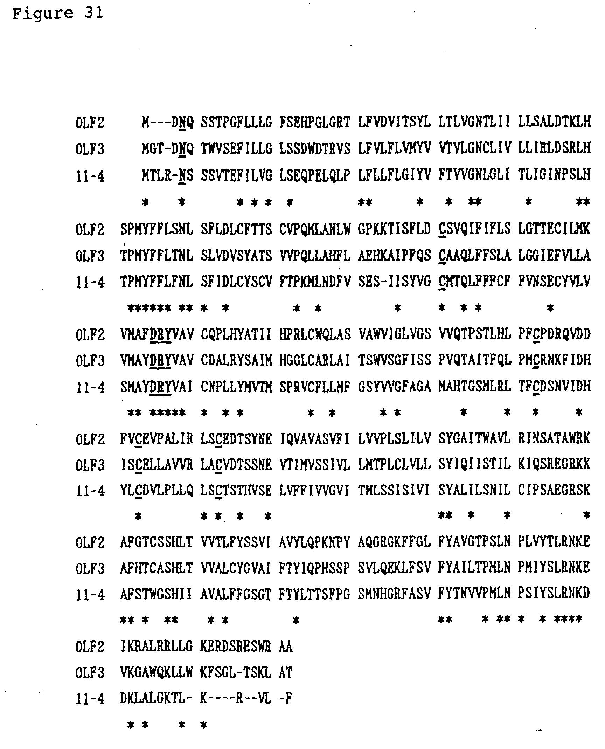

Figure 31 shows comparison of the amino acid sequences among known human olfactory receptors and GTAR11-4. Dotted residues are matched in all three proteins. The N residue of [N-x-S/T] motif (x, any amino acid residue) that is modified by sugar, conserved cysteine residues that form a disulfide bond required for cross-linking, and the D-R-Y sequence in [M-A-Y-D-R-Y-L/V-A-I/V-C] motif in the second intracellular domain, which is suggested to be essential for the binding to intracellular G proteins, are underlined. Abbreviations are as follows:

OLF2 for human olfactory receptor 2 (GenBank Accession# L35475); OLF3 for human olfactory receptor 3 (GenBank Accession# L56421); and 11-4 for GTAR11-4.

### Best Mode for Carrying Out the Invention

### Example 1. Isolation and analysis of GTAR14 genes

### (1) BLAST search

BLAST search was performed on the Non-redundant Sequence (nr) database in GenBank, and a human genomic sequence that contains a gene encoding a novel seven transmembrane G protein-coupled receptor, which has a high structural homology to known olfactory receptor (OR) gene family, was found. The gene is contained in the sequence of BAC129 clone (GenBank Accession# U85195), originating from the vicinity of the α/δ T-cell receptor locus on human chromosome 14, and shows about 50 to 60% homology to known human olfactory receptors, OLF1, OLF2, and OLF3 at the amino acid sequence level.

Based on the results of analysis of gene expression pattern as described below and prediction of function, the three isolated genes were named G protein-coupled T-cell Activating Receptor 14-1 (GTAR14-1), G protein-coupled T-cell Activating Receptor 14-3 (GTAR14-3), and G protein-coupled T-cell Activating Receptor 14-5 (GTAR14-5), respectively.

The genomic structures of known OR gene family have a unique feature in that the ORF from the initiation codon to the stop codon is contained in a single exon of the genomic sequence encoding the known OR gene. The full length ORFs of GTAR14-1, GTAR14-3, and GTAR14-5 are also contained in a sequence that is supposed to be a single exon.

Within the sequence of the BAC129 clone, other novel OR-like genes were also found, which were named GTAR14-2 and GTAR14-4. However, in order to obtain identity in amino acid sequence to other OR gene family proteins, the ORF of GTAR14-2 must be split in two by deletion of a single nucleotide in the middle. On the other hand, by comparing sequences of GTAR14-4 with other OR genes to obtain identity at the amino acid level, the ORF of GTAR14-4 has to be divided into two portions by an in-frame stop codon in the second extracellular domain (loop-1). Accordingly, these genes were judged to be a typical pseudogene of the OR gene family, and were excluded from further study.

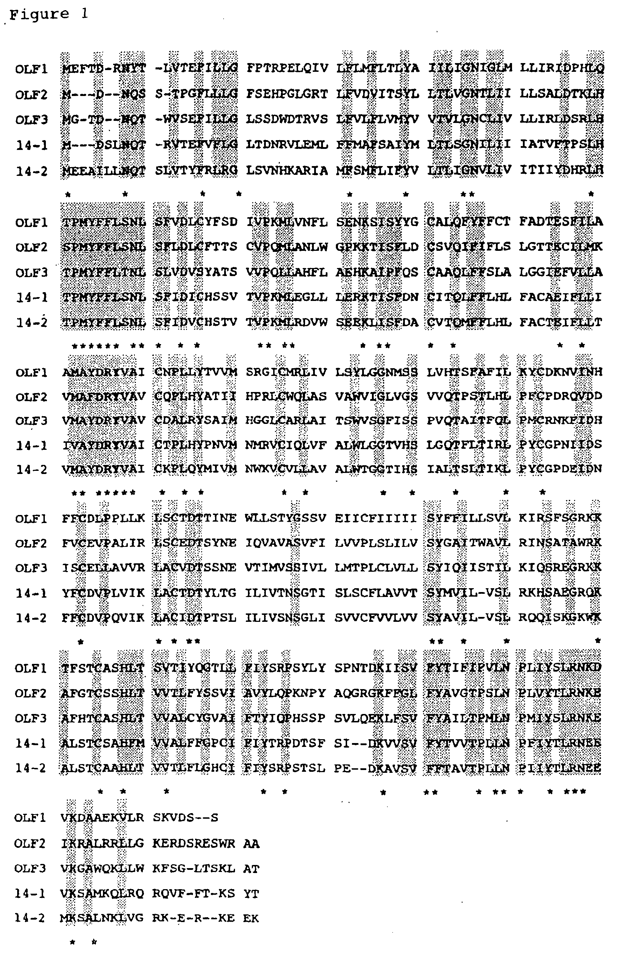

In Figure 1, comparison of the amino acid sequences of known human OLF1, OLF2, and OLF3, and proteins of the present invention, GTAR14-1 and GTAR14-2, is shown. The sequence of GTAR14-2 is of an artificial translational frame created by inserting a single nucleotide at the position of the missing nucleotide.

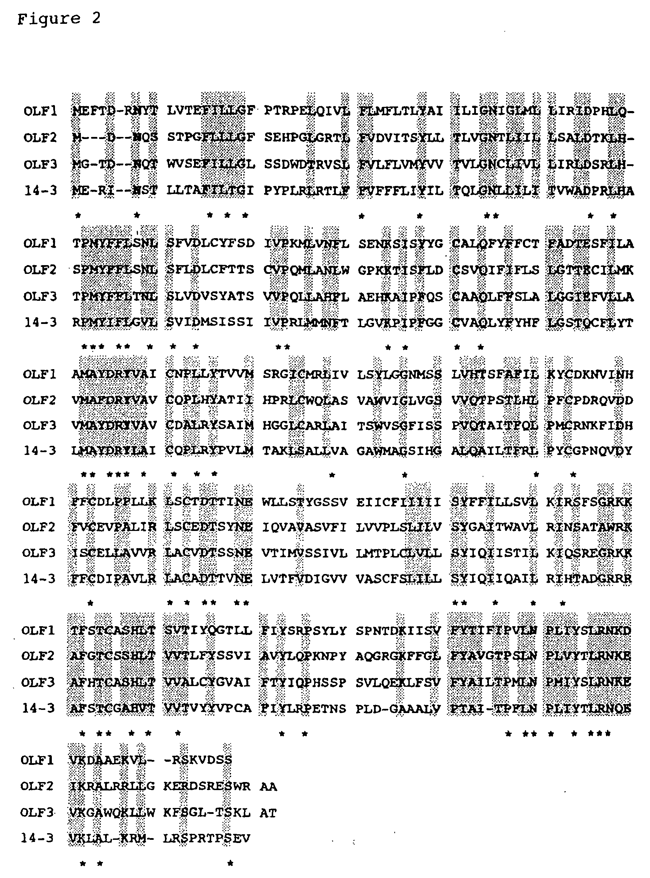

Figure 2 shows comparison of the amino acid sequences among known human OLF1, OLF2, and OLF3, and protein of the present invention, GTAR14-3.

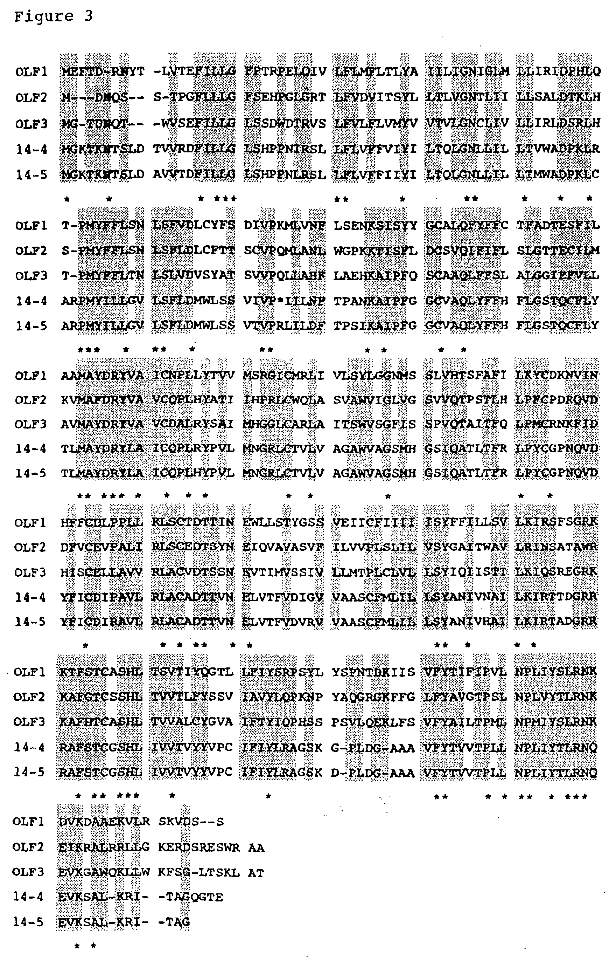

Figure 3 shows comparison of the amino acid sequences among known human OLF1, OLF2, and OLF3, and proteins of the present invention, GTAR14-4 and GTAR14-5. The amino acid sequence of GTAR14-4 is artificially created by reading through the in frame stop codon.

### (2) Isolation of the genes of GTAR14-1, GTAR14-3, and GTAR14-5 by genomic PCR

The sequence of BAC129 clone U85195 was analyzed for the presence of an exon that is broadly conserved in known OLF receptors at the amino acid sequence level, and the predicted exon was used to design primers as follows.

For amplification of GTAR14-1, GTAR14-1-S1 (5'-ATG GAC AGT CTA AAC CAA ACA AGA GTG-3'; SEQ ID NO: 7), GTAR14-1-S2 (5'-ATG GCA TTC TCA GCC ATT TAT ATG CTA-3'; SEQ ID NO: 8), and GTAR14-1-S3 (5'-GGG AAC ATT CTC ATC ATC ATT GCC ACA-3'; SEQ ID NO: 9) were used as sense primers, and GTAR14-1-A1 (5'-TTA TGT ATA TGA TTT CGT GAA AAA AAC-3'; SEQ ID NO: 10), GTAR14-1-A2 (5'-TAC CTC CTC ATT CCT CAA GGT GTA AAT-3'; SEQ ID NO: 11), and GTAR14-1-A3 (5'-GGT GAC CAC TGT GTA GAA GAC AGA CAC-3'; SEQ ID NO: 12) were used as antisense primers.

For amplification of GTAR14-3, GTAR14-3-S1 (5'-ATG GAA AGA ATC AAC AGC ACA CTG TTG-3'; SEQ ID NO: 13), and GTAR14-3-S2 (5'-TCT AAT CTA CAT CCT GAC TCA GCT GGG-3'; SEQ ID NO: 14) were used as sense primers, and GTAR14-3-A1 (5'-TTC AAA CCT CAC TCG GAG TTC TTG GGC-3'; SEQ ID NO: 15), and GTAR14-3-A2 (5'-AGC TTC ACC TCT TGG TTC CGC AGA GTG-3'; SEQ ID NO: 16) were used as antisense primers.

For amplification of GTAR14-5, GTAR14-5-S1 (5'-ATG GGA AAG ACC AAA AAC ACA TCG CTG-3'; SEQ ID NO: 17), and GTAR14-5-S2 (5'-CGT GGT GAC AGA TTT CAT TCT TCT GGG-3'; SEQ ID NO: 18) were used as sense primers, and GTAR14-5-A1 (5'-TCA ACC TGC TGT TAT CCT CTT CAG GGC-3'; SEQ ID NO: 19), and GTAR14-5-A2 (5'-CCT GGT TCC TCA GTG TAT AGA TGA GGG-3'; SEQ ID NO: 20) were used as antisense primers.

Genomic PCR was performed using Human Genomic DNA (Clontech #6550-1) as a template, and primer sets of "14-1-S1 and 14-1-A1," "14-1-S1 and 14-1-A2," "14-1-S2 and 14-1-A1," and "14-1-S2 and 14-1-A2" for amplification of GTAR14-1, "14-3-S1 and 14-3-A1," "14-3-S1 and 14-3-A2," "14-3-S2 and 14-3-A1," and "14-3-S2 and 14-3-A2" for amplification of GTAR14-3, and "14-5-S1 and 14-5-A1," "14-5-S1 and 14-5-A2," "14-5-S2 and 14-5-A1," and "14-5-S2 and 14-5-A2" for amplification of GTAR14-5. PCR was performed using the Advantage cDNA Polymerase Mix (Clontech #8417-1) on a thermal cycler (Perkin Elmer Gene Amp PCR System 2400). By performing PCR under the following conditions, a single band was observed with a predicted size for each primer set.

PCR condition: 94°C for 4 min, 5 cycles of 94°C for 20 sec and 72°C for 2 min, 5 cycles of 94°C for 20 sec and 70°C for 2 min, 28 cycles of 94°C for 20 sec and 68°C for 2 min, 72°C for 4 min, and termination at 4°C.

The obtained products of genomic PCR were subcloned into the pGEM-T Easy vector (Promega #A1360), and the nucleotide sequence was determined. The PCR products were cloned into the pGEM-T Easy vector by ligation using T4 DNA ligase (Promega #A1360) at 4°C for 12 hr. The sample was used to transform *E*. *coli* DH5α (Toyobo # DNA-903) to obtain recombinants of the PCR products and the pGEM-T Easy vector. Recombinants were selected by using the Insert Check Ready (Toyobo #PIK-101). The nucleotide sequence was determined by using the dRhodamine Terminator Cycle Sequencing kit (ABI/Perkin Elmer #4303141) on a DNA Sequencer (ABI PRISM 377).

Ten independent clones of recombinants were selected for each gene from the products of genomic PCR that had been performed using a set of primers that are located in the most external sites of the target gene fragment; that is, the product of the primer set of "14-1-S1 and 14-1-A1" for GTAR14-1 gene, that of "14-3-S1 and 14-3-A1" for GTAR14-3 gene, and that of "14-5-S1 and 14-5-A1" for GTAR14-5 gene. The complete nucleotide sequence of the inserts of all the clones were determined.

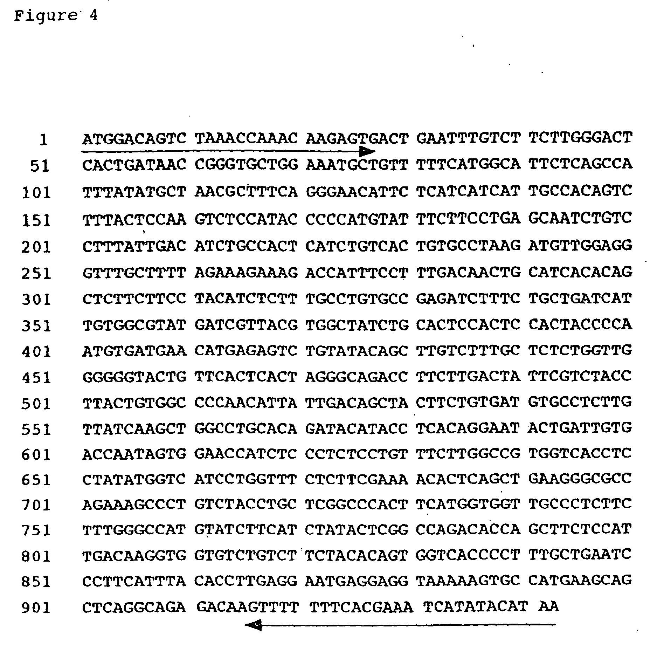

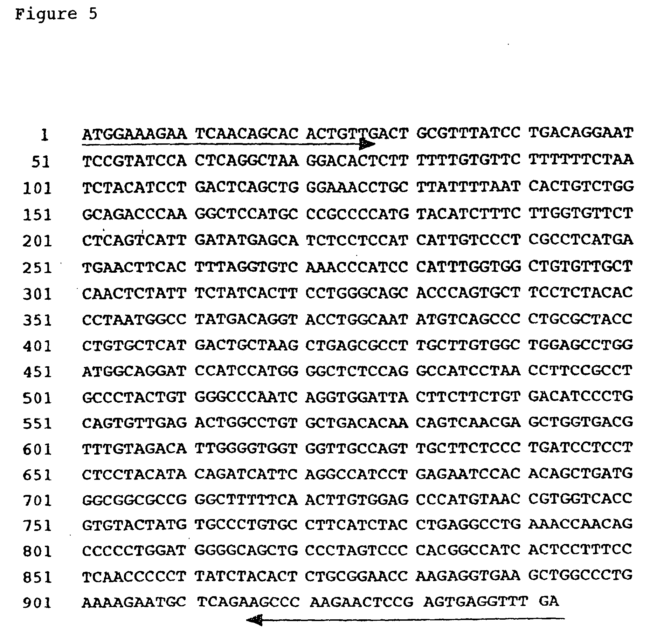

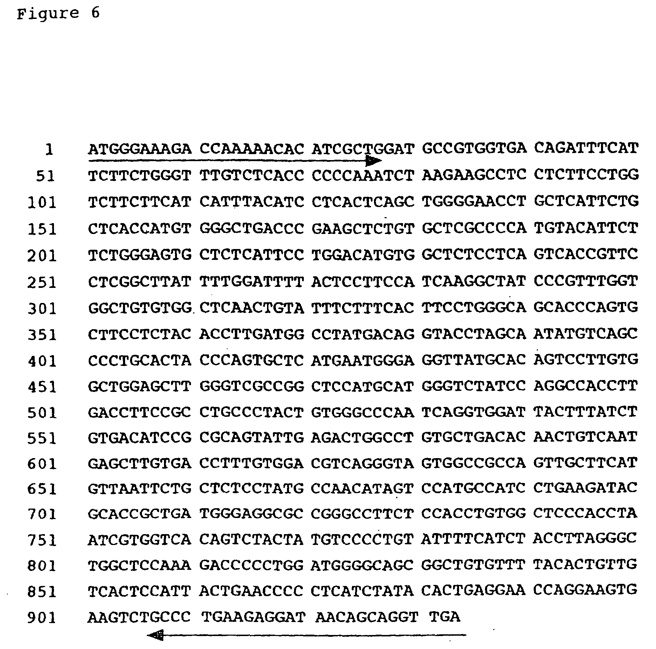

As a result, all clones of GTAR14-1 gene and GTAR14-3 gene showed a single nucleotide sequence of 942 bp. All clones of GTAR14-5 exhibited a single nucleotide sequence of 933 bp. The obtained sequences were verified to be a partial nucleotide sequence of respective genes and confirmed to not be a non-specific product of genomic PCR. Accordingly, these primer sets are expected to be able to work correctly in RT-PCR as described in the following (3). The partial nucleotide sequences of GTAR14-1, GTAR14-3, and GTAR14-5, obtained by genomic PCR, are shown in Figures 4, 5, and 6, and SEQ ID NO: 21, 22, and 23, respectively.

### (3) Analysis of tissue distribution and patterns of GTAR14-1 GTAR14-3, and GTAR14-5 gene expressions by RT-PCR

For analysis of the tissue distribution and patterns of GTAR14-1, GTAR14-3, and GTAR14-5 gene expressions in human tissues, RT-PCR was performed using a primer set of "14-1-S2 and 14-1-A2," "14-3-S2 and 14-3-A2," and "14-5-S2 and 14-5-A2," respectively.

Human Fetal Multiple Tissue cDNA Panel (Clontech #K1425-1), Human Multiple Tissue cDNA Panel I (Clontech #K1420-1), and Human Multiple Tissue cDNA Panel II (Clontech #K1421-1) were used as a template. PCR was performed by using the Advantage cDNA Polymerase Mix (Clontech #8417-1) on a thermal cycler (Perkin Elmer Gene Amp PCR System 2400). PCR reactions were performed under conditions with a single cycle of 94°C for 4 min, sequentially followed by 5 cycles of 94°C for 20 sec and 72°C for 2 min, 5 cycles of 94°C for 20 sec and 70°C for 2 min, 38 cycles of 94°C for 20 sec and 68°C for 2 min, a single cycle of 72°C for 4 min, and termination at 4°C.

The result showed that a strong amplification of GTAR14-1 mRNA was observed only in thymus among human fetal tissues, and also in lymphatic/hematopoietic tissues including thymus and spleen, and testis and pancreas among adult tissues (Figure 7).

GTAR14-3 was strongly amplified in thymus, brain, heart, smooth muscle, and kidney among human fetal tissues, and weakly expressed in liver and lung as well. GTAR14-3 was strongly detected in thymus and testis among adult tissues, and also expressed in digestive organs such as small intestine and colon, and endocrine organs such as placenta and prostate, showing a broad distribution among tissues (Figure 8).

GTAR14-5 was strongly amplified in thymus, spleen, and lung among human fetal tissues, and weakly expressed in brain and kidney. It was detected strongly in lymphatic/hematopoietic tissues such as thymus, spleen, and peripheral leukocytes, and testis, and pancreas in adult tissues (Figure 9).

The products of PCR were subcloned into the pGEM-T Easy vector (Promega #A1360), and the nucleotide sequence were determined. The PCR products were cloned into the pGEM-T Easy vector by ligation using T4 DNA ligase (Promega #A1360) at 4°C for 12 hr. The samples were used to transform *E*. *coli* DH5α (Toyobo # DNA-903) to obtain recombinants of the PCR products and the pGEM-T Easy vector. Recombinants were selected by using the Insert Check Ready (Toyobo #PIK-101). The nucleotide sequence were determined by using the dRhodamine Terminator Cycle Sequencing kit (ABI/Perkin Elmer #4303141) on a DNA Sequencer (ABI PRISM 377). For each gene, ten independent recombinant clones were selected, and the complete nucleotide sequence of the inserts were determined. As a result, all clones showed a single nucleotide sequence.

The obtained sequences were verified to be a partial sequence of the GTAR14-1, GTAR14-3, or GTAR14-5, and confirmed not to be a non-specific product of RT-PCR.

### (4) Full length cDNA cloning by 5'- and 3'-RACE

### (i) 5'-RACE

5'-RACE-PCR was performed to isolate full length cDNAs of GTAR14-1, GTAR14-3, and GTAR14-5. Primers used for amplification of GTAR14-1, GTAR14-3, and GTAR14-5 genes were "14-1-A1," "14-3-A1," and "14-5-A1" for the first PCR, and "14-1-A2, " "14-3-A2, " and "14-5-A2" for the second PCR, respectively.

Human Testis Marathon-Ready cDNA Library (Clontech #7414-1) was used as a template, and the Advantage cDNA Polymerase Mix was used for PCR. PCR was performed on a thermal cycler (Perkin Elmer Gene Amp PCR System 2400) under the following conditions:
first PCR: a single cycle of 94°C for 4 min, sequentially followed by 5 cycles of 94°C for 20 sec and 72°C for 2 min, 5 cycles of 94°C for 20 sec and 70°C for 2 min, 30 cycles of 94°C for 20 sec and 68°C for 2 min, and a single cycle of 72°C for 4 min followed by termination at 4°C;
second PCR: a single cycle of 94°C for 4 min, sequentially followed by 5 cycles of 94°C for 20 sec and 72°C for 2 min, 28 cycles of 94°C for 20 sec and 68°C for 2 min, and a single cycle of 72°C for 4 min followed by termination at 4°C.

The obtained 5'-RACE-PCR products, which showed a single band, were subcloned into the pGEM-T Easy vector as described above. For six independent clones of recombinants, the nucleotide sequence of all inserts were determined by using the dRhodamine Terminator Cycle Sequencing kit (ABI/Perkin Elmer #4303141) on a DNA Sequencer (ABI PRISM 377), as described above.

### (ii) 3'-RACE

3'-RACE-PCR was further performed to isolate full length cDNAs of GTAR14-1, GTAR14-3, and GTAR14-5 genes. Primers used for amplification of GTAR14-1, GTAR14-3, and GTAR14-5 genes were "14-1-S1," "14-3-S1," and "14-5-S1" for first PCR, and "14-1-S2," "14-3-S2," and "14-5-S2" for second PCR, respectively.

Again, Human Testis Marathon-Ready cDNA Library (Clontech #7414-1) was used as a template. PCR was performed using the Advantage cDNA Polymerase Mix under similar conditions to that for 5'-RACE-PCR described above, and PCR products of a single size were obtained. As described above, the obtained PCR products for respective genes were subcloned into the pGEM-T Easy vector. For six independent clones of recombinants, the nucleotide sequence of all inserts were determined by using the dRhodamine Terminator Cycle Sequencing kit (ABI/Perkin Elmer #4303141) on a DNA Sequencer (ABI PRISM 377). For each gene, all six independent clones showed a single nucleotide sequence. Finally, the nucleotide sequences obtained by 5'-RACE and 3'-RACE were combined to determine the nucleotide sequence of full length cDNA of GTAR14-1, GTAR14-3, and GTAR14-5. The determined nucleotide sequences of full length cDNAs are shown in Figures 10 and 11 (GTAR14-1), Figures 12 and 13 (GTAR14-3), and Figures 14 and 15 (GTAR14-5), and SEQ ID NO: 1 (GTAR14-1), SEQ ID NO: 2 (GTAR14-3), and SEQ ID NO: 3 (GTAR14-5).

### Example 2. Isolation and analysis of GTAR11 genes

### (1) BLAST search

When members of known olfactory receptor (OR) gene family are structurally compared one another, they are found to share a high homology in the second and seventh transmembrane domains at the amino acid sequence level. Accordingly, among these two domains, the present inventors chose a conserved amino acid sequence [LHTPMYFFLSNLSF] within the second transmembrane domain for query in BLAST search on the GenBank High Throughput Genomic Sequence (htgs) database, and tried to find a sequence of a novel useful gene belonging to the OR gene family. BLAST search was performed using default values of the TblastN (Ver.2.0.5) program. Besides detecting many sequences containing a known OR genes as a positive control, multiple human genomic sequences having a high structural homology to known OR genes were found. These sequences are found in a sequence of BAC clone originating from human chromosome 11 (GenBank Accession# AC002556), and show about 30 to 40% homology to known human olfactory receptor 1 (OLF1), OLF2, and OLF3 at the amino acid sequence level. Based on the results of analysis of gene expression pattern as described below and prediction of function, the four discovered genes were named G protein-coupled T-cell Activating Receptor 11-1 (GTAR11-1), G protein-coupled T-cell Activating Receptor 11-2 (GTAR11-2), G protein-coupled T-cell Activating Receptor 11-3 (GTAR11-3), and G protein-coupled T-cell Activating Receptor 11-4 (GTAR11-4), respectively.

### (2) Isolation of GTAR11 genes by genomic PCR

The sequence of the BAC clone AC002556 was analyzed for the presence of an exon that is broadly conserved in known OLF receptors at the amino acid sequence level, and the exon predicted was used to design primers as follows.

For amplification of GTAR11-1, GTAR11-1-S1 (5'-GAA GAG CAG TGA GGG TCC ATG TTA AGG-3'; SEQ ID NO: 34), and GTAR11-1-S2 (5'-CAG CAG CTT GTC CTT CGT CGA TTT CTG C-3'; SEQ ID NO: 35) were used as sense primers, and GTAR11-1-A2 (5'-GCT AGG GTG GGC ACC AAG GTG TTA AAC CC-3'; SEQ ID NO: 36), and GTAR11-1-A3 (5'-TGC AAA AGG ACA GTT TCA TCA TGG CAC-3'; SEQ ID NO: 37) were used as antisense primers.

For amplification of GTAR11-2, GTAR11-2-S1 (5'-CAA AGA ACT CAC CCA AAT TCC TAC AGC T-3'; SEQ ID NO: 38), and GTAR11-2-S2 (5'-CAT GGT AGG CAA CCT TGG CTT GAT CAC-3'; SEQ ID NO: 39) were used as sense primers, and GTAR11-2-A1 (5'-GTT TAT TAA ATC ACA CAT AAC ACC ATC TG-3'; SEQ ID NO: 40), and GTAR11-2-A2 (5'-CAG AGA CAG AGC AAT GAC ATG AGA GCT AC-3'; SEQ ID NO: 41) were used as antisense primers.

For amplification of GTAR11-3, GTAR11-3-S1 (5'-CAA AGA ACT CAC CCA AAT TCC TAC AGC C-3'; SEQ ID NO: 42), and GTAR11-3-S2 (5'-CAT GGT AGG CAA CCT TGG CTT GAT CAT-3'; SEQ ID NO: 43) were used as sense primers, and GTAR11-3-A1 (5'-GTT TAT TAA ATC ACA CAT AAC ACC ATC TG-3'; SEQ ID NO: 44), and GTAR11-3-A2 (5'-CAG AGA CAG AGC AAT GAC ATG AGA GCT AC-3'; SEQ ID NO: 45) were used as antisense primers.

For amplification of GTAR11-4, GTAR11-4-S1 (5'-CCA GAC AGC TCG CCA AGA GAG AAT GAC-3'; SEQ ID NO: 46), and GTAR11-4-S2 (5'-CCT TTA TAG ATC TCT GTT ATT CCT GTG TG-3'; SEQ ID NO: 47) were used as sense primers, and GTAR11-4-A1 (5'-TCG GTT GCC AGT GAT ATG AAG AGA CCC-3'; SEQ ID NO: 48), and GTAR11-4-A2 (5'-GGC TTT GGA TCT GCC CTC TGC AGA AGG-3'; SEQ ID NO: 49) were used as antisense primers.

Genomic PCR was performed using Human Genomic DNA (Clontech #6550-1) as a template, and primer sets of "11-1-S1 and 11-1-A2," "11-1-S1 and 11-1-A3," "11-1-S2 and 11-1-A2," and "11-1-S2 and 11-1-A3" for amplification of GTAR11-1, "11-2-S1 and 11-2-A1," "11-2-S1 and 11-2-A2," "11-2-52 and 11-2-A1," and "11-2-S2 and 11-2-A2" for amplification of GTAR11-2, "11-3-S1 and 11-3-A1," "11-3-S1 and 11-3-A2," "11-3-S2 and 11-3-A1," and "11-3-S2 and 11-3-A2" for amplification of GTAR11-3, and "11-4-S1 and 11-4-A1," "11-4-S1 and 11-4-A2," "11-4-S2 and 11-4-A1," and "11-4-S2 and 11-4-A2" for amplification of GTAR11-4. PCR was performed using the Advantage cDNA Polymerase Mix (Clontech #8417-1) on a thermal cycler (Perkin Elmer Gene Amp PCR System 2400). Following conditions were used for PCR; a single cycle of 94°C for 4 min, sequentially followed by 5 cycles of 94°C for 20 sec and 72°C for 2 min, 5 cycles of 94°C for 20 sec and 70°C for 2 min, 28 cycles of 94°C for 20 sec and 68°C for 2 min, and then a single cycle of 72°C for 4 min followed by termination at 4°C. As a result, a single band was detected with predicted size for each primer set.

The products obtained in the above genomic PCR were subcloned into the pGEM-T Easy vector (Promega #A1360), and the nucleotide sequence were determined. PCR products were cloned into the pGEM-T Easy vector by ligation using T4 DNA ligase (Promega #A1360) at 4°C for 12 hr. The samples were used to transform *E*. *coli* DH5α (Toyobo # DNA-903) to obtain recombinants of the PCR products and the pGEM-T Easy vector. Recombinants were selected by using the Insert Check Ready (Toyobo #PIK-101). The nucleotide sequences were determined by using the dRhodamine Terminator Cycle Sequencing kit (ABI/Perkin Elmer #4303141) on a DNA Sequencer (ABI PRISM 377).

For each gene, ten independent clones of recombinants were selected from the products of genomic PCR that had been performed using a set of primers that are located within the ORF of the target gene fragment; that is the product of the primer set of "11-1-S2 and 11-1-A3" for GTAR11-1 gene, that of "11-2-S2 and 11-2-A2" for GTAR11-2 gene, that of "11-3-S2 and 11-3-A2" for GTAR11-3 gene, and that of "11-4-S2 and 11-4-A2" for GTAR11-4 gene. The nucleotide sequence of all the inserts were determined.

As a result, a single nucleotide sequence of 450 bp was obtained from all clones of GTAR11-1 gene. From all clones of GTAR11-2 and GTAR11-3 genes, a single nucleotide sequence of 637 bp was obtained. A single nucleotide sequence of 509 bp was obtained from all clones of GTAR11-4 gene. The obtained sequences were verified to be a partial nucleotide sequence of the respective genes to confirm that they are not a non-specific product of genomic PCR. Accordingly, these primer sets are expected to work correctly in RT-PCR described in the following (3). The partial nucleotide sequences of GTAR11-1, GTAR11-2, GTAR11-3, and GTAR11-4 obtained by the genomic PCR are shown in Figures 16, 17, 18, and 19, and SEQ ID NO: 50, 51, 52, and 53, respectively.

### (3) Analysis of tissue distribution and patterns of GTAR11 gene expressions by RT-PCR

For analysis of the tissue distribution and patterns of GTAR11 gene expressions in human tissues, RT-PCR was performed using a primer set of "11-1-S2 and 11-1-A2," "11-2-S2 and 11-2-A2," "11-3-S2 and 11-3-A2," and "11-4-S2 and 11-4-A2" for GTAR11-1, GTAR11-2, GTAR11-3, and GTAR11-4 genes, respectively.

Human Fetal Multiple Tissue cDNAPanel (Clontech#K1425-1), Human. Multiple Tissue cDNA Panel I (Clontech #K1420-1), and Human Multiple Tissue cDNA Panel II (Clontech #K1421-1) were used as a template. PCR was performed by using the Advantage cDNA Polymerase Mix (Clontech #8417-1) on a thermal cycler (Perkin Elmer Gene Amp PCR System 2400). PCR reactions were performed under conditions with a single cycle of 94°C for 4 min, sequentially followed by 5 cycles of 94°C for 20 sec and 72°C for 2 min, 5 cycles of 94°C for 20 sec and 70°C for 2 min, 38 cycles of 94°C for 20 sec and 68°C for 2 min, and then a single cycle of 72°C for 4 min followed by termination at 4°C.

The result showed a strong amplification of GTAR11-1 mRNA in spleen and weak expression in brain, lung, and smooth muscle among human fetal tissues. GTAR11-1 gene was also expressed in multiple tissues in adult including lymphatic/hematopoietic tissues such as thymus, spleen, and peripheral lymphocytes, digestive organs such as small intestine and colon, genital organs, neuronal tissues, and endocrine organs (Figure 20).

GTAR11-2 gene was strongly amplified in thymus, and weakly expressed in spleen, smooth muscle, lung, and kidney among fetal tissues. GTAR11-2 gene was also detected in multiple tissues in adult including lymphatic/hematopoietic tissues such as thymus and peripheral lymphocytes, digestive organs, genital organs, neuronal tissues, and endocrine organs (Figure 21).

GTAR11-3 gene was strongly amplified in smooth muscle among human fetal tissues, and weakly expressed in brain, lung, and liver as well. The gene was also detected in multiple tissues in adult including lymphatic/hematopoietic tissues such as thymus, spleen, and peripheral lymphocytes, digestive organs such as small intestine and colon, muscle, genital organs, neuronal tissues, and endocrine organs (Figure 22).

GTAR11-4 gene was strongly amplified only in smooth muscle in fetus. It was also expressed in multiple tissues in adult including lymphatic/hematopoietic tissues such as thymus, spleen, and peripheral lymphocytes, digestive organs such as small intestine and colon, muscle, genital organs, and endocrine organs (Figure 23).

The obtained PCR products were subcloned into the pGEM-T Easy vector (Promega #A1360), and the nucleotide sequence were determined. The PCR products were cloned into the pGEM-T Easy vector by ligation using T4 DNA ligase (Promega #A1360) at 4°C for 12 hr. The samples were used to transform *E. coli* DH5α (Toyobo # DNA-903) to obtain recombinants of the PCR products and the pGEM-T Easy vector. Recombinants were selected by using the Insert Check Ready (Toyobo #PIK-101). The nucleotide sequence were determined by using the dRhodamine Terminator Cycle Sequencing kit (ABI/Perkin Elmer #4303141) on a DNA Sequencer (ABI PRISM 377). For each gene, ten independent clones of recombinants were selected, and the nucleotide sequence of all the inserts were determined. All clones of each gene showed a single nucleotide sequence.

The obtained sequences were verified to be a partial sequence of GTAR11-1, GTAR11-2, GTAR11-3, and GTAR11-4 genes, and confirmed not to be a non-specific product of RT-PCR.

### (4) Cloning of full length cDNA by 5'- and 3'-RACE

### (i) 5'-RACE

To isolate full length cDNAs of GTAR11 genes, 5'-RACE-PCR was performed. Primers used for amplification of GTAR11-1, GTAR11-2, GTAR11-3, and GTAR11-4 genes were "11-1-A2," "11-2-A1," "11-3-A1," and "11-4-A1" for first PCR, and "11-1-A3," "11-2-A2," "11-3-A2," and "11-4-A2" for second PCR, respectively.

Human Testis Marathon-Ready cDNA Library (Clontech #7414-1) was used as a template, and the Advantage cDNA Polymerase Mix was used for PCR. PCR was performed on a thermal cycler (Perkin Elmer Gene Amp PCR System 2400) under the following conditions:
first PCR: a single cycle of 94°C for 4 min, sequentially followed by 5 cycles of 94°C for 20 sec and 72°C for 2 min, 5 cycles of 94°C for 20 sec and 70°C for 2 min, 30 cycles of 94°C for 20 sec and 68°C for 2 min, and a single cycle of 72°C for 4 min followed by termination at 4°C;
second PCR: a single cycle of 94°C for 4 min, sequentially followed by 5 cycles of 94°C for 20 sec and 72°C for 2 min, 28 cycles of 94°C for 20 sec and 68°C for 2 min, and a single cycle of 72°C for 4 min followed by termination at 4°C.

The obtained 5#-RACE-PCR products, detected as a single band, were subcloned into the pGEM-T Easy vector, six independent clones of recombinants were selected for each, and the nucleotide sequence of all inserts were determined by using the dRhodamine Terminator Cycle Sequencing kit (ABI/Perkin Elmer #4303141) on a DNA Sequencer (ABI PRISM 377), as described above.

### (ii) 3'-RACE '

3'-RACE-PCR was further performed to isolate full length cDNAs of GTAR11 genes. Primers used for amplification of GTAR11-1, GTAR11-2, GTAR11-3, and GTAR11-4 genes were "11-1-S1," "11-2-S1," "11-3-S1," and "11-4-S1" for first PCR, and "11-1-S2," "11-2-S2," "11-3-S2," and "11-4-S2" for second PCR, respectively.

Again, Human Testis Marathon-Ready cDNA Library (Clontech #7414-1) was used as a template. PCR was performed using the Advantage cDNA Polymerase Mix under similar conditions to that of 5'-RACE-PCR described above, and PCR products of a single size were obtained. As described above, the obtained products were subcloned into the pGEM-T Easy vector, six independent clones of recombinants were selected for each, and the nucleotide sequence of all inserts were determined by using the dRhodamine Terminator Cycle Sequencing kit (ABI/Perkin Elmer #4303141) on a DNA Sequencer (ABI PRISM 377). All six clones of each gene showed a single sequence. The nucleotide sequences obtained by 5'-RACE and 3'-RACE were combined to generate the sequence of full length cDNA of each GTAR11 gene. The resulting nucleotide sequences of cDNAs are shown in Figure 24 (GTAR11-1), Figure 25 (GTAR11-2), Figure 26 (GTAR11-3), and Figure 27 (GTAR11-4), and SEQ ID NO: 24 (GTAR11-1), SEQ ID NO: 25 (GTAR11-2), SEQ ID NO: 26 (GTAR11-3), and SEQ ID NO: 27 (GTAR11-4).

The GTAR11-1 cDNA obtained by the first 3'-RACE-PCR had a deletion at the C-terminus, and thus was not full length (the nucleotide sequence of the cDNA and the amino acid sequence of the peptide encoded by the cDNA are shown in SEQ ID NO: 32, and SEQ ID NO: 33, respectively). Consequently, 3'-RACE-PCR was performed again for GTAR11-1, and the full length cDNA of GTAR11-1 was obtained as described above.

The genome of known OR gene family shares a unique structural feature that the ORF from the initiation codon to the stop codon is contained in a single exon of the genome sequence encoding known OR genes. The isolated full length CDNAS of GTAR11-1, GTAR11-2, GTAR11-3, and GTAR11-4 genes also have their whole ORF within a sequence that is supposed to be a single exon.

Figure 28 shows comparison of the amino acid sequences of known human OLF1, OLF2, and OLF3, and GTAR11-1. Figure 29 shows comparison of the amino acid sequences of known human OLF2 and OLF3, and GTAR11-2. Figure 30 shows comparison of the amino acid sequences of known human OLF2 and OLF3, and GTAR11-3. Figure 31 shows comparison of the amino acid sequences of known human OLF2 and OLF3, and GTAR11-4.

### Industrial Applicability

The present invention provides novel G protein-coupled receptor proteins and their genes. According to their particular expression patterns and such, the receptor proteins of the present invention appear to be involved in immune response, hematopoiesis, etc. Thus, one can use these proteins in screening assays, for developing drugs that can regulate immune response and/or hematopoietic cells. Using the receptor proteins of the present invention, one can also screen for ligands capable of binding to the receptor proteins of the invention, such ligands also being potentially involved in immune response, hematopoiesis, and so on.

## Claims

1. A G protein-coupled receptor protein selected from the group consisting of:
(a) a protein having an amino acid sequence selected from the group consisting of SEQ ID NO: 4, 5, 6, 28, 29, 30, and 31;
(b) a protein of (a), wherein one or more amino acids are modified by deletion; addition, insertion, and/or substitution by another amino acid residue; and
(c) a protein encoded by DNA that hybridizes with DNA having a nucleotide sequence selected from the group consisting of SEQ ID NO: 1, 2, 3, 24, 25, 26, and 27.

2. A fusion protein comprising the protein of claim 1 and another peptide or polypeptide.

3. A peptide comprising a part of the protein of claim 1.

4. A DNA encoding the protein or peptide of any of claims 1 to 3.

5. A vector comprising the DNA of claim 4 inserted therein.

6. A transformant carrying the DNA of claim 4 in an expressible manner.

7. A method of producing the protein or peptide of any of claims 1 to 3, comprising the steps of: cultivating the transformant of claim 6, and recovering the protein or peptide expressed therein.

8. A method of screening for a compound that is capable of binding to the protein of claim 1, comprising the steps of:
(a) exposing a test sample to the protein or peptide of any of claims 1 to 3, and
(b) selecting the compound that binds to the protein or peptide of any of claims 1 to 3.

9. A method of screening for a ligand and/or agonist that is capable of binding to the protein of claim 1, comprising the steps of:
(a) exposing a test sample to a cell expressing the protein or peptide of any of claims 1 to 3 on its surface,
(b) measuring a biochemical change in said cell, and
(c) selecting the compound that induces said biochemical change in said cell.

10. An antibody that binds to the protein of claim 1.

11. A method of detecting or measuring the protein or peptide of any of claims 1 to 3, comprising the steps of: exposing the antibody of claim 10 to a sample which is assumed to comprise said protein or peptide, and detecting or measuring the generation of an immune complex between said antibody and said protein or peptide.

12. A DNA of a length of 15 nucleotides or longer that hybridizes with a DNA having a nucleotide sequence selected from the group consisting of SEQ ID NO: 1, 2, 3, 24, 25, 26, 27, and their complementary strands.
